# FASCICULE DE BREVET EUROPEEN

(11) **EP 3 700 918 B1**
(45) Date de publication et mention de la délivrance du brevet: **27.08.2025**
(21) Numéro de dépôt: 18801020.1
(22) Date de dépôt: 25.10.2018
(51) Int. Cl.: C07K 14/005, C12N 7/00, C12N 15/63, C12N 15/86, C12N 9/10, C12N 15/90

(54) **SYSTÈME D'EXPRESSION BACULOVIRUS**
BACULOVIRUS-EXPRESSIONSSYSTEM
BACULOVIRUS EXPRESSION SYSTEM

(30) Priorité: 25.10.2017 FR 1760068
(43) Date de publication de la demande: 02.09.2020
(73) Titulaire: Centre National de la Recherche Scientifique (CNRS), 75016 Paris (FR); Institut national de recherche pour l'agriculture, l'alimentation et l'environnement, 75007 Paris (FR)
(72) Inventeur: CERUTTI, Martine, 30380 Saint Christol les Ales (FR); JULIANT, Sylvie, 30380 Saint Christol les Ales (FR); BERNON, Coralie, 30140 Boisset-et-Gaujac (FR)
(74) Mandataire: Novagraaf Technologies
(86) Numéro de dépôt international: PCT/FR2018/052652
(87) Numéro de publication internationale: WO 2019/081858

(56) Documents cités:
- WO-A1-2018/024998
- WO-A2-01/12829
- WO-A2-2010/055292
- POSSEE ROBERT D ET AL: "Generation of Baculovirus Vectors for the High-Throughput Production of Proteins in Insect Cells", BIOTECHNOLOGY AND BIOENGINEERING, WILEY & SONS, HOBOKEN, NJ, US, vol. 101, no. 6, 1 December 2008 (2008-12-01), pages 1115 - 1122, XP002552791, ISSN: 0006-3592, [retrieved on 20080604], DOI: 10.1002/BIT.22002
- YUTA KANAI ET AL: "Multiple large foreign protein expression by a single recombinant baculovirus: A system for production of multivalent vaccines", PROTEIN EXPRESSION AND PURIFICATION., vol. 91, no. 1, 1 September 2013 (2013-09-01), SAN DIEGO, CA., pages 77 - 84, XP055324848, ISSN: 1046-5928, DOI: 10.1016/j.pep.2013.07.005
- POUL M-A ET AL: "Design of cassette baculovirus vectors for the production of therapeutic antibodies in insect cells", IMMUNOTECHNOLOGY, ELSEVIER SCIENCE PUBLISHERS BV, NL, vol. 1, no. 3, 1 December 1995 (1995-12-01), pages 189 - 196, XP004052721, ISSN: 1380-2933, DOI: 10.1016/1380-2933(95)00019-4
- LAKSHMI S VIJAYACHANDRAN ET AL: "Gene gymnastics: Synthetic biology for baculovirus expression vector system engineering", BIOENGINEERED, vol. 4, no. 5, 1 September 2013 (2013-09-01), pages 279 - 287, XP055145260, ISSN: 2165-5979, DOI: 10.4161/bioe.22966
- DIETER PALMBERGER ET AL: "SweetBac: A New Approach for the Production of Mammalianised Glycoproteins in Insect Cells", PLOS ONE, vol. 7, no. 4, 2 April 2012 (2012-04-02), pages e34226, XP055472218, DOI: 10.1371/journal.pone.0034226
- CHANG G-D ET AL: "Improvement of glycosylation in insect cells with mammalian glycosyltransferases", JOURNAL OF BIOTECHNOLOGY, ELSEVIER, AMSTERDAM, NL, vol. 102, no. 1, 10 April 2003 (2003-04-10), pages 61 - 71, XP002387943, ISSN: 0168-1656
- D. L. JARVIS ET AL: "Novel Baculovirus Expression Vectors That Provide Sialylation of Recombinant Glycoproteins in Lepidopteran Insect Cells", JOURNAL OF VIROLOGY., vol. 75, no. 13, 1 July 2001 (2001-07-01), US, pages 6223 - 6227, XP055472152, ISSN: 0022-538X, DOI: 10.1128/JVI.75.13.6223-6227.2001

## Description

### Domaine technique

L'invention concerne un procédé pour produire un baculovirus recombinant dont le génome comprend un ou plusieurs transgène(s) codant chacun une enzyme de maturation des protéines et au moins deux transgènes codant chacun un polypeptide d'intérêt, des baculovirus ou des génomes de baculovirus recombinants obtenus par ce procédé, des jeux d'éléments de recombinaison homologue, des cellules comprenant un baculovirus recombinant ou un génome de baculovirus recombinant ainsi que l'utilisation des baculovirus ou des génomes de baculovirus recombinants pour la production de polypeptides d'intérêt.

### Arrière-plan technologique

Les baculovirus sont une famille de virus en forme de bâtonnet, spécifiques des arthropodes, qui compte quatre genres (Alphabaculovirus, Betabaculovirus, Deltabaculovirus, Gammabaculovirus) regroupant 49 espèces. Les baculovirus ne sont pas capables de se répliquer dans les cellules de mammifères ou d'autres vertébrés.

Le génome des baculovirus est constitué d'une molécule d'ADN bicaténaire, circulaire, d'une taille comprise entre 80 et 180 kpb. Le génome des baculovirus s'associe à des protéines hautement basiques de 6,5 kDa, au sein d'une nucléocapside à symétrie hélicoïdale, qui contient une protéine de capside de 39 kDa. La taille du génome détermine la longueur de la nucléocapside. La nucléocapside est ensuite enfermée dans une enveloppe lipoprotéique pour former la particule virale ou virion. Ces structures peuvent être recouvertes d'une matrice cristalline ou polyèdre constituée essentiellement d'une seule protéine (la polyédrine) d'environ 30 kDa. Les polyèdres sont de grandes structures dont la taille varie de 1 à 15 µm de diamètre avec une enveloppe externe polysaccharidique qui confère une protection supplémentaire.

Les baculovirus, dont le génome a été modifié génétiquement, sont utilisés en biotechnologie pour la production de protéines recombinantes (i.e. baculovirus recombinants). Après pénétration dans une cellule d'insecte, ces baculovirus recombinants vont utiliser la machinerie de la cellule d'insecte pour produire la protéine recombinante.

Les baculovirus recombinants sont obtenus en insérant un ou plusieurs gènes provenant d'autres espèces (par exemple des êtres humains, d'autres vertébrés, plantes, bactéries et virus) dans le génome d'un baculovirus parent. Ces gènes sont placés sous le contrôle d'un promoteur viral ou cellulaire (par exemple le promoteur du gène de la polyédrine) pour générer un génome de baculovirus recombinant. Le promoteur permet la transcription du gène étranger en ARN messager qui à son tour est traduit en protéine dans la cellule d'insecte infectée par le baculovirus recombinant. L'avantage d'utiliser ce système est que le niveau de production de la protéine recombinante dans les cellules d'insecte infectées par le baculovirus recombinant peut être très important. La protéine recombinante peut ensuite être purifiée à partir des cellules infectées si la protéine est intracellulaire ou bien à partir du milieu de culture si la protéine est sécrétée. Le système d'expression baculovirus est largement utilisé dans l'industrie et dans les laboratoires de recherche. En plus de l'importante productivité du système d'expression baculovirus, ce système est également très apprécié car il permet de produire des protéines recombinantes biologiquement actives. En effet, les cellules d'insectes permettent généralement d'obtenir des modifications post-traductionnelles appropriées.

Néanmoins, certaines protéines nécessitent des modifications post-traductionnelles que les cellules d'insecte ne sont pas capables de faire. Ces modifications post-traductionnelles étant normalement effectuées par des enzymes de maturation des protéines spécifiques.

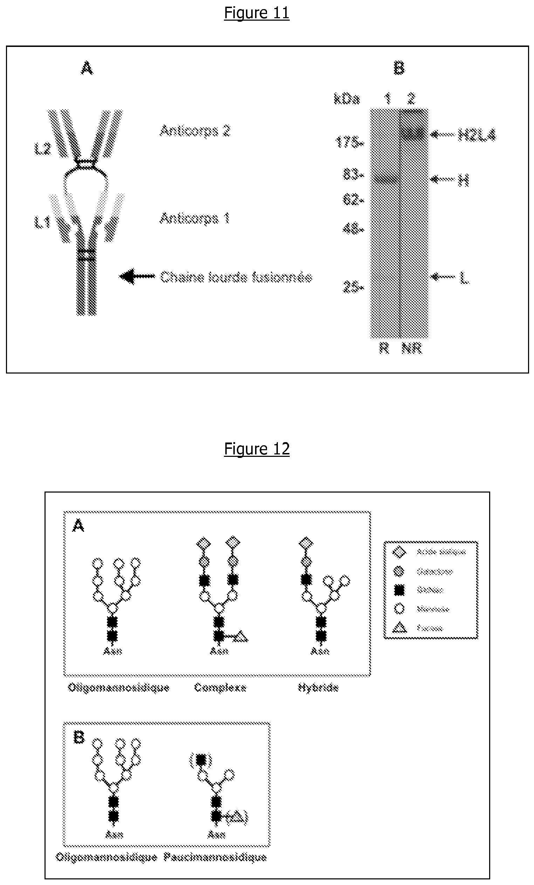

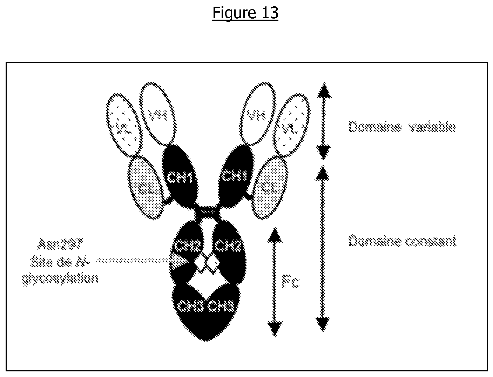

Par exemple, la majorité des glycoprotéines humaines présentent une glycosylation dite complexe, elles sont en général sialylées **(****Figure 12A****).** La sialylation est un élément important de la stabilisation de la structure de certaines protéines, elle les rend plus solubles, plus résistantes à la chaleur et aux protéases. Le taux de sialylation d'une protéine peut directement influencer sa demi-vie dans le sérum, les protéines désialylées étant rapidement captées par les récepteurs aux asialoprotéines. Il y a une exception à ce modèle de demi-vie, les anticorps. En effet, la demi-vie des anticorps est contrôlée essentiellement via leur interaction avec un récepteur au domaine Fc (FcR) (Fc pour « fragment cristallisable » : région constante de l'anticorps **(****Figure 13****)),** particulier appelé récepteur FcRn. La glycosylation a cependant un rôle très important dans le contrôle de l'activité des anticorps tout particulièrement la N-glycosylation présente dans le domaine CH2 de la région constante des IgG sur Asn297 **(****Figure 13****).** En effet, la nature de cette N-glycosylation permet la modulation de ce que l'on appelle les activités effectrices de l'anticorps, activités qui vont permettre par exemple de tuer une cellule tumorale ciblée par un anticorps. Il a notamment été démontré que (i) l'ADCC (Antibody Dependent Cell-mediated Cytotoxicity) est augmentée très significativement lorsque le fucose α1,6 est éliminé du core du glycanne et (ii) la CDC (Complement Dependent Cytotoxicity) est dépendante de la présence de galactose. Enfin, même si le mécanisme d'action est encore très controversé, la sialylation de ce motif glycannique serait importante pour bloquer l'activité proinflammatoire des anticorps.

Ainsi si l'on veut produire des anticorps très cytotoxiques, anticorps capables d'induire des activités ADCC et CDC élevées, par exemple pour détruire spécifiquement une tumeur, l'idéal est de produire des anticorps galactosylés. Par contre, un anticorps qui sera utilisé simplement comme ligand, ligand d'un récepteur cellulaire par exemple pour induire une apoptose, ou bien pour faire de l'imagerie en marquant spécifiquement un tissu, il sera souhaitable d'utiliser un anticorps incapable d'induire de la cytotoxicité cellulaire, dans ce cas un anticorps sialylé sera le plus adapté.

De nombreuses études sur le potentiel de N-glycosylation des cellules d'insectes montrent très clairement que si l'addition des glycannes est spécifique, c'est-à-dire qu'elle est toujours effectuée sur les résidus asparagine identiques à ceux qui sont glycosylés naturellement sur la protéine d'origine exprimée par le tissu (par exemple l'Asn297 des anticorps), la structure des glycannes est différente, ils sont plus courts et il n'y a pas de glycannes complexes **(****Figure 12B****).** Il a également été montré que ces structures tronquées résultaient de l'absence ou de la faible activité de plusieurs enzymes impliquées dans la biosynthèse des glycannes complexes comme, la N-acétylglucosaminyltransférase II (GnT-II) et la β-1,4 galactosyltransférase (β-1,4 GaIT), les sialyltransférases et de l'absence d'un nucléotide-sucre, le CMP-NeuAc.

Dans le but d'obtenir des protéines d'intérêt correctement maturées, il est donc important de compléter le potentiel enzymatique de maturation de la cellule d'insecte.

Des baculovirus comprenant des gènes codant des enzymes de maturation des protéines ont déjà été décrits.

L'article de Palmberger et al. (2012) est relatif à un baculovirus recombinant comprenant dans son génome des séquences codant pour deux enzymes de glycosylation dans un même locus. Ce baculovirus est utilisé pour la production de l'anticorps 3D6 anti-gp41 du VIH. Deux gènes codant les chaines lourde et légère de l'anticorps ont été insérés dans le génome de ce baculovirus. Ces baculovirus recombinants sont générés à partir d'un bacmid (infectieux en cellules d'insectes) dans une bactérie. Le procédé de construction utilise le système Cre/Lox et la transposition Tn7 pour une intégration itérative des différents gènes (gènes d'intérêt et gènes de maturation des protéines). Une sélection des bactéries sur différents antibiotiques est alors nécessaire pour isoler les bacmids recombinants infectieux qui seront ensuite introduits, sous forme d'ADN, dans des cellules d'insectes pour produire des baculovirus recombinants.

L'article de Chang et al. (2003) divulgue un baculovirus recombinant exprimant à la fois un polypeptide d'intérêt (human α1-antitrypsin) et une série de glycosyltransférases dans un seul locus. La recombinaison est réalisée dans la cellule d'insectes, avec un ADN viral non infectieux linéarisé. La réparation de cet ADN consécutive à la recombinaison homologue avec les vecteurs de transfert permet de reconstruire un ADN viral circulaire et donc infectieux.

Les procédés de construction de ces baculovirus sont basés sur des étapes d'intégrations itératives des gènes d'intérêt et des gènes de maturation des protéines mettant en jeux des systèmes d'intégration classiques,
(i) soit dans une bactérie comme la transposition Tn7 ou le système cre/lox (Palmberger et al. 2012)
(ii) soit des étapes de recombinaisons homologues classiques dans une cellule d'insectes entre un ADN viral linéarisé et un vecteur de transfert (Chang et al. 2003).

Dans les 2 cas, pour chaque transgène intégré, une étape de sélection est nécessaire soit (i) dans la bactérie, une sélection basée sur la présence d'un gène de résistance à un antibiotique adjacent au transgène, soit (ii) dans la cellule d'insecte, chaque étape de recombinaison nécessite la présence, dans l'ADN viral, d'un nouveau site unique de clivage spécifique d'une enzyme de restriction pour pouvoir de nouveau linéariser l'ADN viral et procéder à une nouvelle intégration. La réparation de cet ADN consécutive à la recombinaison homologue avec les vecteurs de transfert permet de reconstruire un ADN viral circulaire et donc infectieux.

Ainsi, il existe encore un besoin de mettre au point des procédés faciles à mettre en œuvre et qui permettent de produire des protéines recombinantes d'intérêt, notamment des protéines comprenant plusieurs sous-unités distinctes, tels que les anticorps dans des systèmes d'expression baculovirus et qui peuvent notamment être développés à l'échelle industrielle et qui sont capables d'induire une maturation appropriée de la protéine d'intérêt. Il est en particulier nécessaire, pour produire certaines protéines recombinantes d'intérêt, composées de plusieurs sous-unités peptidiques, de posséder un système d'expression baculovirus dans lequel plusieurs transgènes, chacun codant une sous-unité, peuvent être intégrés aisément, et de préférence en une seule étape.

Or, pour pouvoir intégrer plusieurs transgènes dans un baculovirus, les procédés connus jusqu'à présent nécessitaient :
- Soit de procéder en plusieurs étapes successives, chaque étape permettant l'intégration d'un ou de deux transgènes (en « tête bêche »). Ainsi, il était techniquement possible, mais très fastidieux, d'intégrer plus de deux transgènes dans un génome de baculovirus en une seule étape;
- Soit d'intégrer les multiples transgènes dans un seul locus. Toutefois, la recombinaison homologue pour l'intégration d'un fragment d'ADN de grande taille composé de plusieurs transgènes (cassettes d'expression) est souvent compliquée, voire impossible, sans induire des remaniement du génome viral. Il est donc préférable de répartir uniformément, dans le génome d'un baculovirus, les positions d'intégration de plusieurs transgènes.
Afin d'éliminer l'étape de sélection des baculovirus recombinants ayant intégré avec succès un transgène d'intérêt, il a été proposé un procédé basé sur l'utilisation conjointe :
- d'un baculovirus dans lequel un gène essentiel à la réplication virale est non-fonctionnel, et
- d'un vecteur de transfert comprenant une séquence nucléotidique permettant de restaurer la fonction d'un gène essentiel à la réplication, et un transgène codant pour un polypeptide d'intérêt.
Ce procédé est notamment décrit dans la demande de brevet WO 01/12829 et dans l'article de Possee et al., 2008.

Toutefois, ce procédé ne permet d'intégrer qu'un ou deux transgènes d'intérêt (en tête-bêche), dans un seul locus. L'opération doit être répétée plusieurs fois pour intégrer un troisième ou un quatrième transgène dans un autre locus, lié à un autre gène essentiel de réplication qui devra être non-fonctionnel.

De plus, l'intégration d'un transgène en amont ou en aval d'un gène essentiel à la réplication comme décrit par Possee est susceptible de générer des virus dont la réplication pourra être altérée, et donc des virus se répliquant insuffisamment, c'est-à-dire présentant dans le surnageant de culture un taux de particules virales infectieuses (PFU/ml) nettement plus faible qu'un virus sauvage.

Partant de ce constat, le demandeur a mis au point un procédé particulièrement efficace et facile à mettre en œuvre pour préparer des baculovirus recombinants homogènes et stables, et qui permet d'envisager un développement au niveau industriel de la production de protéines recombinantes, par exemple de protéines multimériques comprenant plusieurs sous-unités distinctes.

### Résumé de l'invention

Dans un premier aspect l'invention concerne un procédé pour produire un baculovirus recombinant dont le génome comprend un ou plusieurs transgène(s) codant chacun une enzyme de maturation des protéines et n transgènes codant chacun un polypeptide d'intérêt, ledit procédé comprend les étapes de :
a) Préparer, dans une cellule d'insecte, un génome de baculovirus capable de se répliquer qui comprend un ou plusieurs transgène(s) codant chacun une enzyme de maturation des protéines et n transgènes codant chacun un polypeptide d'intérêt, par recombinaison homologue entre :
   a1) un génome de baculovirus recombinant déficient pour la réplication dans lequel n gènes essentiels à la réplication virale sont non-fonctionnels et qui comprend un ou plusieurs transgène(s) codant chacun une enzyme de maturation des protéines, et
   a2) n vecteurs de transfert comprenant chacun:
      i) une séquence nucléotidique permettant de restaurer la fonction d'un des n gènes essentiels à la réplication virale non-fonctionnel,
      ii) un des n transgènes codant un polypeptide d'intérêt,
   l'ensemble des séquences nucléotidiques i) des n vecteurs de transfert étant capables de restaurer la réplication du génome de baculovirus déficient pour la réplication,
   n étant un nombre entier au moins égal à 2 ; et
b) générer un baculovirus recombinant dans une cellule d'insecte qui comprend le génome de baculovirus recombinant obtenu à l'étape a),
ledit procédé étant caractérisé en ce que la recombinaison se fait en une seule étape dans la cellule d'insecte.

Est également ici décrit un baculovirus recombinant ou un génome de baculovirus recombinant comprenant :
a) n séquences nucléotidiques de formule (I) :
   [transgène codant un polypeptide d'intérêt]-[séquence nucléotidique intercalaire]-[gène essentiel à la réplication virale fonctionnel] (I),
   ladite séquence d'acides nucléiques intercalaire est constituée de 0 à 600 pb, de préférence de 1 à 600 paires de bases,
   ledit gène essentiel à la réplication virale fonctionnel est sélectionné parmi 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), helicase (ORF95), Vp80 (ORF104), ORF106-107, odv-ec43 (ORF109), gp64/67 (ORF128), ORF132, ORF133, odv-ec27 (ORF144), ORF146, ie1 (ORF147), lef-2 (ORF6);
   n étant un nombre entier au moins égal à 2 ; et
b) un ou plusieurs transgène(s) codant chacun une enzyme de maturation des protéines.

Est également ici décrit un jeu d'éléments de recombinaison homologue comprenant :
a) un génome de baculovirus déficient pour la réplication dans lequel n gènes essentiels à la réplication virale sont non-fonctionnels et qui comprend un ou plusieurs transgène(s) codant chacun une enzyme de maturation des protéines ;
b) n vecteurs de transfert comprenant chacun:
   i) une séquence d'acides nucléiques permettant de restaurer la fonction d'un des n gènes essentiels à la réplication virale non-fonctionnel,
   ii) un transgène codant un polypeptide d'intérêt,
n étant un nombre entier au moins égal à 2.

La présente description concerne aussi une cellule comprenant un baculovirus recombinant ou un génome de baculovirus recombinant ou un jeu d'éléments de recombinaison homologue.

La présente description concerne aussi l'utilisation d'un baculovirus recombinant ou d'un génome de baculovirus recombinant ou d'une cellule telle que décrite ci-dessuspour la production de n polypeptides d'intérêt.

### Description détaillée de l'invention

### Définitions

Dans le cadre de la présente invention, on entend désigner par l'expression « baculovirus » un virus en forme de bâtonnet spécifique des arthropodes. Un baculovirus comprend généralement une nucléocapside renfermant un génome de baculovirus. Des exemples de baculovirus sont BmNPV, AcMNPV, ApNPV, BsSNPV, CfMNPV, EoSNPV, HaNPV, HzNPV, LdMNPV, MbMNPV, OpMNPV, SIMNPV, SeMNPV et TeNPV.

Dans le cadre de la présente invention, on entend désigner par l'expression « génome de baculovirus » l'ensemble du matériel génétique d'un baculovirus, comprenant en particulier toutes les séquences nucléotidiques codantes et non codantes d'un baculovirus.

Dans le cadre de la présente invention, on entend désigner par l'expression « génome de baculovirus déficient pour la réplication », un génome de baculovirus dans lequel au moins deux gènes essentiels à la réplication virale ont été soit délétés (entièrement ou partiellement) soit mutés de telle sorte que le génome de baculovirus a perdu sa capacité de réplication dans une cellule d'insecte. Par exemple, le gène essentiel à la réplication virale ne s'exprime plus ou il est transcrit puis traduit en une protéine non-fonctionnelle. Ainsi, les gènes délétés (entièrement ou partiellement) ou mutés sont appelés « gènes essentiels à la réplication virale non-fonctionnels ». Les génomes de baculovirus déficients pour la réplication virale sont fabriqués à partir de génomes de baculovirus parents en utilisant des techniques de biologie moléculaire bien connues de l'homme du métier, et permettant notamment l'insertion et/ou la délétion de séquences nucléotidiques dans le génome de baculovirus parent. De préférence, le génome de baculovirus déficient pour la réplication virale comprend au moins une séquence nucléotidique qui lui permet de se répliquer au sein d'une cellule bactérienne. Les séquences nucléotidiques permettant la réplication au sein d'une cellule bactérienne ne sont pas des transgènes d'intérêt au sens de l'invention. Un exemple d'élément de réplication bactérienne est la séquence nucléotidique « Mini-F ». De tels éléments de réplication sont bien connus dans l'état de la technique. La cellule bactérienne peut être *Escherichia coli.* Un génome de baculovirus qui comprend une séquence nucléotidique qui lui permet de se répliquer au sein d'une cellule bactérienne est connu sous la dénomination « Bacmid ». De préférence, le génome de baculovirus déficient pour la réplication comprend également une ou plusieurs séquences nucléotidiques codant un ou plusieurs marqueurs de sélection permettant de sélectionner ou d'identifier les cellules bactériennes transfectées par le génome de baculovirus déficient pour la réplication. Les séquences nucléotidiques de sélection ne sont pas des transgènes d'intérêt au sens de l'invention. Il peut s'agir par exemple du gène de résistance à l'ampicilline, du gène de résistance à la kanamycine, du gène de résistance à l'hygromycine, du gène de résistance à la zéocine et/ou du gène de résistance à la tétracycline.

Dans le cadre de la présente invention, on entend désigner par l'expression « génome de baculovirus recombinant », un génome de baculovirus qui comprend un ou plusieurs transgène(s) codant chacun une enzyme de maturation des protéines et n transgènes codant chacun un polypeptide d'intérêt. Le terme « génome de baculovirus recombinant » selon l'invention correspond au génome de baculovirus obtenu par la mise en œuvre du procédé selon l'invention, c'est-à-dire le génome de baculovirus obtenu par recombinaison homologue entre le génome de baculovirus déficient pour la réplication et n vecteurs de transfert.

Dans le cadre de la présente invention, on entend désigner par l'expression « baculovirus recombinant », un baculovirus dont le génome est un génome de baculovirus recombinant, c'est-à-dire un baculovirus dont le génome comprend un ou plusieurs transgène(s) codant chacun une enzyme de maturation des protéines et n transgènes codant chacun un polypeptide d'intérêt. Le baculovirus recombinant peut être produit après réplication du génome de baculovirus recombinant dans une cellule d'insecte. Le baculovirus recombinant est capable d'infecter des cellules d'insecte. De préférence, le baculovirus recombinant est infectieux pour une cellule d'insecte.

**Par** « **gène »,** on entend une séquence nucléotidique capable d'être transcrite puis traduite en polypeptide, par exemple en protéine. On parle alors de gène codant un polypeptide.

Dans le cadre de la présente invention, on entend par « transgène », un gène qui n'est pas naturellement présent dans le génome d'un baculovirus. Il peut s'agir par exemple d'un gène d'origine humaine, d'un gène d'origine animale, d'un gène d'origine végétale, d'un gène d'origine virale ou d'un gène d'origine bactérienne. Dans le cadre de l'invention, le transgène est soit un « transgène codant une enzyme de maturation des protéines », soit un « transgène codant un polypeptide d'intérêt ».

On entend par « transgènes distincts », des transgènes n'ayant pas la même séquence nucléotidique.

Un transgène au sens de la présente invention est placé sous le contrôle d'éléments appropriés pour son expression dans la cellule d'insecte. Par "éléments appropriés", on entend l'ensemble des éléments nécessaires à sa transcription en ARN messager (ARNm) et à la traduction de l'ARNm en polypeptide. Parmi les éléments nécessaires à la transcription, le promoteur revêt une importance particulière. Il peut s'agir d'un promoteur constitutif ou d'un promoteur régulable et il peut être d'origine baculovirale ou d'origine arthropode (e.g. d'origine insecte). L'important est que le promoteur choisi soit adapté pour l'expression du transgène dans la cellule d'insecte. D'une façon générale, un promoteur en usage dans la présente invention, peut être modifié de manière à contenir des séquences régulatrices. A titre d'exemples de promoteurs, on peut citer le promoteur polyédrine, le promoteur P10, les promoteurs synthétiques dérivés des promoteurs polyédrine et P10, le promoteur IE1 du baculovirus CfMNPV, le promoteur IE1 du baculovirus LdMNPV, le promoteur gp64 du baculovirus OpMNPV, le promoteur IE1 du virus de crevette WSSV (White spot syndrome virus), le promoteur P9 du densovirus de *Junonia coenia* (JcDNV), le promoteur cellulaire A3 (actine 3) du vers à soie *Bombyx mori.* Dans un mode de réalisation particulier, un ou plusieurs des transgènes est placé sous le contrôle d'un promoteur synthétique dérivé du promoteur P10 sauvage (SEQ ID NO : 1), de préférence le promoteur synthétique P10S1A (SEQ ID NO : 2) ou P10S1B (SEQ ID NO : 3).

Par « cassette d'expression », on entend une séquence nucléotidique généralement constituée d'un ou plusieurs gènes et des éléments appropriés pour son/leur expression, par exemple un transgène et les éléments appropriés pour son expression dans la cellule d'insecte.

Par « enzyme de maturation des protéines », on entend une enzyme impliquée dans la maturation des protéines. En particulier, la maturation opérée par une enzyme de maturation des protéines conduit à la production d'une protéine stable et/ou ayant toutes ou partie de son activité biologique. Par exemple, l'enzyme de maturation des protéines peut agir au niveau de la séquence peptidique d'une protéine (par exemple par clivage), au niveau du repliement (c'est le cas par exemple des protéines chaperonnes), au niveau de la glycosylation, ou au niveau de toute autre modification post-traductionnelle comme par exemple la phosphorylation ou la méthylation. L'enzyme de maturation des protéines peut être une peptidase signal, une furine, une proprotéine convertase, une glycosyltransférase, une glycosidase, une protéine chaperone, une disulfide isomérase, une acyltransférase, une méthyltransférase, une hydroxylase, une transglutaminase, une farnésyltransférase, une géranylgéranyltransférase, une N-myristoyltransférase, une palmityltransférase, une protéine kinase, une phosphatase, une transpeptidase, une carboxylase et/ou une ubiquitine ligase.

Par « glycosyltransférase », on entend une enzyme capable de catalyser le transfert d'un monosaccharide, depuis un sucre activé (donneur), généralement par un phosphate, vers une molécule accepteur (le plus souvent un alcool ou une amine). L'accepteur du transfert peut aussi être un résidu peptidique, le plus souvent la sérine, la thréonine ou plus rarement la tyrosine, l'hydroxylysine et hydroxyproline lors des O-glycosylations (O-mannose, O-fucose, O-GalNAc, O-GlcNAc, O-galactose et O-glucose), ou une asparagine lors d'une N-glycosylation. Un mannose activé peut également être transféré sur un tryptophane pour former un C-mannosyl tryptophane. La glycosyltransférase peut être choisie parmi les N-acétylglucosaminyltransférases I, II, III, IV, V, VB, VI et IX, une galactosyltransférase, par exemple une béta-1,4-galactosyltransférase, par exemple choisie parmi la béta-1,4-galactosyltransférase 1, 2, 3, 4, 5, 6 et 7, la CMP-NeuAc synthase, la NeuAc synthase, les protéine-O-mannosyltransférases 1 et 2, les protéine-O-fucosyltransférases 1 et 2, la protéine-O-glucosyltransférase 1, la protéine-O-GlcNAc transférase, la GalNAc transférase, les fucosyltransférases 1, 2, 3, 4, 5, 6, 7, 8, 9, 10 et 11 (FUT1 à FUT11) et les sialyltransférases, par exemple l'α2,3 sialyltransférase et l'α2,6 sialyltransférase.

On entend par « polypeptide » une chaine d'acides aminés reliés par des liaisons peptidiques. Par exemple, un polypeptide peut être une protéine, une sous-unité de protéine, un fragment de protéine ou simplement une chaine d'acides aminés. Généralement, un polypeptide est formé d'au moins 10 acides aminés.

Par « polypeptide d'intérêt » ou « polypeptide recombinant d'intérêt », on entend un polypeptide codé par un transgène. Par exemple, le polypeptide d'intérêt peut être une sous-unité d'une protéine multimérique. Avantageusement, il s'agit d'un polypeptide d'intérêt thérapeutique et/ou diagnostique, c'est-à-dire un polypeptide qui peut être utilisé en thérapie ou en diagnostique.

On entend par « polypeptides d'intérêt distincts », des polypeptides n'ayant pas la même séquence en acides aminés.

On entend par « sous-unités distinctes », des sous-unités d'une protéine multimérique n'ayant pas la même séquence en acides aminés. Ainsi, une « protéine comprenant n sous-unités distinctes » est une protéine qui comprend n sous-unités ayant chacune une séquence en acides aminés propre qui sont liées entre elles par des liaisons non-covalentes et/ou des liaisons covalentes.

Une « protéine multimérique » est une protéine comprenant plusieurs sous-unités. Une protéine multimérique peut comprendre plusieurs sous-unités identiques (protéine homomultimérique) ou plusieurs sous-unités distinctes (protéine hétéromultimérique).

On entend par « complexe protéique » un ensemble constitué de plusieurs protéines ayant un lien fonctionnel ou structurel entre elles, par exemple une « Virus-Like-Particle » (VLP) ou un complexe multienzymatique.

Au sens de la présente invention, l'expression « réplication » ou « réplication virale » s'entend à la fois par la réplication du génome de baculovirus et du baculovirus. Etant entendu que la réplication du génome de baculovirus dans une cellule d'insecte est essentielle à la réplication du baculovirus dans la cellule d'insecte. Ainsi, un gène essentiel à la réplication virale s'entend comme un gène essentiel à la réplication du baculovirus dans la cellule d'insecte. La réplication du baculovirus dans la cellule d'insecte permet la génération de baculovirus infectieux. Ainsi, le procédé de l'invention permet de générer un baculovirus recombinant infectieux dans une cellule, notamment une cellule d'insecte.

Dans le cadre de la présente invention, on entend désigner par l'expression « recombinaison homologue », l'échange d'information génétique entre deux séquences nucléotidiques différentes, nécessitant la présence de séquences homologues entre les deux séquences nucléotidiques différentes.

Le terme « anticorps » est utilisé ici dans le sens le plus large et englobe diverses structures d'anticorps largement décrites dans la littérature, y compris, mais sans s'y limiter, les anticorps quelle que soit leur origine, les anticorps monoclonaux, les anticorps polyclonaux et les fragments d'anticorps tant qu'ils présentent l'activité souhaitée (par exemple la liaison à l'antigène). Il peut s'agir d'un anticorps mono-spécifique ou multi-spécifique, par exemple bispécifique. L'anticorps peut être une IgA, IgD, IgE, IgG ou une IgM. Des exemples de fragments d'anticorps comprennent, mais sans s'y limiter, les fragments Fv, Fab, Fab', F(ab')2 ; les diabodies; les scFv/Fc ; les anticorps de type camélidés (par exemple les VHH); les molécules d'anticorps à chaîne unique (par exemple scFv).

### Procédé de préparation d'un baculovirus recombinant

L'invention concerne un procédé pour produire un baculovirus recombinant dont le génome comprend un ou plusieurs transgène(s) codant chacun une enzyme de maturation des protéines et n transgènes codant chacun un polypeptide d'intérêt, ledit procédé comprend les étapes de :
a) Préparer, dans une cellule d'insecte, un génome de baculovirus capable de se répliquer qui comprend un ou plusieurs transgène(s) codant chacun une enzyme de maturation des protéines et n transgènes codant chacun un polypeptide d'intérêt, par recombinaison homologue entre :
   a1) un génome de baculovirus déficient pour la réplication dans lequel n gènes essentiels à la réplication virale sont non-fonctionnels et qui comprend un ou plusieurs transgène(s) codant chacun une enzyme de maturation des protéines, et
   a2) n vecteurs de transfert comprenant chacun:
      i) une séquence nucléotidique permettant de restaurer la fonction d'un des n gènes essentiels à la réplication virale non-fonctionnel,
      ii) un des n transgènes codant un polypeptide d'intérêt,
   l'ensemble des séquences nucléotidiques i) des n vecteurs de transfert étant capables de restaurer la réplication du génome de baculovirus déficient pour la réplication,
   n étant un nombre entier au moins égal à 2 ; et
b) Générer un baculovirus recombinant dans une cellule d'insecte qui comprend le génome de baculovirus recombinant obtenu à l'étape a),
ledit procédé étant caractérisé en ce que la recombinaison se fait en une seule étape dans la cellule d'insecte.

Les n transgènes codant chacun un polypeptide d'intérêt sont portés par les n vecteurs de transfert qui recombinent avec le génome de baculovirus déficient pour la réplication virale dans lequel n gènes essentiels à la réplication virale sont non-fonctionnels et qui comprend un ou plusieurs transgène(s) codant chacun une enzyme de maturation des protéines. Après recombinaison, les n transgènes codant chacun un polypeptide d'intérêt se retrouvent intégrés dans le génome du baculovirus recombinant.

### Etape a)

La recombinaison se fait dans une cellule d'insecte entre (a1) un génome de baculovirus déficient pour la réplication dans lequel n gènes essentiels à la réplication virale sont non-fonctionnels et qui comprend un ou plusieurs transgène(s) codant chacun une enzyme de maturation des protéines et (a2) les n vecteurs de transfert.

La recombinaison dans le cadre de la présente invention se fait en une seule étape dans la cellule d'insecte, et ce quel que soit le nombre n de transgènes à intégrer. C'est-à-dire que la recombinaison des n transgènes codant chacun un polypeptide d'intérêt avec le génome de baculovirus déficient pour la réplication se fait de manière simultanée ou quasi-simultanée dans la cellule d'insecte. Cette recombinaison simultanée est un des principaux avantages du procédé selon l'invention car il permet de produire le génome de baculovirus recombinant désiré rapidement et en une seule étape.

Dans un mode de réalisation particulier, le génome de baculovirus déficient pour la réplication est obtenu à partir d'un génome de baculovirus sélectionné parmi ou dérivé du génome de BmNPV, AcMNPV, ApNPV, BsSNPV, CfMNPV, EoSNPV, HaNPV, HzNPV, LdMNPV, MbMNPV, OpMNPV, SIMNPV, SeMNPV ou TeNPV, de préférence AcMNPV.

Dans un mode de réalisation préféré, le génome de baculovirus déficient pour la réplication mis en œuvre dans le procédé est sous forme circulaire. Ainsi, le procédé selon l'invention ne nécessite pas la linéarisation du génome déficient pour la réplication.

Les vecteurs de transfert peuvent également contenir une ou plusieurs séquences nucléotidiques qui leur permettent de se répliquer au sein d'une cellule bactérienne. Ils peuvent également contenir des gènes codant un marqueur de sélection permettant de sélectionner ou identifier les cellules bactériennes transformées avec un vecteur de transfert.

L'un des principaux avantages du procédé de l'invention est que la capacité à se répliquer du génome de baculovirus déficient pour la réplication virale est restaurée par la recombinaison avec les n vecteurs de transfert. En effet, chacun des n vecteurs de transfert code, en plus d'un des n transgènes codant un polypeptide d'intérêt, une séquence nucléotidique permettant de restaurer la fonction d'un des n gènes essentiels à la réplication virale non-fonctionnels. Ainsi, seule une recombinaison avec l'ensemble des n vecteurs de transfert permet de restaurer la réplication du génome de baculovirus déficient pour la réplication. Ainsi le procédé de l'invention garantit que seuls les génomes de baculovirus recombinants contenant les n transgènes codant un polypeptide d'intérêt pourront générer des baculovirus recombinants infectieux. Ce procédé évite d'avoir à utiliser des tests coûteux et chronophages pour identifier les baculovirus recombinants contenant les n transgènes codant un polypeptide d'intérêt.

Dans un mode de réalisation préféré, les gènes essentiels à la réplication virale sont choisis parmi 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), hélicase (ORF95), vp80 (ORF104), ORF106-107, odv-ec43 (ORF109), gp64/67 (ORF128), ORF132, ORF133, odv-ec27 (ORF144), ORF146, ie1 (ORF147) et lef-2 (ORF6). Ces gènes sont préférés car ils sont adjacents à un gène non-essentiel à la réplication virale. Ainsi, dans un mode de réalisation préféré, dans le génome de baculovirus déficient pour la réplication virale, les n gènes essentiels à la réplication virale non-fonctionnels sont chacun adjacents à un gène non-essentiel à la réplication virale. Comme détaillé tout au long de la présente demande, les n transgènes recombinent chacun au niveau d'un gène non-essentiel à la réplication virale adjacent à un gène essentiel à la réplication virale non-fonctionnel. Etant donné que le gène non-essentiel à la réplication virale n'est pas essentiel pour la réplication du génome de baculovirus, la recombinaison n'affecte pas la capacité du génome de baculovirus recombinant à se répliquer.

Les n transgènes sont intégrés soit au sein d'un gène non-essentiel à la réplication virale soit dans une zone intergénique, entre 2 gènes non essentiels, ou encore en amont ou en aval d'un gène essentiel à la réplication virale.

Lorsque le transgène est intégré en amont ou en aval d'un gène essentiel (construction du BacMid2-GNTII-ß1,4GT-CMPNeuAcS-NeuAcS-ST3 (ou BacSia3) présenté dans l'exemple 12, avec l'intégration du gène ST3 en aval de orf51), les baculovirus ainsi générés, bien que viables, peuvent néanmoins présenter une réplication altérée et ainsi des titres viraux relativement faibles.

Selon une mise en œuvre préférée de l'invention, les n transgènes sont intégrés au sein de n gènes non-essentiels à la réplication virale, ce qui permet d'obtenir des baculovirus bien plus stables au cours des cycles de réplication, et ainsi d'obtenir des titres viraux suffisants pour envisager une production industrielle.

Des exemples d'intégration d'un transgène au sein d'un gène non essentiel à la réplication virale sont présentés dans les exemples, notamment dans l'Exemple 9, avec l'intégration du gène *fur* dans les gènes *Chit*/*Cath,* dans l'Exemple 10, avec l'intégration du gène β1,4GalT dans le gène *egt,* dans l'Exemple 12 avec l'intégration des gènes NeuAc synthase et CMP-NeuAc synthase dans le gène *iap2* et dans l'exemple 14 relatif au clonage du transgène α2,6-sialyltransferase I (ST6GalI) dans l'ORF119 (PIF1) du BacMid2-GNTII- ß1,4GT-CMPNeuAcS-NeuAcS.

Au sens de la présente invention, un gène essentiel à la réplication virale non-fonctionnel est adjacent à un gène non-essentiel à la réplication virale lorsque les deux gènes se succèdent ou se recouvrent partiellement sur le génome du baculovirus, de préférence aucun autre gène n'est compris entre le gène essentiel à la réplication virale non-fonctionnel et le gène non-essentiel à la réplication virale. Avantageusement, les deux gènes susmentionnés sont séparés par une séquence nucléotidique intercalaire, par exemple une séquence nucléotidique intercalaire non codante. En particulier, la séquence nucléotidique intercalaire a une longueur allant de 1 pb à 600 pb. Il est également possible qu'aucune séquence nucléotidique intercalaire (i.e. 0 pb) ne sépare les deux gènes susmentionnés, c'est-à-dire que les deux gènes susmentionnés sont accolés sur le génome du baculovirus ou se recouvrent partiellement. Alternativement, la séquence nucléotidique intercalaire peut comprendre un gène non-essentiel à la réplication virale.

Le demandeur s'est aperçu que le choix de gènes essentiels à la réplication virale non-fonctionnels adjacents à des gènes non-essentiels à la réplication virale était particulièrement avantageux et permettait d'obtenir des recombinaisons homologues homogènes et donc d'obtenir des génomes baculovirus recombinants homogènes. Comme expliqué par ailleurs, seule une parfaite recombinaison des n vecteurs permet d'obtenir un génome de baculovirus recombinant capable de se répliquer dans une cellule d'insecte. Ainsi, les génomes des baculovirus recombinants préparés par le procédé selon l'invention sont homogènes à plus de 90%, avantageusement à plus de 95%, de préférence à plus de 99% et de manière tout à fait préférée à environ 100%, par exemple les génomes de baculovirus recombinants préparés par le procédé selon l'invention sont tous identiques.

Dans un mode de réalisation particulier, le gène non-essentiel à la réplication virale est choisi parmi Ph (ORF 8), ORF11, ORF13, egt (ORF15), v-ubiquitin (ORF35), 39K (ORF36), ORF38, p43 (ORF39), lef-12 (ORF41), pcna (ORF49), ORF52, ORF55, Fp (ORF61), ORF63, gp37 (ORF64), ORF68, ORF72, ORF74, ORF82, cg30 (ORF88), ORF91, pif-4 (ORF96), he65 (ORF105), ORF108, ORF110, cathepsine (ORF127), p24 (ORF129), pp34 (ORF131), ORF134, ORF145, odv-e56 (ORF148), ORF5.

Avantageusement, le couple gène essentiel à la réplication virale/gène non-essentiel à la réplication virale est choisi parmi les couples listés dans le tableau 1 suivant :

**Tableau 1. Couples de gènes adjacents comprenant un gène essentiel et un gène non essentiel à la réplication virale**

| **Couple** | **Gène essentiel** | **Gène non essentiel** | **Espacement** entre les 2 gènes |
|---|---|---|---|
| 1 | 1629 (ORF9) | Ph (ORF 8) | 29 bp |
| 2 | Pk1 (ORF10) | ORF11 | 163 bp |
| 3 | lef-1 (ORF14) | ORF13 | Chevauchement |
| | | ou egt (ORF15) | 112 bp |
| 4 | ORF34 | v-ubiquitin (ORF35) | 20 bp |
| 5 | lef-11 (ORF37) | 39K (ORF36) | Chevauchement |
| | | ou ORF38 | Chevauchement |
| 6 | p47 (ORF40) | p43 (ORF39) | 7 bp |
| | | ou lef-12 (ORF41) | Chevauchement |
| 7 | Lef8 (ORF50) | pcna (ORF49) | 109 bp |
| 8 | DNAJ domain (ORF51) | ORF52 | 202 bp |
| 9 | ORF53 | ORF52 | 1 bp |
| 10 | vp1054 (ORF54) | ORF55 | 91 bp |
| 11 | Lef-9 (ORF62) | Fp (ORF61) | 26 bp |
| | | ou ORF63 | 60 bp |
| 12 | DNA Pol (ORF65) | qp37(ORF64) | 137 bp |
| 13 | lef-3 (ORF67) | ORF68 | Chevauchement |
| 14 | ORF73 | ORF72 | 8 bp |
| 15 | ORF75 | ORF74 | 17 bp |
| 16 | ORF81 | ORF82 | Chevauchement |
| 17 | p95 (ORF83) | ORF82 | Chevauchement |
| 18 | vp39 (ORF89) | cg30 (ORF88) | 2 bp |
| 19 | lef-4 (ORF90) | ORF91 | 0 bp |
| 20 | p33 (ORF92) | ORF91 | 37 bp |
| 21 | helicase (ORF95) | pif-4 (ORF96) | Chevauchement |
| 22 | vp80 (ORF104) | he65 (ORF105) | 27 bp |
| 23 | ORF106-107 | he65 (ORF105) | 544 bp |
| 24 | odv-ec43 (ORF109) | ORF108 | 11 bp |
| | | ou ORF110 | 35 bp |
| 25 | gp64/67 (ORF128) | cathepsine (ORF127) | 224 bp |
| | | ou p24 (ORF129) | 182 bp |
| 26 | ORF132 | pp34 (ORF131) | 211 bp |
| 27 | ORF133 | ORF134 | 50 bp |
| 28 | odv-ec27 (ORF144) | ORF145 | 69 bp |
| 29 | ORF146 | ORF145 | Chevauchement |
| 30 | ie1 (ORF147) | odv-e56 (ORF148) | 61 bp |
| 31 | lef-2 (ORF6) | ORF5 | Chevauchement |

Les lignes grisées représentent les gènes essentiels non-fonctionnels dans les baculovirus des exemples 1, 2 et 3.

Dans un mode de réalisation avantageux, les n séquences nucléotidiques permettant de restaurer la fonction des n gènes essentiels à la réplication virale non-fonctionnels recombinent chacune avec un gène essentiel à la réplication virale non-fonctionnel tel que listé dans le tableau 1; tandis que les n transgènes codant un polypeptide d'intérêt recombinent chacun au niveau d'un gène non-essentiel à la réplication virale, ledit gène non-essentiel étant le gène adjacent audit gène essentiel dont la fonction a été restaurée lors de cette étape de recombinaison homologue, tel que présenté dans le tableau 1.

Ainsi, lorsque le gène essentiel dont la fonction est restauré est le gène 1629 (ORF9), le transgène codant pour un peptide d'intérêt sera intégré dans le gène non essentiel Ph (ORF8) ; lorsque le gène essentiel dont la fonction est restauré est le gène Pk1 (ORF10), le transgène codant pour un peptide d'intérêt sera intégré dans le gène non essentiel ORF11, et ainsi de suite pour chaque couple de gènes adjacents listés dans le tableau 1.

Le procédé selon l'invention met en œuvre un mécanisme de recombinaison homologue intermoléculaire. D'une manière générale, le mécanisme de recombinaison homologue consiste en l'échange de séquences nucléotidiques homologues entre le génome de baculovirus déficient pour la réplication et les n vecteurs de transfert. Ces séquences nucléotidiques peuvent être identiques ou substantiellement homologues.

Dans un mode de réalisation particulièrement avantageux, les vecteurs de transfert comprennent, de part et d'autre de la cassette d'expression du transgène codant un polypeptide d'intérêt, des séquences flanquantes homologues au génome de baculovirus déficient pour la réplication. Le degré d'homologie des séquences flanquantes avec la partie correspondante du génome de baculovirus déficient pour la réplication peut être variable mais doit être suffisant pour permettre la recombinaison intermoléculaire. Aux fins de la présente invention, il est préférable qu'il soit supérieur à 70%, avantageusement supérieur à 80%, de préférence supérieur à 90% et de manière tout à fait préférée aux environs de 100%, de préférence identique. De plus, une courte région d'homologie peut être suffisante pour permettre la recombinaison intermoléculaire, c'est-à-dire au moins 10 nucléotides (ou paires de bases) consécutifs communs entre les séquences flanquantes et leurs séquences homologues dans le génome de baculovirus déficient pour la réplication. Dans le cadre de la présente invention, la longueur des séquences flanquantes peut aller de 10 pb (i.e. 10 paires de bases) à 10 kb (i.e. 10 000 paires de bases), avantageusement de 100 pb à 6 kb, de préférence de 200 pb à 6 kb et, de manière tout à fait préférée de 400 pb à 6 kb. Ainsi, le matériel génétique localisé entre les séquences flanquantes des n vecteurs de transfert remplace le matériel génétique localisé entre les deux séquences homologues aux séquences flanquantes du génome de baculovirus déficient pour la réplication. Cet échange intermoléculaire permet d'obtenir un génome de baculovirus recombinant capable de générer un baculovirus recombinant infectieux dans la cellule d'insecte.

Selon l'invention, l'ensemble des séquences nucléotidiques i) (i.e. les séquences nucléotidiques permettant de restaurer la fonction des n gènes essentiels à la réplication virale non-fonctionnels) des n vecteurs de transfert sont capables de restaurer la réplication du génome de baculovirus déficient pour la réplication. En effet, l'échange intermoléculaire permet de restaurer la fonction des n gènes essentiels à la réplication virale non-fonctionnels. En d'autres termes, la restauration de la fonction des n gènes essentiels à la réplication virale non-fonctionnels se fait lorsque la recombinaison homologue se produit correctement. Cela vient du fait que les n vecteurs de transfert comprennent chacun une séquence nucléotidique permettant de restaurer la fonction d'un des n gènes essentiels à la réplication virale non-fonctionnels.

Par exemple, lorsque n=2, un génome de baculovirus déficient pour la réplication dans lequel deux gènes essentiels à la réplication virale sont non-fonctionnels recombine avec deux vecteurs de transfert qui comprennent chacun une séquence nucléotidique permettant de restaurer la fonction d'un des deux gènes essentiels à la réplication virale non-fonctionnel. Ce qui veut dire que la recombinaison avec le premier vecteur de transfert permet de restaurer la fonction d'un premier gène essentiel à la réplication virale non-fonctionnel et la recombinaison avec le deuxième vecteur de transfert permet de restaurer la fonction du deuxième gène essentiel à la réplication virale non-fonctionnel. Ainsi, seule la recombinaison des deux vecteurs de transfert avec le génome de baculovirus déficient pour la réplication permet de restaurer la fonction des deux gènes essentiels à la réplication virale non-fonctionnels et donc de restaurer la réplication du génome de baculovirus déficient pour la réplication. Cette restauration de la fonction des deux gènes essentiels permet d'obtenir un génome de baculovirus recombinant capable de générer des baculovirus recombinants infectieux dans la cellule d'insecte.

Ainsi, selon le procédé de l'invention, la recombinaison avec les n vecteurs de transfert, ou multirecombinaison, est nécessaire pour restaurer la réplication du génome de baculovirus déficient pour la réplication.

De manière surprenante, les inventeurs ont mis en évidence que la multirecombinaison pouvait se faire en une seule étape, de manière simultanée, dans la cellule d'insecte. Ceci est particulièrement avantageux puisque le génome de baculovirus déficient pour la réplication et les n vecteurs de transfert peuvent être introduits en même temps dans la cellule d'insecte, c'est-à-dire que le génome de baculovirus déficient pour la réplication et les n vecteurs de transfert sont introduits simultanément dans la cellule d'insecte, en d'autres termes le génome de baculovirus déficient pour la réplication et les n vecteurs de transfert sont introduits en une seule étape dans la cellule d'insecte, et ce quel que soit le nombre n de vecteurs de transfert. La multirecombinaison en une seule étape permet d'obtenir facilement et rapidement des génomes de baculovirus recombinants homogènes.

Le procédé selon l'invention permet de produire un baculovirus recombinant qui comprend n transgènes codant chacun un polypeptide d'intérêt. L'ensemble des n polypeptides d'intérêt peuvent former, par exemple, une protéine comprenant plusieurs sous-unités. L'ensemble des n polypeptides d'intérêt peuvent également être les protéines constitutives d'un complexe protéique, par exemple d'une VLP. L'ensemble des n polypeptides d'intérêt sont produits par une cellule d'insecte infectée par le baculovirus recombinant qui comprend les n transgènes codant chacun un polypeptide d'intérêt.

Ainsi, dans un mode de réalisation particulier, les n polypeptides d'intérêt forment plusieurs protéines d'intérêt distinctes. Il peut s'agir de plusieurs protéines d'intérêt distinctes comprenant une seule chaine polypeptidique, de plusieurs protéines d'intérêt distinctes comprenant plusieurs sous-unités identiques et/ou de plusieurs protéines d'intérêt distinctes comprenant plusieurs sous-unités distinctes. Le nombre de protéines d'intérêt distinctes formées par les n polypeptides d'intérêt sera égal ou inférieur à n. Par exemple, trois polypeptides d'intérêt (n=3) peuvent former (i) une première protéine d'intérêt comprenant une seule chaine polypeptidique et une seconde protéine d'intérêt comprenant deux sous-unités distinctes, (ii) trois protéines d'intérêt distinctes comprenant chacune une seule chaine polypeptidique, (iii) une première protéine d'intérêt comprenant plusieurs sous-unités identiques et une seconde protéine d'intérêt comprenant deux sous-unités distinctes, ou (iv) trois protéines d'intérêt distinctes comprenant chacune plusieurs sous-unités identiques.

Dans un autre mode de réalisation particulier, les n polypeptides d'intérêt forment une seule protéine. Dans ce mode de réalisation, la protéine comprend alors n sous-unités distinctes, chacune des sous-unités étant un des n polypeptides d'intérêt.

Le procédé selon l'invention est donc particulièrement avantageux pour préparer un baculovirus recombinant qui comprend des transgènes codant une protéine d'intérêt comprenant plusieurs sous-unités distinctes, par exemple une protéine d'intérêt qui n'est active que lorsqu'elle comprend toutes ses sous-unités. Les sous-unités étant généralement liées entre elles par des liaisons non-covalentes (e.g. liaisons hydrophobes) et/ou des liaisons covalentes (e.g. ponts disulfures entre deux cystéines). Le procédé de l'invention est donc particulièrement avantageux pour préparer un baculovirus recombinant qui comprend des transgènes codant une protéine multimérique, par exemple un anticorps ou un fragment d'anticorps.

Le nombre de vecteurs de transfert dépendra du nombre souhaité de polypeptides d'intérêt distincts que l'on souhaite produire. Par exemple, deux vecteurs de transfert seront utilisés pour une protéine comprenant deux sous-unités distinctes, trois vecteurs de transfert seront utilisés pour une protéine comprenant trois sous-unités distinctes, etc. Avantageusement, chaque vecteur de transfert comprend un transgène codant un polypeptide d'intérêt différent des transgènes codant les autres polypeptides d'intérêt compris dans les autres vecteurs de transfert. Dans un mode de réalisation particulier, un vecteur de transfert peut comprendre plus d'un transgène, par exemple deux transgènes, codant chacun un polypeptide d'intérêt. Dans ce mode de réalisation particulier, les transgènes peuvent être présents dans un même locus, de préférence au maximum deux transgènes par locus.

Avantageusement, n est un nombre entier allant de 2 à 31, par exemple allant de 2 à 10. Par exemple, pour une protéine d'intérêt comprenant plusieurs sous-unités distinctes, la valeur de n correspondra au nombre de sous-unités distinctes de ladite protéine d'intérêt.

Dans un mode de réalisation particulier, n=2. Dans ce cas, on peut distinguer les mises en œuvre suivantes :
- les deux transgènes codent chacun une sous-unité d'une protéine comprenant deux sous-unités, avantageusement les deux transgènes codent chacun une sous-unité distincte d'une protéine comprenant deux sous-unités distinctes. Par exemple, le premier transgène code la chaine légère d'un anticorps et le deuxième transgène code la chaine lourde d'un anticorps. Un tel exemple de configuration est présenté dans l'exemple 6. La protéine comprenant deux sous unités distinctes peut aussi être un anticorps monospécifique ou une hormone peptidique comprenant deux sous unités distinctes ; ou
- les deux transgènes codent chacun un polypeptide d'intérêt distinct. Par exemple, les deux polypeptides d'intérêt distincts constituent un ensemble de deux protéines virales, un complexe multienzymatique, un complexe protéique, par exemple une VLP composée de deux protéines distinctes.

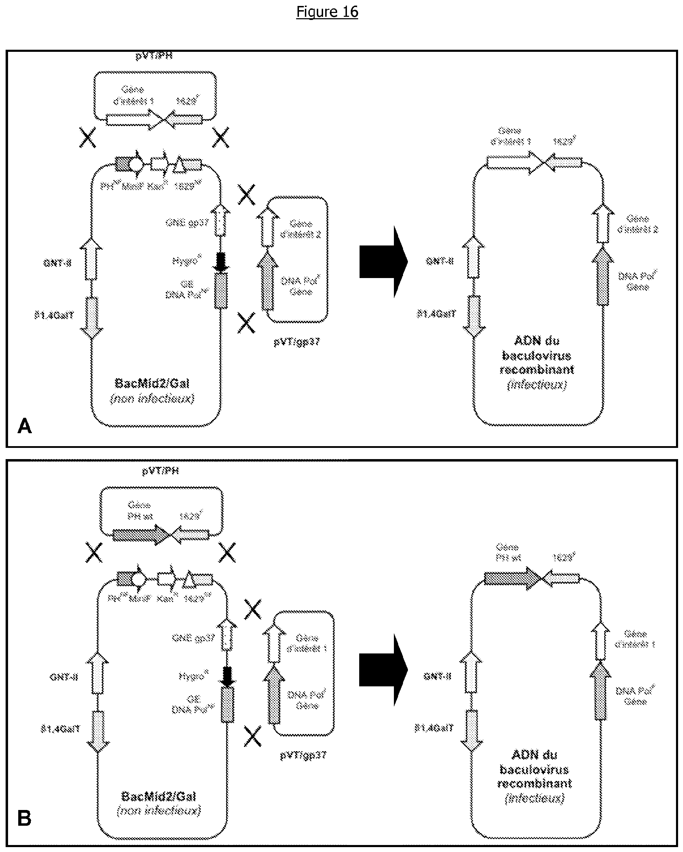

Les figures 16, 17 et 19 présentent des représentations schématiques de génomes de baculovirus comprenant deux transgènes d'intérêt.

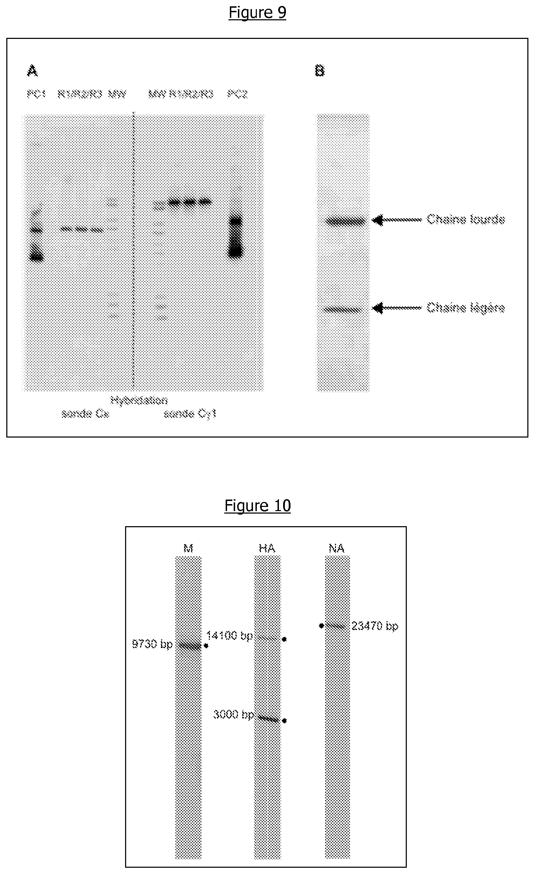

Dans un mode de réalisation particulier, n=3. Dans ce cas, on peut distinguer les mises en œuvre suivantes :
- les trois transgènes codent chacun une sous-unité d'une protéine comprenant trois sous-unités, avantageusement les trois transgènes codent chacun une sous-unité distincte d'une protéine comprenant trois sous-unités distinctes. La protéine comprenant trois sous unités distinctes peut être un anticorps bispécifique ou une hormone peptidique comprenant trois sous unités distinctes. Par exemple, le premier transgène code une première chaine légère d'un anticorps bispécifique, le deuxième transgène code une deuxième chaine légère d'un anticorps bispécifique et le troisième transgène une chaine lourde d'un anticorps bispécifique ; ou
- les trois transgènes codent chacun un polypeptide d'intérêt distinct. Par exemple, les trois polypeptides d'intérêt constituent un ensemble de trois protéines virales, un complexe multienzymatique, un complexe protéique, par exemple une VLP composée de trois protéines distinctes. Un tel exemple de configuration est présenté dans l'exemple 7, où le « BacMid3 » est utilisé pour la production de 3 protéines du virus de la grippe (voir aussi figure 10).

Pour ce mode de réalisation particulier, le baculovirus BacMid3 présenté dans l'exemple 3, dont les gènes essentiels à la réplication 1629, DNApol et gp64 sont non-fonctionnels, pourra être utilisé pour l'intégration simultanée de ces trois transgènes. Dans un mode de réalisation particulier, n=4. Dans ce cas, on peut distinguer les mises en œuvre suivantes :
- les quatre transgènes codent chacun une sous-unité d'une protéine comprenant quatre sous-unités, avantageusement les quatre transgènes codent chacun une sous-unité distincte d'une protéine comprenant quatre sous-unités distinctes. La protéine comprenant quatre sous unités distinctes peut être une hormone peptidique comprenant quatre sous unités distinctes ; ou
- les quatre transgènes codent chacun un polypeptide d'intérêt distinct. Par exemple, les quatre polypeptides d'intérêt constituent un ensemble de quatre protéines virales, un complexe multienzymatique, un complexe protéique, par exemple une VLP composée de quatre protéines distinctes.

Avantageusement, le baculovirus recombinant produit par la mise en œuvre du procédé de l'invention ne comprend pas de séquence d'acides nucléiques qui lui permet de se répliquer au sein d'une cellule bactérienne. Eventuellement, la séquence d'acides nucléiques qui permet de répliquer le génome de baculovirus déficient pour la réplication au sein d'une cellule bactérienne peut être éliminée lors de l'étape de recombinaison homologue dans la cellule d'insecte.

L'étape a) est réalisée après introduction dans la cellule d'insecte des vecteurs de transfert et du génome de baculovirus déficient pour la réplication. Cette introduction peut être réalisée avec les techniques largement décrites dans l'art antérieur. On peut citer notamment la technique au phosphate de calcium, la technique au DEAE dextran, l'électroporation, les méthodes basées sur les chocs osmotiques, la microinjection ou les méthodes basées sur l'emploi de liposomes, de préférence la lipofection. Le procédé selon l'invention est particulièrement avantageux car il permet d'introduire les n vecteurs de transfert et le génome de baculovirus déficient pour la réplication en une seule étape dans la cellule d'insecte. Les quantités de génome de baculovirus déficient pour la réplication et de vecteurs de transfert introduits dans la cellule d'insecte peuvent varier. On préfère employer une quantité 5 fois plus importante de chacun des n vecteurs de transfert par rapport à la quantité de génome de baculovirus déficient pour la réplication. Le génome de baculovirus déficient pour la réplication est avantageusement introduit dans la cellule d'insecte sous forme circulaire, c'est-à-dire sans avoir été linéarisé au préalable. La linéarisation est inutile puisque le génome de baculovirus est déficient pour la réplication, même sous forme circulaire puisqu'il comprend des gènes essentiels à la réplication virale non-fonctionnels. L'absence d'étape de linéarisation est un des avantages majeurs du procédé de l'invention.

Comme détaillé plus haut, l'enzyme de maturation des protéines peut être choisie parmi une peptidase signal, une furine, une proprotéine convertase, une glycosyltransférase, une glycosidase, une protéine chaperone, une disulfide isomérase, une acyltransférase, une méthyltransférase, une hydroxylase, une transglutaminase, une farnésyltransférase, une géranylgéranyl-transférase, une N-myristoyltransférase, une palmityltransférase, une protéine kinase, une phosphatase, une transpeptidase, une carboxylase ou une ubiquitine ligase.

Le choix de la ou des enzyme(s) de maturation des protéines dépendra des polypeptides d'intérêt, notamment du type de maturation que devront subir les polypeptides d'intérêt. Par exemple, lorsque les polypeptides d'intérêt forment les sous-unités d'une protéine d'intérêt glycosylée, par exemple un anticorps, la ou les enzyme(s) de maturation peuvent être une ou plusieurs glycosyltransférase(s) permettant d'obtenir la glycosylation souhaitée. L'homme du métier pourra aisément sélectionner la ou les glycosyltransférase(s) appropriée(s) en fonction de la glycosylation souhaitée. Par exemple, la ou les glycosyltransférase(s) peut/peuvent être choisie(s) parmi les N-acétylglucosaminyltransférase I, II, II, IV, V; VB, VI, IX, une galactosyltransférase (e.g. une béta-1,4-galactosyltransférase, par exemple choisie parmi la béta-1,4-galactosyltransférase 1, 2, 3, 4, 5, 6 et 7), la CMP-NeuAc synthase, la NeuAc synthase, une sialyltransférase (e.g. l'α2,3 sialyltransférase ou l'α2,6 sialyltransférase), les protéine-O-mannosyltransférases 1 et 2, les protéine-O-fucosyltransférases 1 et 2, la protéine-O-glucosyltransférase 1, la protéine-O-GlcNAc transférase, la GalNAc transférase, les fucosyltransférases 1, 2, 3, 4, 5, 6, 7, 8, 9, 10 et 11 (FUT1 à FUT11).
Dans un mode de réalisation particulier, la glycosyltransférase est une ou plusieurs glycosyltransférase(s) choisie(s) parmi la N-acétylglucosaminyltransférase II, une béta-1,4-galactosyltransférase et une sialyltransférase.

### Etape a1

Dans un mode de réalisation particulier, le génome de baculovirus déficient pour la réplication de l'étape a1) est préparé, dans une cellule bactérienne, par recombinaison homologue entre :
i) un génome de baculovirus déficient pour la réplication dans lequel n gènes essentiels à la réplication virale sont non-fonctionnels, et
ii) une ou plusieurs séquence(s) nucléotidique(s) comprenant chacune un ou plusieurs transgène(s) codant chacun une enzyme de maturation des protéines.

Ainsi, dans ce mode de réalisation particulier, le procédé de l'invention comprend les étapes de :
a') Préparer, dans une cellule bactérienne, un premier génome de baculovirus déficient pour la réplication dans lequel n gènes essentiels à la réplication virale sont non-fonctionnels et qui comprend un ou plusieurs transgène(s) codant chacun une enzyme de maturation des protéines, par recombinaison homologue entre :
a'1) un génome de baculovirus déficient pour la réplication dans lequel n gènes essentiels à la réplication virale sont non-fonctionnels, et
a'2) une ou plusieurs séquence(s) nucléotidique(s) comprenant chacune un ou plusieurs transgène(s) codant chacun une enzyme de maturation des protéines ;

a) Préparer, dans une cellule d'insecte, un deuxième génome de baculovirus capable de se répliquer qui comprend un ou plusieurs transgène(s) codant chacun une enzyme de maturation des protéines et n transgènes codant chacun un polypeptide d'intérêt (génome de baculovirus recombinant), par recombinaison homologue entre :
   a1) le premier génome de baculovirus déficient pour la réplication obtenu à l'étape a'), et
   a2) n vecteurs de transfert comprenant chacun:
      i) une séquence nucléotidique permettant de restaurer la fonction d'un des n gènes essentiels à la réplication virale non-fonctionnel,
      ii) un des n transgènes codant un polypeptide d'intérêt,
   l'ensemble des séquences nucléotidiques i) des n vecteurs de transfert étant capables de restaurer la réplication du premier génome de baculovirus déficient pour la réplication,
   n étant un nombre entier au moins égal à 2 ; et
b) Générer un baculovirus recombinant dans une cellule d'insecte qui comprend le génome de baculovirus recombinant obtenu à l'étape b),
ledit procédé étant caractérisé en ce que la recombinaison homologue permettant d'obtenir le deuxième génome de baculovirus capable de se répliquer se fait en une seule étape dans la cellule d'insecte.

Dans ce mode de réalisation, une première recombinaison se fait dans une cellule bactérienne entre (a'1) un génome de baculovirus déficient pour la réplication dans lequel n gènes essentiels à la réplication virale sont non-fonctionnels et (a'2) une ou plusieurs séquence(s) nucléotidique(s) comprenant chacune un ou plusieurs transgène(s) codant chacun une enzyme de maturation des protéines.

De préférence, le ou les transgène(s) codant une enzyme de maturation des protéines recombinent chacun au niveau d'un gène non-essentiel à la réplication virale, de préférence au niveau d'un gène non-essentiel à la réplication virale non-adjacent à un gène essentiel à la réplication virale non fonctionnel. Le gène non-essentiel à la réplication virale est avantageusement sélectionné parmi ptp (ORF1), ctx (ORF3), ORF4, ORF7, odv-e26 (ORF16), ORF17, ORF18, ORF19, ARIF-1 ORF20-21, pif2 (ORF22), protéine F (ORF23), iap1 (ORF27), lef6 (ORF28), ORF29, ORF30, sod (ORF31), fgf (ORF32), gta (ORF42), ORF43, ORF44, ORF45, odv-e66 (ORF46), ORF47, ORF56, ORF57, chaB-like (ORF58/59), chaB-like (ORF60), mtase (ORF69), hcf-1 (ORF70), iap2 (ORF71), ORF86, ORF87, ORF111, ORF114, pif3 (ORF115), ORF116, ORF117, pif1 (ORF119), ORF120, ORF121, ORF122, pk2 (ORF123), ORF124, lef7 (ORF125), chitinase (ORF126), gp16 (ORF130), p35 (ORF135), p26 (ORF136), p10 (ORF137), p74 (ORF138), ORF149, ORF150, ie2 (ORF151), pe38 (ORF153) et ORF154.

Dans ce mode de réalisation particulier, la cellule bactérienne est une bactérie qui supporte la réplication des génomes de baculovirus contenant une origine de réplication mini-F. La cellule bactérienne est de préférence *E. coli,* notamment DH10B ou EL350.

### Etape b)

L'étape b) consiste à générer un baculovirus recombinant dans une cellule d'insecte qui comprend le génome de baculovirus recombinant obtenu à l'étape a). Par exemple, il peut s'agir de la cellule d'insecte de l'étape a).

Avantageusement, la cellule d'insecte est cultivée dans des conditions adaptées pour qu'elle exprime le baculovirus recombinant, notamment dans un milieu de culture adapté à la croissance des cellules. Le milieu de culture peut contenir un sérum d'origine animale ou peut être un milieu de culture sans sérum.

Avantageusement, la cellule d'insecte est sélectionnée parmi Sf9, Sf21, Tn5-b14, les lignées cellulaires de lépidoptères sensibles au baculovirus AcMNPV, les lignées Sf21, la lignée « High Five », de préférence il s'agit de Sf9.

Le baculovirus recombinant ainsi généré peut être utilisé pour infecter d'autres cellules d'insectes. Ces cellules d'insecte infectées par le baculovirus recombinant peuvent alors produire chacun des transgènes. Cette production de chacun des transgènes permet donc d'obtenir les n polypeptides d'intérêt ayant été maturés par la ou les enzyme(s) de maturation des protéines.

Dans un mode de réalisation particulier, le baculovirus recombinant peut être utilisé pour infecter des cellules eucaryotes. Il a en effet été montré que les baculovirus pouvaient infecter des cellules eucaryotes.

Le procédé selon l'invention permet donc de produire facilement et rapidement un baculovirus recombinant dont le génome comprend un ou plusieurs transgène(s) codant chacun une enzyme de maturation des protéines et n transgènes distincts codant chacun un polypeptide d'intérêt distinct.

### Baculovirus recombinant

Est également décrit ici un baculovirus recombinant ou génome de baculovirus recombinant comprenant :
a) un ou plusieurs transgène(s) codant chacun une enzyme de maturation des protéines, et
b) n séquences nucléotidiques de formule (I) :
   [transgène codant un polypeptide d'intérêt]-[séquence nucléotidique intercalaire]-[gène essentiel à la réplication virale fonctionnel] (I),
   ladite séquence d'acides nucléiques intercalaire est constituée de 0 à 600 pb, de préférence de 1 à 600 paires de bases,
   ledit gène essentiel à la réplication virale fonctionnel est sélectionné parmi 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), helicase (ORF95), Vp80 (ORF104), ORF106-107, odv-ec43 (ORF109), gp64/67 (ORF128), ORF132, ORF133, odv-ec27 (ORF144), ORF146, ie1 (ORF147), lef-2 (ORF6);
   n étant un nombre entier au moins égal à 2.

Avantageusement, ledit baculovirus recombinant ou le génome de baculovirus recombinant ne comprend pas de séquence d'acides nucléiques qui lui permet de se répliquer au sein d'une cellule bactérienne. Il a en effet été démontré que l'absence d'une telle séquence permettait d'augmenter la stabilité du baculovirus recombinant, comparé à un baculovirus recombinant comportant une telle séquence (Pijlman et al. (2003) Journal of General Virology).

Avantageusement, ledit baculovirus recombinant ou génome de baculovirus recombinant ne comprend pas n gènes non-essentiels à la réplication virale choisis parmi Ph (ORF 8), ORF11, ORF13, egt (ORF15), v-ubiquitin (ORF35), 39K (ORF36), ORF38, p43 (ORF39), lef-12 (ORF41), pcna (ORF49), ORF52, ORF55, Fp (ORF61), ORF63, gp37(ORF64), ORF68, ORF72, ORF74, ORF82, cg30 (ORF88), ORF91, pif-4 (ORF96), he65 (ORF105), ORF108, ORF110, Cathepsine (ORF127), p24 (ORF129), pp34 (ORF131), ORF134, ORF145, odv-e56 (ORF148), ORF5. Avantageusement, un des n gènes non-essentiels à la réplication virale non compris dans le baculovirus recombinant ou génome de baculovirus recombinant est le gène codant la cathepsine car il a été montré que la cathepsine peut avoir un effet délétère sur les polypeptides d'intérêt produits.

n est un nombre entier au moins égal à 2, par exemple un nombre entier allant de 2 à 30, par exemple allant de 2 à 10, et plus spécifiquement étant égal à 2, 3 ou 4, comme détaillé dans la partie « Procédé de préparation du baculovirus recombinant » ci-dessus.

Selon une mise en œuvre particulière, n est supérieur ou égal à 3.

Est également décrit ici un baculovirus recombinant ou un génome de baculovirus recombinant, susceptible d'être obtenu par le procédé de production selon l'invention, comprenant :
a) un ou plusieurs transgène(s) codant chacun une enzyme de maturation des protéines, et
b) n séquences nucléotidiques de formule (I) :
   [transgène codant un polypeptide d'intérêt]-[séquence nucléotidique intercalaire]-[gène essentiel à la réplication virale fonctionnel] (I),
   ladite séquence d'acides nucléiques intercalaire est constituée de 0 à 600 pb, de préférence de 1 à 600 paires de bases,
   ledit gène essentiel à la réplication virale fonctionnel est sélectionné parmi 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), helicase (ORF95), Vp80 (ORF104), ORF106-107, odv-ec43 (ORF109), gp64/67 (ORF128), ORF132, ORF133, odv-ec27 (ORF144), ORF146, ie1 (ORF147), lef-2 (ORF6);
   n étant un nombre entier au moins égal à 2.

Avantageusement, les n séquences nucléotidiques de formule (I) sont réparties sur tout le génome du baculovirus recombinant, ce qui permet d'en améliorer la stabilité. Les n séquences nucléotidiques de formule (I) sont donc suffisamment espacées sur le génome du baculovirus. Avantageusement, chacune des n séquences nucléotidiques de formule (I) est espacée d'au moins 500 nucléotides par rapport à une autre des n séquences nucléotidiques de formule (I). L'obtention d'un baculovirus recombinant ou d'un génome de baculovirus recombinant avec n séquences nucléotidiques de formule (I) réparties sur tout le génome ne présente aucune difficulté particulière pour l'homme du métier puisque la répartition sur le génome sera liée aux couples « gène essentiel/gène non essentiel » qui seront choisis.

Avantageusement, et cela est inhérent à la mise en œuvre du procédé de préparation selon l'invention, les n séquences nucléotidiques de formule (I) ne sont pas dupliquées sur le génome du baculovirus recombinant. Il a en effet été démontré une diminution de la stabilité du baculovirus recombinant lorsque les séquences sont dupliquées sur le génome (données non-présentées).

### Jeu d'éléments de recombinaison homologue

Est également décrit ici un jeu d'éléments de recombinaison homologue comprenant :
a) un génome de baculovirus déficient pour la réplication dans lequel n gènes essentiels à la réplication virale sont non-fonctionnels et qui comprend un ou plusieurs transgène(s) codant chacun une enzyme de maturation des protéines ;
b) n vecteurs de transfert comprenant chacun:
   i) une séquence d'acides nucléiques permettant de restaurer la fonction d'un des n gènes essentiels à la réplication virale non-fonctionnel,
   ii) un transgène codant un polypeptide d'intérêt,
   n étant un nombre entier au moins égal à 2.

Avantageusement, les gènes essentiels à la réplication virale non-fonctionnels sont chacun adjacents à un gène non-essentiel à la réplication virale, comme décrit dans la partie « Procédé de préparation du baculovirus recombinant » ci-dessus.

Dans un mode de réalisation particulier, les vecteurs de transfert comprennent, de part et d'autre de la cassette d'expression du transgène, des séquences flanquantes homologues au génome de baculovirus déficient pour la réplication. Avantageusement, les séquences **flanquantes** de chacun des vecteurs de transfert sont homologues à tout ou partie dudit gène essentiel à la réplication virale non-fonctionnel et à tout ou partie dudit gène non-essentiel à la réplication virale. Avantageusement, les séquences flanquantes ont une longueur qui peut aller de 10 pb (i.e. 10 paires de bases) à 10 kb (i.e. 10 000 paires de bases), avantageusement allant de 100 pb à 6 kb, de préférence allant de 200 pb à 6 kb et, de manière tout à fait préférée allant de 400 pb à 6 kb. Les séquences flanquantes sont décrites en détail dans la partie « Procédé de préparation du baculovirus recombinant » ci-dessus.

Avantageusement, n est un nombre entier allant de 2 à 31, par exemple allant de 2 à 10, comme détaillé dans la partie « Procédé de préparation du baculovirus recombinant » ci-dessus.

### Cellule

La présente description concerne également une cellule comprenant un baculovirus recombinant ou un génome de baculovirus recombinanttel que décrit ci-dessus, ou une cellule comprenant un jeu d'éléments de recombinaison homologue tel que décrit ci-dessus.

Dans un mode de réalisation particulier, la cellule est une cellule d'insecte, de préférence sélectionnée parmi Sf9, Sf21, Tn5-b14, les lignées cellulaires de lépidoptères sensibles au baculovirus AcMNPV, les lignées Sf21, de préférence Sf9, comme décrit plus en détail dans la partie « Procédé de préparation du baculovirus recombinant » ci-dessus.

### Utilisation

La présente description concerne également l'utilisation d'un baculovirus recombinant ou d'un génome de baculovirus recombinant tel que décrit ci-dessus ou d'une cellule telle que décrite ci-dessus pour la production de n transgènes codant chacun un polypeptide d'intérêt.

La production de polypeptide recombinant d'intérêt à partir d'un baculovirus est bien décrite dans l'art antérieur et peut facilement être mise en œuvre par des techniques bien connues de l'homme de l'art.

### Baculovirus « mono-recombinant »

Dans un mode de réalisation particulier, le procédé de l'invention peut être utilisé pour produire un baculovirus « mono-recombinant », c'est-à-dire un baculovirus comprenant un seul transgène codant un polypeptide d'intérêt.
Dans ce mode de réalisation particulier, le procédé pour produire un baculovirus recombinant dont le génome comprend un ou plusieurs transgène(s) codant chacun une enzyme de maturation des protéines et un transgène codant un polypeptide d'intérêt, comprend les étapes de :
a) Préparer, dans une cellule d'insecte, un génome de baculovirus recombinant capable de se répliquer qui comprend un ou plusieurs transgène(s) codant chacun une enzyme de maturation des protéines et un transgène codant un polypeptide d'intérêt, par recombinaison homologue entre :
   a1) un génome de baculovirus déficient pour la réplication dans lequel n gènes essentiels à la réplication virale sont non-fonctionnels et qui comprend un ou plusieurs transgène(s) codant chacun une enzyme de maturation des protéines, et
   a2) n vecteurs de transfert comprenant chacun une séquence nucléotidique (i) permettant de restaurer la fonction d'un des n gènes essentiels à la réplication virale non-fonctionnel,
      un des n vecteurs comprenant le transgène codant le polypeptide d'intérêt,
         l'ensemble des séquences nucléotidiques (i) des n vecteurs de transfert étant capables de restaurer la réplication du génome de baculovirus déficient pour la réplication,
      n étant un nombre entier au moins égal à 2 ; et
b) Générer un baculovirus recombinant dans une cellule d'insecte qui comprend le génome de baculovirus recombinant obtenu à l'étape a),
   ledit procédé étant caractérisé en ce que la recombinaison se fait en une seule étape dans la cellule d'insecte.

### FIGURES

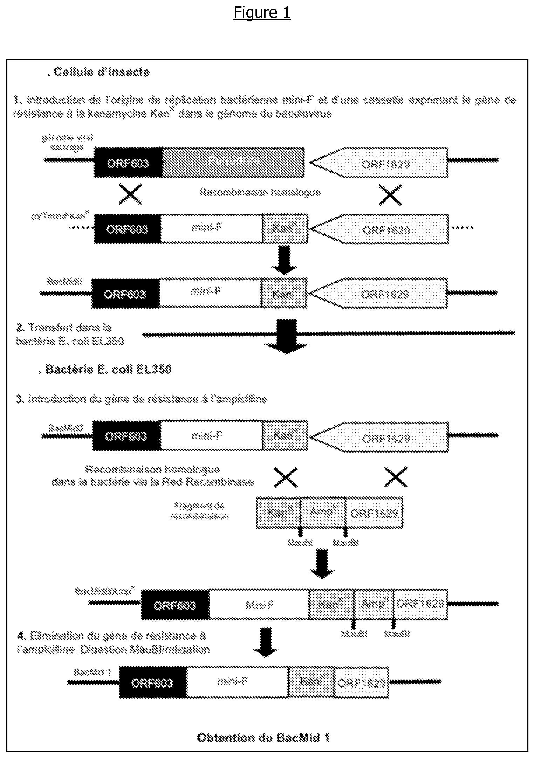

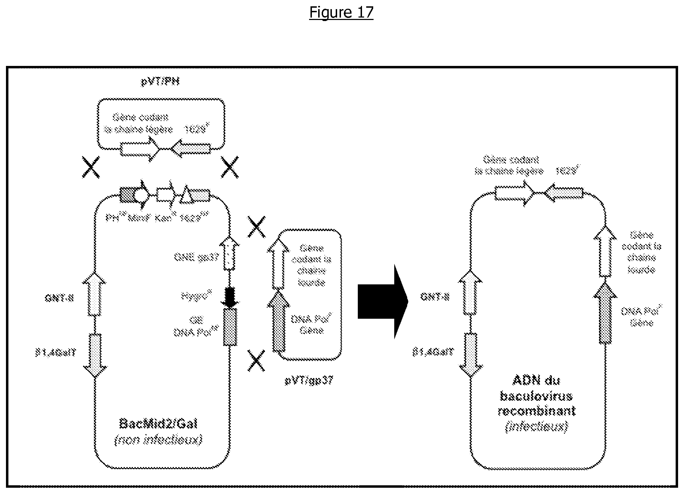

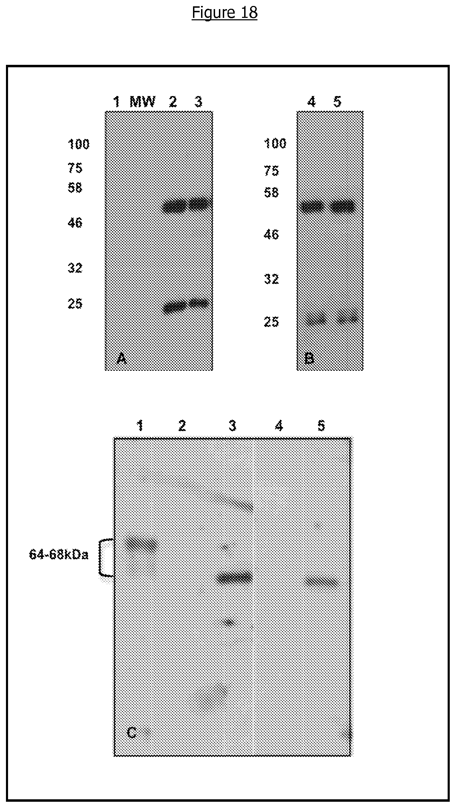

**La** **Figure 1** est un schéma qui illustre les étapes de préparation du BacMid1.
   *Légende:*
   ORF : open reading frame
   Polyédrine ou PH: gène baculovirus codant la polyédrine : gène non essentiel à la réplication virale.
   ORF603 : gène baculovirus codant la protéine 603, gène non essentiel.
   ORF1629 : gène baculovirus codant la protéine 1629: gène essentiel à la réplication virale
   pVT : plasmide vecteur de transfert.
   Mini-F : origine de réplication bactérienne.
   Kan^{R} : cassette d'expression bactérienne exprimant le gène de résistance à la kanamycine.
   Amp^{R} : cassette d'expression bactérienne exprimant le gène de résistance à l'ampicilline.
   Fragment de recombinaison : fragment d'ADN contenant la cassette d'expression à intégrer dans l'ADN cible. Ce fragment présente des régions flanquantes de part et d'autre de la cassette d'expression pour pouvoir cibler spécifiquement la région qui subira la recombinaison homologue via la Red recombinase.

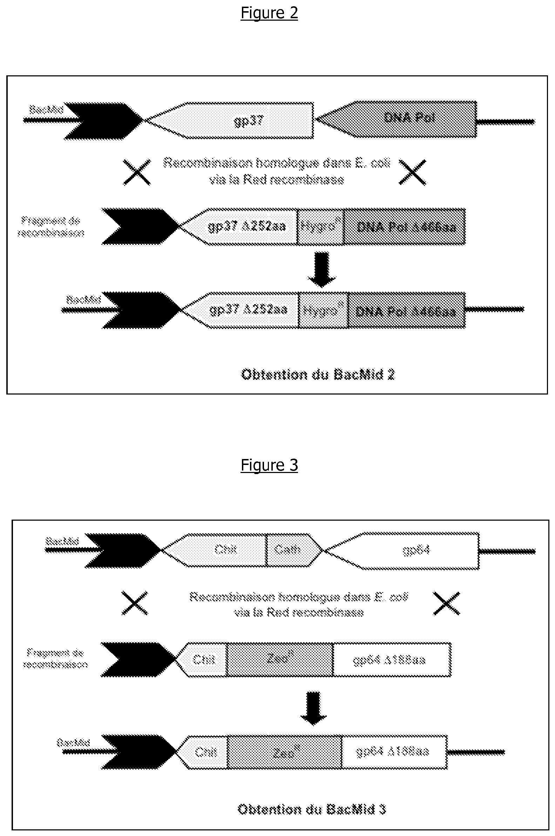

La **Figure 2** est un schéma qui illustre l'étape de délétion partielle du gène codant l'ADN polymérase virale, le gène dna pol pour la préparation du BacMid2.
   *Légende* :
   gp37 : gène baculovirus codant la glycoprotéine gp37, gène non essentiel à la réplication virale.
   gp37 Δ252 aa : gène gp37 délété de la région codant les 252 acides aminés N-terminaux.
   DNAPol : gène baculovirus codant l'ADN polymérase virale, gène essentiel à la réplication virale.
   DNAPol· Δ466 aa : gène dna pol délété de la région codant les 466 acides aminés C-terminaux.
   Hygro^{R} : cassette d'expression bactérienne exprimant le gène de résistance à l'hygromycine.
   Fragment de recombinaison : fragment d'ADN contenant la cassette d'expression à intégrer dans l'ADN cible. Ce fragment présente des régions flanquantes de part et d'autre de la cassette d'expression pour pouvoir cibler spécifiquement la région qui subira la recombinaison homologue via la Red recombinase.
La **Figure 3** est un schéma qui illustre l'étape de délétion partielle du gène codant la gp64 pour la préparation du BacMid3.
   *Légende* :
   Chit : gène baculovirus codant la chitinase, gène non essentiel à la réplication virale.
   Cath : gène baculovirus codant la cathepsine virale, gène non essentiel à la réplication virale.
   gp64 : gène baculovirus codant la glycoprotéine gp64 virale, gène essentiel à la réplication virale.
   gp64· Δ188 aa : gène gp64 délété de la région codant les 188 acides aminés C-terminaux.
   Zeo^{R} : cassette d'expression bactérienne exprimant le gène de résistance à la zéocine.
   Fragment de recombinaison : fragment d'ADN contenant la cassette d'expression à intégrer dans l'ADN cible. Ce fragment présente des régions flanquantes de part et d'autre de la cassette d'expression pour pouvoir cibler spécifiquement la région qui subira la recombinaison homologue via la Red recombinase.

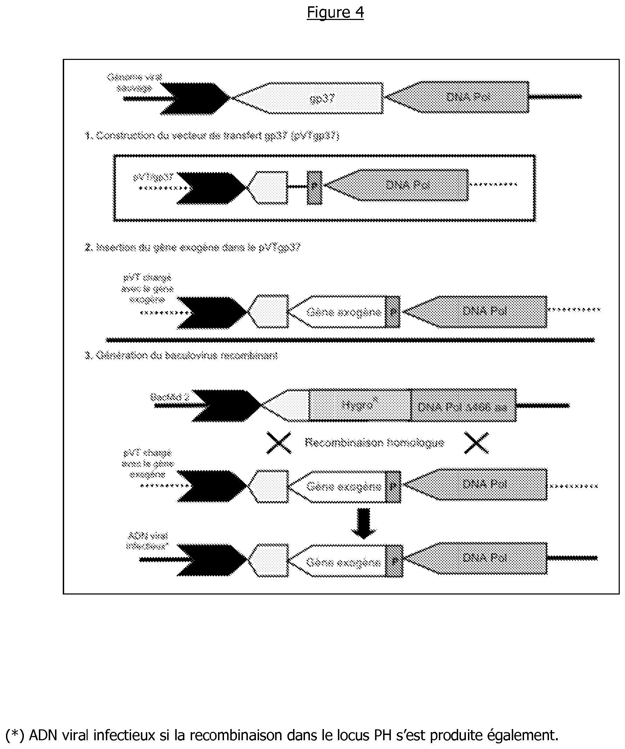

La **Figure 4** est un schéma qui illustre le vecteur de transfert pVT/gp37 et son utilisation avec le BacMid2 pour la génération d'un génome de baculovirus recombinant comprenant un transgène X.
   *Légende:*
   pVT : plasmide vecteur de transfert.
   gp37 : gène baculovirus codant la glycoprotéine gp37, gène non essentiel à la réplication virale.
   DNAPol : gène baculovirus codant l'ADN polymérase virale, gène essentiel à la réplication virale.
   DNAPol· Δ466 aa : gène dna pol délété de la région codant les 466 acides aminés C-terminaux.
   Gène exogène : transgène d'intérêt.
   P : promoteur viral ou cellulaire qui contrôle l'expression du transgène.
   Hygro^{R} : cassette d'expression bactérienne exprimant le gène de résistance à la hygromycine.

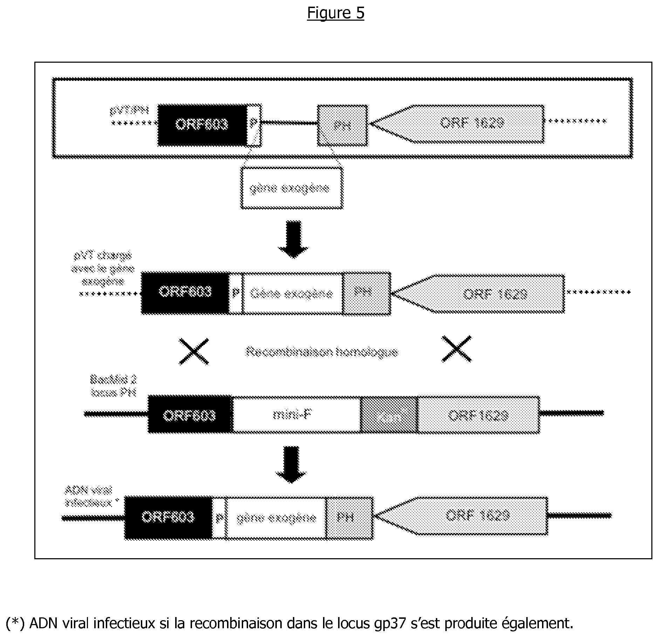

La **Figure 5** est un schéma qui illustre la construction et l'utilisation du vecteur de transfert PH pVT/PH avec le BacMid2 et pour la génération d'un génome de baculovirus recombinant comprenant un transgène X.
   *Légende:*
   pVT/PH : plasmide vecteur de transfert polyédrine.
   PH : tout ou partie du gène baculovirus codant la polyédrine, gène non essentiel à la réplication virale.
   ORF603: gène baculovirus codant la protéine 603, gène non essentiel.
   ORF1629 : gène baculovirus codant la protéine 1629, gène essentiel à la réplication virale.
   Gène exogène : transgène d'intérêt.
   P : promoteur viral ou cellulaire qui contrôle l'expression du transgène.
   Kan^{R} : cassette d'expression bactérienne exprimant le gène de résistance à la kanamycine.
   Mini-F : origine de réplication bactérienne.

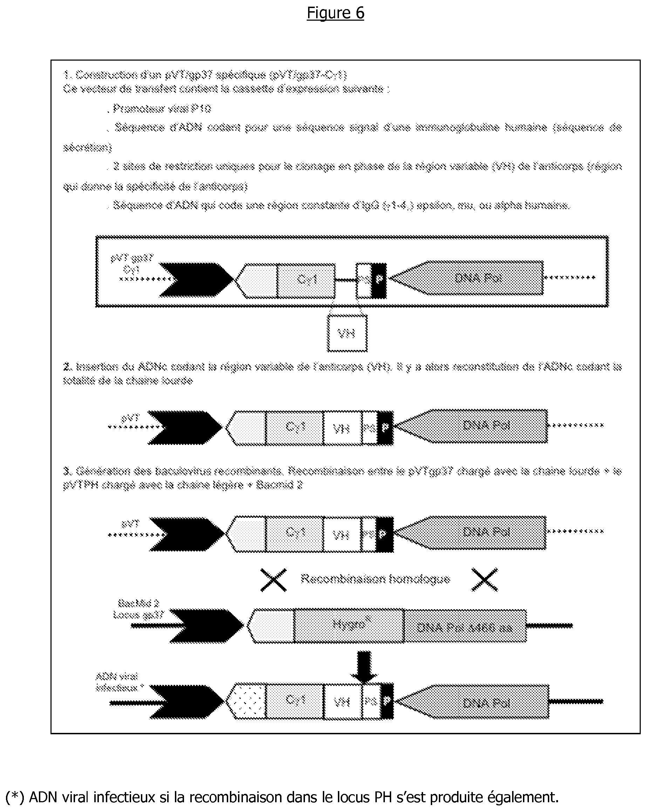

La **Figure 6** est un schéma qui illustre la construction et l'utilisation du vecteur de transfert pVT/gp37Cγ· avec le BacMid2 pour la génération d'un génome baculovirus recombinant comprenant la chaine lourde d'un anticorps.
   *Légende* :
   pVT : plasmide vecteur de transfert.
   pVT/gp37-Cγ1: plasmide vecteur de transfert spécifique de la chaine lourde d'une immunoglobuline.
   Cγ1 : ADNc codant le domaine constant y1 d'une immunoglobuline humaine.
   VH : ADNc codant le domaine variable de la chaine lourde d'une immunoglobuline.
   DNAPol : gène baculovirus codant l'ADN polymérase virale, gène essentiel à la réplication virale.
   DNAPolΔ466 aa : gène DNAPol délété de la région codant les 466 acides aminés C-terminaux.
   P : promoteur viral ou cellulaire qui contrôle l'expression du transgène.
   Hygro^{R} : cassette d'expression bactérienne exprimant le gène de résistance à la hygromycine.
   PS : ADNc codant une séquence signal (sécrétion de la chaîne lourde).

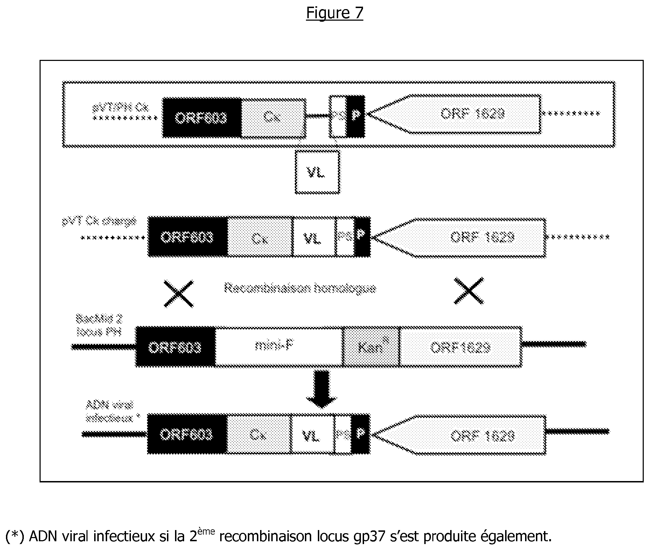

La **Figure 7** est un schéma qui illustre la construction et l'utilisation du vecteur de transfert pVT/PHCκ avec le BacMid2 pour la génération d'un génome de baculovirus recombinant comprenant la chaine légère d'un anticorps.
   *Légende* :
   ORF603 : gène baculovirus codant la protéine 603.
   ORF1629 : gène baculovirus codant la protéine 1629: gène essentiel à la réplication virale.
   Mini-F : origine de réplication bactérienne.
   pVT : plasmide vecteur de transfert.
   pVT/PH-Cκ : plasmide vecteur de transfert spécifique de la chaine légère d'une immunoglobuline.
   Cκ : ADNc codant le domaine constant kappa d'une immunoglobuline humaine.
   VL : ADNc codant le domaine variable de la chaine légère d'une immunoglobuline.
   P : promoteur viral ou cellulaire qui contrôle l'expression du transgène.
   Kan^{R} : cassette d'expression bactérienne exprimant le gène de résistance à la kanamycine.
   PS : ADNc codant une séquence signal (sécrétion de la chaîne légère).

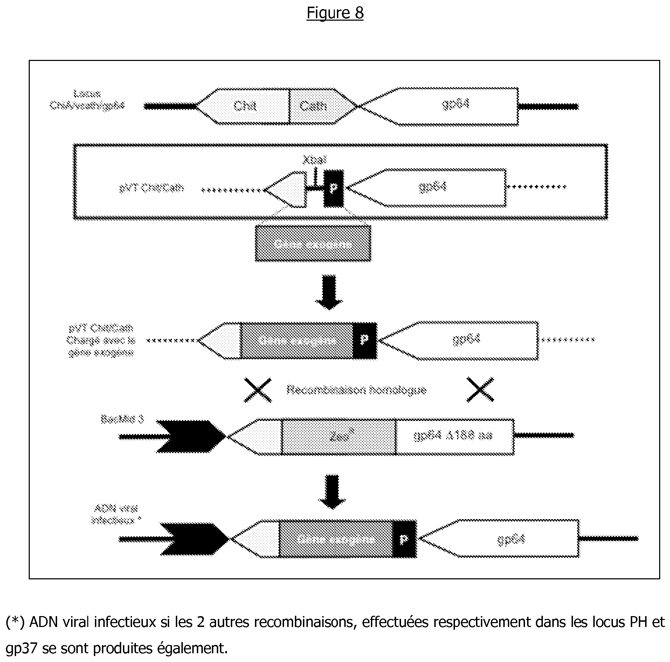

La **Figure 8** est un schéma qui illustre la construction du vecteur pVT/Chit-Cath et son utilisation avec le BacMid3 et la génération d'un génome de baculovirus recombinant comprenant un transgène.
   *Légende* :
   pVT Chit/Cath : plasmide vecteur de transfert capable de recombiner au niveau de la région comprenant les 2 gènes non essentiels, ChiA codant la chitinase et Cath codant la cathepsine.
   gp64Δ188 aa : gène gp64, gène essentiel, délété de la région codant les 188 acides aminés C-terminaux.
   Gène exogène : transgène d'intérêt.
   P : promoteur viral ou cellulaire qui contrôle l'expression du transgène.
   Zeo^{R} : cassette d'expression bactérienne exprimant le gène de résistance à la zéocine.
La **Figure 9** est A) l'analyse par Southern blot du génome de 3 baculovirus recombinants indépendants purifiés, générés au cours de la même transfection, et B) l'analyse de l'anticorps recombinant après purification sur Protéine A Sepharose (GE Healthcare).
   *Légende* :
   A : analyse de l'organisation des génomes de 3 baculovirus recombinants indépendants exprimant l'anticorps 13B8II. Ces baculovirus ont été isolés à partir d'une seule expérience de transfection. Les hybridations réalisées respectivement avec une sonde spécifique de la région constante de la chaîne légère kappa : sonde Cκ et une sonde spécifique de la région constante de la chaine lourde gamma 1 : sonde Cγ1 démontrent une organisation correcte et identique des 3 virus recombinants.
   B. Analyse par électrophorèse en gel de polyacrylamide (SDS, 2-mercaptoéthanol) et coloration argentique de l'anticorps recombinant purifié. L'anticorps sécrété dans le milieu de culture des cellules infectées par le baculovirus recombinant a été purifié sur colonne de Protéine A Sepharose.
   PC1 : Plasmide contrôle, plasmide contenant le gène de la chaine légère kappa, PC2 : Plasmide contrôle, plasmide contenant le gène de la chaine lourde γ1, R1-3, baculovirus recombinant 1, 2 et 3, MW : marqueur de taille.
La **Figure 10** est l'analyse en Southern blot du génome d'un virus triple recombinant exprimant les protéines M, HA et NA du virus de la grippe.
   *Légende* :
   M : gène du virus de la grippe codant la protéine de Matrice.
   HA : gène du virus de la grippe codant l'hémagglutinine.
   NA : gène du virus de la grippe codant la neuraminidase.
   bp : taille des fragments d'ADN exprimée en paire de base.
La **Figure 11** est un schéma qui illustre en A la structure de l'anticorps bispécifique en B l'analyse par électrophorèse en gel de polyacrylamide de l'anticorps bispécifique purifié sur colonne de protéine A Sepharose.
   Légende :
   A : Représentation schématique de la structure de l'anticorps bispécifique. L1 : chaine légère de l'anticorps 1 ; L2, chaine légère de l'anticorps 2.
   B : Anticorps bispécifique purifié, analysé par électrophorèse sur gel de polyacrylamide. Les protéines ont été révélées par coloration argentique. (1) électrophorèse en conditions réductrices (SDS, 2-mercaptoéthanol). (2) électrophorèse en conditions non réductrices. H : chaine lourde de l'anticorps, L : chaîne légère de l'anticorps, H2L4 : composition de l'anticorps bispécifique : 2 chaînes lourdes fusionnées reliées par 4 ponts disulfures au niveau de 2 régions charnières + 4 chaines légères (2 chaines L1 + 2 chaines L2) appariées de manière spécifique aux régions VH1-CH1 et VH2-CH1 correspondantes.
La **Figure 12** est une représentation des structures glycanniques liées aux glycoprotéines synthétisées par (A) des cellules humaines (B) des cellules de lépidoptères.
La **Figure 13** est représentation d'une immunoglobuline (IgG) humaine. Le résidu aspargine 297, (Asn297) lié à un *N*-glycanne est représenté par un losange. La nature de cette *N-*glycosylation, comme la présence de galactose et d'acide sialique, peut être un élément important de la structure de l'anticorps puisqu'elle permet une modulation de certaines activités effectrices, par exemple l'ADCC et la CDC.

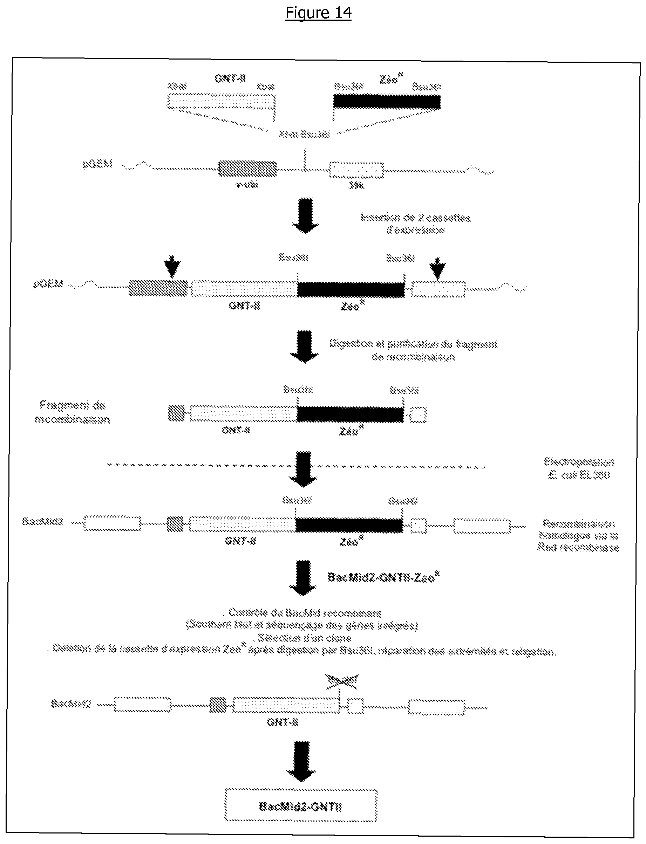

La **Figure 14** est une représentation des différentes étapes nécessaires à la construction d'un BacMid2/MPT (MPT : Modification Post Traductionnelle) c'est-à-dire un BacMid2 dont le génome comprend un transgène codant chacun une enzyme de biosynthèse des glycannes - ou d'une manière plus générale d'un BacMid2 dont le génome comprend un ou plusieurs transgène(s) codant chacun une enzyme de maturation des protéines.
   La figure 14 exemplifie spécifiquement l'intégration d'un transgène codant la GNT-II dans la région intergénique orf35(v-ubi)-orf36(39k) (IG35/36) du BacMid2.
   Deux cassettes d'expression ont été successivement insérées dans la région IG35/36 préalablement clonée dans un plasmide pUC (i) une cassette d'expression virale, composée d'un promoteur précoce viral (voir le **Tableau 2)** et du gène codant la GNT-II et (ii) une cassette d'expression bactérienne contrôlant le gène de la résistance à la zéocine (Zeo^{R}). Un « fragment de recombinaison » contenant les 2 cassettes a été généré par digestion du plasmide ci-dessus par 2 endonucléases de restriction. Ce dernier a été introduit dans la bactérie EL350/BacMid2 par électroporation. Une recombinaison homologue s'est produite via la Red Recombinase entre les régions flanquantes du fragment de recombinaison et l'ADN du BacMid2 permettant l'intégration des 2 cassettes d'expression. Les bactéries recombinantes ainsi obtenues ont été sélectionnées avec de la zéocine puis le gène codant la résistance à cet antibiotique a été éliminé de l'ADN du bacmid par simple digestion/réparation/religation. Le bacmid obtenu appelé BacMid2-GNTII, a été réintroduit par électroporation dans une bactérie EL350 (EL350/BacMid2-GNT-II).

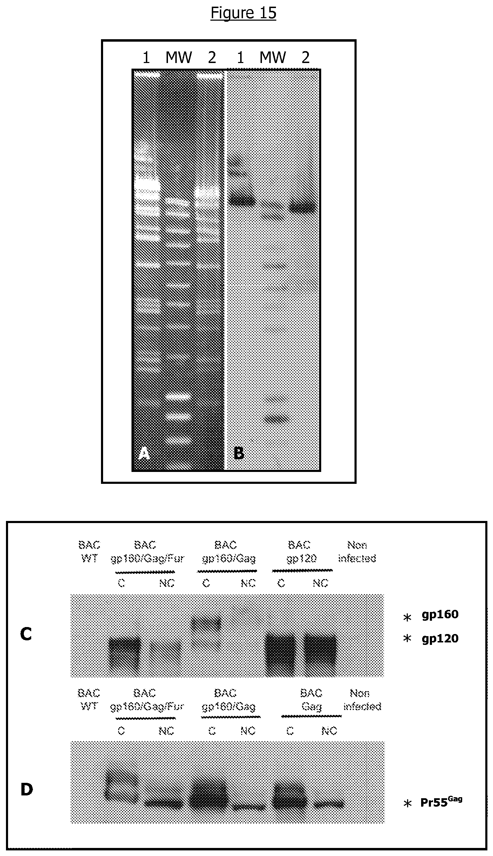

La **Figure 15** présente les BacMid2-fur et BacMid2Gal-Fur.
   A et B : Contrôle de l'organisation génomique des BacMids BacFur et BacGal-Fur. A. L'ADN des 2 bacmids a été digéré par EcoRI, les fragments générés ont été séparés en gel d'agarose à 1% puis colorés au bromure d'éthidium. B. L'ADN a été transféré sur une membrane de nylon selon la technique de Southern. Les membranes ont été incubées avec une sonde spécifique du gène *fur* exprimée par la cellule Sf9.
   Légende :
   A, Gel d'agarose coloré au bromure d'éthidium, *Puits 1:* Patron de restriction EcoRI du BacMid2Gal-Fur, *Puits* 2: Patron de restriction EcoRI du BacMid2-Fur. B. Southern blot, *Puits* 1: Patron de restriction EcoRI du BacMid2Gal-Fur, *Puits* 2: Patron de restriction EcoRI du BacMid2-Fur.
   C et D : Deux virus recombinants co-exprimant la polyprotéine Pr55Gag et gp160 du virus VIH-1 ont été construits l'un à partir du BacMid2 et l'autre à partir du BacMid2-Fur. Les cellules Sf9 ont été infectées pendant 48 heures avec les différents virus et les protéines sécrétées dans le surnageant de culture ont été concentrées ou non avec une solution de « Retro Concentin^{™} virus precipitation (SBI, référence RV100A-1)» puis déposées sur un gel de polyacrylamide à 10% en conditions dénaturantes et réductrices puis analysées en western blot. C. Les protéines ont été révélées avec un anticorps anti-gp120 (référence Ab21179, Abcam). D. Les protéines ont été révélées avec un anticorps anti-Pr55^{Gag} (référence. 63917, Abcam).
   Légende :
   BACWT : baculovirus sauvage, BACgp160/Gag/Fur : baculovirus triple-recombinant exprimant la gp160 et la polyprotéine Pr55^{Gag} du VIH-1 ainsi que la furine de la cellule Sf9 BACgp160/Gag: baculovirus double-recombinant exprimant la gp160 et la polyprotéine Pr55^{Gag} du VIH-1, BACgp120 : baculovirus mono-recombinant exprimant la gp120 du VIH-1, BACGag : baculovirus mono-recombinant exprimant la polyprotéine Pr55^{Gag} du VIH-1.
La **Figure 16** est une représentation du principe de l'utilisation du BacMid2-Gal (BacGal) pour la génération de génomes de baculovirus double- ou mono-recombinants.
   A. Génération d'un génome de baculovirus double-recombinant.
      Les 2 transgènes d'intérêt sont clonés dans leur vecteur de transfert respectif, le pVT/PH ciblant le couple GNE/GE PH/1629 et le pVT/gp37 ciblant le couple GNE/GE gp37/DNAPol. Les cellules Sf9 sont transfectées avec les 2 pVT et de l'ADN du BacMid2-Gal. Au cours de la recombinaison homologue, les 2 transgènes d'intérêt sont intégrés dans le génome du BacMid2-Gal tandis que simultanément, les gènes non fonctionnels *1629et dnapol* portés par le BacMid2-Gal sont remplacés par une copie fonctionnelle. Ces évènements auront pour conséquence l'élimination de l'origine de réplication bactérienne et la génération d'un génome de baculovirus recombinant infectieux. Des baculovirus recombinants sont alors produits et sécrétés dans le milieu de culture, puis clonés par la méthode des plages de lyse.
   B. Génération d'un génome de baculovirus mono-recombinant.
      Le gène d'intérêt est cloné dans le vecteur de transfert pVT/gp37. Les cellules Sf9 sont transfectées avec le pVT/gp37 comprenant un transgène, le pVT/PH ne contenant pas de transgène et de l'ADN du BacMid2-Gal. Au cours de la recombinaison homologue, il y a réparation du bacmid dans les 2 locus et donc génération de baculovirus infectieux.
   *Légende* :
   GE : Gène essentiel
   GNE : Gène non essentiel
   pVT/PH : Vecteur de transfert qui cible le couple GNE/GE PH/1629
   pVT/gp37 : vecteur de transfert qui cible le couple GNE/GE gp37/DNAPol
   DNA Pol^{NF} : Gène codant la DNA polymérase virale non fonctionnelle
   DNA Pol^{F} : Gène codant la DNA polymérase virale fonctionnelle
   1629^{NF} : Gène codant la protéine 1629 non fonctionnelle
   1629^{F} : Gène codant la protéine 1629 fonctionnelle
   β1,4 GalT : β1,4 galactosyltransférase
   GNT-II : Nacétylglucosaminyltransférase II
   Kan^{R} : gène de résistance à la kanamycine
   mini-F : Origine de réplication bactérienne
   PH : gène polyédrine
La **Figure 17** est une représentation du principe de la préparation d'un génome de baculovirus double-recombinant exprimant un anticorps galactosylé. Les cellules Sf9 sont transfectées avec les pVT/H (voir **Figure 6****),** pVT/L (voir **Figure 7****)** et de l'ADN du BacMid2-Gal. Au cours de la recombinaison homologue, les transgènes codant les chaînes lourdes et légères sont intégrés dans le génome du BacMid2-Gal tandis que simultanément, les gènes non fonctionnels *1629* et *dnapol* portés par le BacMid2-Gal sont remplacés par une copie fonctionnelle, rendant le génome de baculovirus infectieux. Des baculovirus recombinants sont alors produits, sécrétés dans le milieu de culture puis clonés par la méthode des plages de lyse.
   *Légende* :
   GE : Gène essentiel
   GNE : Gène non essentiel
   pVT/PH : Vecteur de transfert qui cible le couple GNE/GE PH/1629
   pVT/gp37 : vecteur de transfert qui cible le couple GNE/GE gp37/DNAPol
   DNA Pol^{NF} : Gène codant la DNA polymérase virale non fonctionnelle
   DNA Pol^{F} : Gène codant la DNA polymérase virale fonctionnelle
   1629^{NF} : Gène codant la protéine 1629 non fonctionnelle
   1629^{F} : Gène codant la protéine 1629 fonctionnelle
   β1,4 GalT : β1,4 galactosyltransférase
   GNT-II : Nacétylglucosaminyltransférase II
   Kan^{R} : gène de résistance à la kanamycine
   mini-F : Origine de réplication bactérienne
   PH : gène polyédrine
La **Figure 18** représente une analyse par western blot et lectine blot des anticorps produits par une cellule Sf9 infectée par un baculovirus recombinant généré à partir du BacMid2 ou du BacMid2-Gal. Ces anticorps humains (les anticorps recombinants 13B8II) présentent un domaine constant humain) ou murins ne présentant pas de glycosylation dans leur paratope, les analyses révèlent donc la nature de la *N*-glycosylation qui est portée par leur domaine constant.
   *Légende* :
   A et B. Western blot. A : La membrane a été incubée avec un anticorps de mouton anti-IgG humaine entière conjugué à la peroxydase (référence NA933V, GE Heathcare). B :La membrane a été incubée avec un anticorps de mouton anti-IgG murine entière conjugué à la peroxydase (référence NA931V, GE Heathcare).
   *A. Puits 1* : Fétuine (61-68 kDa), fournie dans le kit « Dig glycan differentiation » de chez Roche, cette protéine sialylée en α2,3 et α2,6 constitue le contrôle positif pour les analyses en lectine blot que ce soit pour la SNA, la MAA ou la diCBMA. Selon le fournisseur la masse molaire (*) de cette protéine varie entre 68 et 61 kDa, *Puits 2* : Anticorps recombinant 13B8II/BacGal, *Puits* 3 : Anticorps recombinant 13B8II/BacMan,
   *B. Puits* 1 : Anticorps recombinant murin/BacGal, *Puits* 2 : Anticorps recombinant murin/BacMan,
   C. Lectine blot. La membrane a été incubée en présence de RCA₁₂₀ conjuguée à la biotine. La présence de la lectine a été révélée comme décrit dans l'Exemple 17.
   *Puits* 1: Fétuine (61-68 kDa) *Puits2* : Anticorps recombinant murin/BacMan, *Puits3:* Anticorps recombinant murin/BacGal, *Puits4* : Anticorps recombinant 13B8II/BacMan, *Puits5* : Anticorps recombinant 13B8II/BacGal.

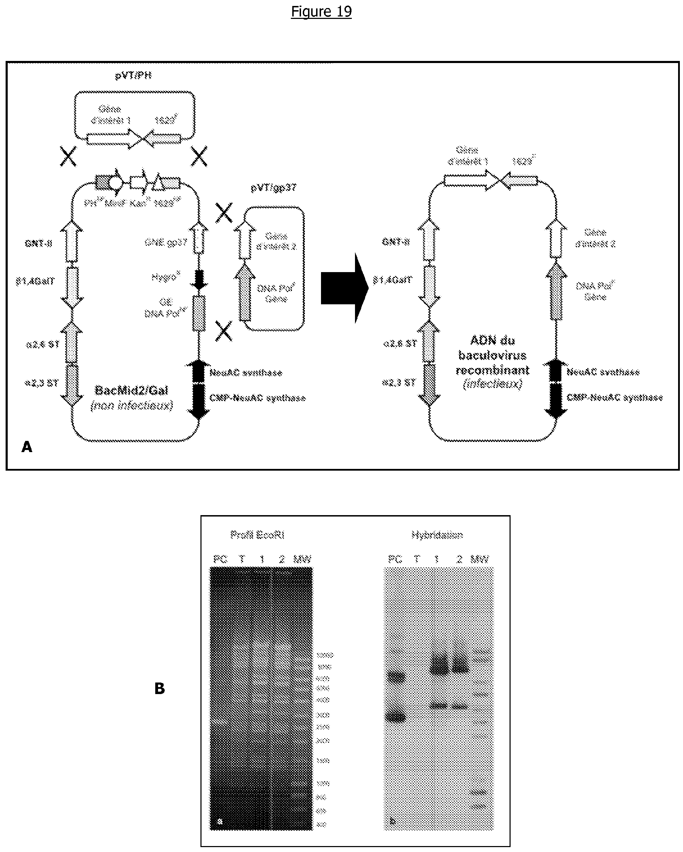

La **Figure 19** est un schéma qui décrit l'utilisation des BacMid2-Sia pour la préparation de génomes de baculovirus multi-recombinants et la construction d'une seconde génération de nouveau BacMidSia, le BacMidSia6-II. Dans la **Figure 19A****,** les transgènes 1 et 2 sont clonés respectivement dans le pVT/PH ciblant le couple GNE/GE PH/1629 et dans le pVT/gp37 ciblant le couple GNE/GE gp37/DNAPol. Le génome du BacMid-Sia est équipé de tous les gènes nécessaires à la sialylation en α2,3 [α2,3 sialyltransférase] (BacMid2-Sia3 ou BacSia3) ou en α2,6 [α2,6 sialyltransférase] (BacMid2-Sia6 ou BacSia6 et BacMid2Sia6-II ou BacSia6-II) ou en α2,3 + α2,6 [α2,3 sialyltransférase + α2,6 sialyltransférase] (BacMid2-Sia3/6 ou BacSia3/6) et des 2 gènes nécessaires à la biosynthèse du nucléotide sucre CMP-NeuAC. Les cellules Sf9 sont transfectées avec les 2 pVT et de l'ADN de l'un des BacMid-Sia. Au cours de la recombinaison homologue, les 2 transgènes sont intégrés dans le génome du BacMid2-Sia tandis que, simultanément, les gènes non fonctionnels *1629* et *dnapol* portés par le BacMid2-Sia sont remplacés par une copie fonctionnelle, rendant le génome de baculovirus infectieux. Des baculovirus recombinants sont alors produits et sécrétés dans le milieu de culture puis clonés par la méthode des plages de lyse.
   *Légende* :
   GE : Gène essentiel
   GNE : Gène non essentiel
   pVT/PH : Vecteur de transfert qui cible le couple GNE/GE PH/1629
   pVT/gp37 : vecteur de transfert qui cible le couple GNE/GE gp37/DNAPol
   DNA Pol^{NF} : Gène codant la DNA polymérase virale non fonctionnelle
   DNA Pol^{F} : Gène codant la DNA polymérase virale fonctionnelle
   1629^{NF} : Gène codant la protéine 1629 non fonctionnelle
   1629^{F} : Gène codant la protéine 1629 fonctionnelle
   β1,4 GalT : β1,4 galactosyltransférase
   GNT-II : Nacétylglucosaminyltransférase II
   α2,3 ST : α2,3 sialyltransférase
   α2,6 ST : α2,6 sialyltransférase
   Kan^{R} : gène de résistance à la kanamycine
   mini-F : Origine de réplication bactérienne
   PH : gène polyédrine
La **Figure 19B** est l'analyse par Southern blot du génome de 2 clones de BacMid2Sia6-II dont la construction est décrite dans l'exemple 14.
   *Légende* :
   a : L'analyse du profil électrophorétique de digestion par EcoRI de 2 clones de BacMid2Sia6-II (1 et 2) en comparaison avec le BacMid2-GNTII- ß1,4GT-CMPNeuAcS-NeuAcS (T) montre que l'intégration de la cassette ST6GalI dans la région Pif1 génère 2 fragments EcoRI de 3122pb et 6138pb.
   b : L'hybridation réalisée avec une sonde spécifique de la ST6GalI permet de vérifier le marquage des 2 fragments EcoRI de 3122pb et 6138pb et du plasmide de contrôle (PC), plasmide contenant le gène de la ST6GalI et démontre une organisation correcte et identique des 2 BacMids obtenus.
   MW : Smart Ladder (Eurogentec)

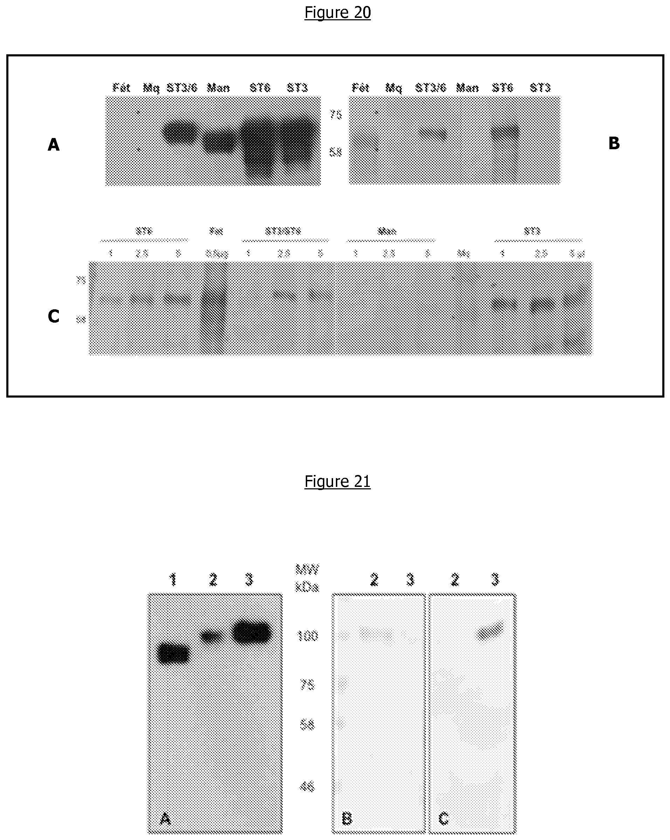

La **Figure 20** représente l'analyse par western blot et lectine blot de la glycosylation de la protéine virale gp64 exprimée par différents virus générés à partir de différents Bacmids. La glycoprotéine gp64 est la glycoprotéine majeure du baculovirus, elle est localisée à la surface du baculovirus. Il a été montré que cette glycoprotéine était susceptible d'être galactosylée et sialylée. Les cellules ont été infectées avec les différents virus recombinants. Les baculovirus sécrétés dans le surnageant de culture ont été sédimentés puis repris par un tampon de lyse pour être analysés en western blot puis en lectine blot.
   A. Western blot. La membrane a été incubée avec l'anticorps anti-gp64 AcV5 (référence SC65499, Santa Cruz Biotechnology)
   B et C : Lectine blot. B. La membrane a été incubée en présence de SNA, lectine qui reconnaît spécifiquement les acides sialiques liés en α2,6 (C) la lectine di-CBM40 qui reconnaît les acides sialiques liés en α2,3 et dans une moindre mesure les acides sialiques liés en α2,6.
   *Légende* :
   Fét : Fétuine
   Mq : Marqueurs de masse moléculaire
   ST3 : Virus généré à partir du bacmid BacSia3
   ST6 : Virus généré à partir du Bacmid BacSia6
   ST3/6 : Virus généré à partir du bacmid BacSia3/6
   Man : Virus généré à partir du BacMid2
La **Figure 21** représente l'analyse par Western blot et lectine blot de la protéine X recombinante produite grâce à un baculovirus recombinant généré à partir du BacSia6. Cette protéine étant soluble, les analyses ci-dessous ont été effectuées sur la protéine purifiée. Après électrophorèse en gel de polyacrylamide puis transfert sur membrane, la protéine a été incubée en présence soit d'un anticorps spécifique pour l'analyse en Western blot (A) soit d'une lectine spécifique pour le lectine blot, la SNA (B), ou la MAA (C).
   *Légende* :
   *Puits 1,* la protéine X a été produite après infection des cellules Sf9 avec un baculovirus recombinant généré à partir du BacMid2. *Puits 2,* la protéine X a été produite après infection des cellules Sf9 avec un baculovirus recombinant généré à partir du BacSia6. *Puits 3,* Protéine X du commerce produite en cellules CHO. La présence de la lectine a été révélée comme décrit dans l'Exemple 17. MW : Marqueur de masse moléculaire (Marqueur précoloré, Biolabs référence P7706).

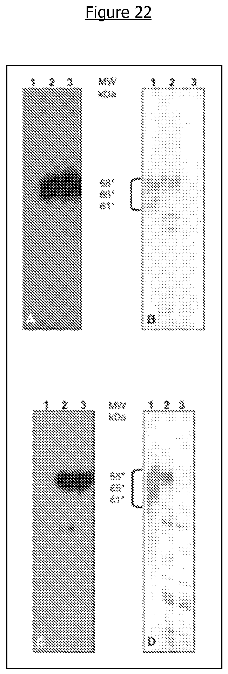

La **Figure 22** représente l'analyse par Western blot et lectine blot de la protéine VSVg produite grâce à un baculovirus recombinant généré à partir du BacSia6. La protéine VSVg étant membranaire, les analyses ont été effectuées sur des culots de cellules Sf9 infectées.
   A et B : Analyse de la protéine VSVa.
   A. Western blot, après électrophorèse en gel de polyacrylamide puis transfert sur membrane, les protéines ont été incubées en présence d'un anticorps spécifique dirigé contre la VSVg (Anticorps de souris conjugué à la peroxydase, référence A5977 Sigma).
   *Légende* :
   *Puits 1,* Fétuine, *Puits 2,* et 3, cellules Sf9 infectées par un baculovirus recombinant exprimant la protéine VSVg généré à partir du BacSia6 *(Puits 2)* ou un Bacmid2 *(Puits 3).*
   B. Lectine blot. Les protéines transférées sur une membrane de nitrocellulose ont été mises en présence de la lectine SNA *(Sambucus nigra* agglutinin) spécifique des résidus d'acides sialiques liés en α2,6. La présence de la lectine a été révélée comme décrit dans l'Exemple 17. C et D. Analyse de la protéine virale gp64.
   Nous avons aussi vérifié que le baculovirus recombinant exprimant la VSVg sialylée portait également une gp64 sialylée. Pour cela, les baculovirus sécrétés dans le surnageant de culture ont été sédimentés puis repris par un tampon de lyse pour être analysés en Western blot puis en lectine blot. C. Western blot, la gp64 a été révélée avec un anticorps spécifique (anticorps anti-gp64 AcV5, référence SC65499, Santa Cruz Biotechnology). D. Lectine blot. en présence de la lectine SNA comme décrit dans l'Exemple 17.
   *Légende* :
   *Puits 1,* Fétuine, *Puits 2,* et 3, particules de baculovirus préparés à partir des surnageants de culture de cellules infectées avec le baculovirus recombinant exprimant la protéine VSVg et généré à partir du BacMid2-Sia6 *(Puits 2)* ou un Bacmid2 *(Puits 3).*

### EXEMPLES

Les exemples 1 à 3 sont relatifs à la construction de baculovirus déficients pour la réplication, dans lesquels 1, 2 ou 3 gènes respectivement sont non-fonctionnels.

Les exemples 4 et 5 décrivent la génération de baculovirus recombinants ayant intégrés 2 ou 3 transgènes, respectivement.

Les exemples 6 à 8 sont relatifs à l'utilisation de ces baculovirus recombinants ayant intégrés 2 ou 3 transgènes pour la production de protéines d'intérêt.

Les exemples 9 à 15 décrivent la construction de baculovirus recombinants comprenant des transgènes codant pour des enzymes de maturation des protéines.

Les exemples 16 à 19 démontrent que des protéines d'intérêt produites grâce aux baculovirus des exemples 9 à 15 présentent une maturation et/ou une glycosylation satisfaisante.

### Exemple 1 : Construction d'un génome de baculovirus déficient pour la réplication dans lequel 1 gène essentiel à la réplication virale est non-fonctionnel (BacMid1)

Le BacMid1 présente la délétion d'un gène essentiel à la réplication virale, le gène 1629.

### 1. Intégration de l'origine de réplication bactérienne dans un génome du baculovirus

Cette opération est réalisée dans la cellule d'insecte.

L'origine de réplication bactérienne Mini-F a été introduite dans le locus polyédrine du génome de baculovirus AcMNPV par recombinaison homologue dans les cellules d'insectes Sf9 (*Spodoptera frugiperda*)*.* Pour cela, les cellules ont été transfectées avec (i) un vecteur de transfert PH (pVT/Mini-F-Kan^{R}) dans lequel la séquence du gène ph a été remplacée par un fragment d'ADN portant le Mini-F + une cassette d'expression bactérienne conférant la résistance à la kanamycine (Kan^{R}), et (ii) un génome de baculovirus AcMNPV (Baculovirus isolé à partir du lépidoptère *Autographa californica*)*.* Les baculovirus générés ont été purifiés par la technique des plages de lyse puis caractérisés afin de confirmer qu'ils avaient bien intégré le Mini-F et la cassette d'expression Kan^{R}. Un baculovirus a été sélectionné et a ensuite été transféré dans la bactérie *E coli* EL350, générant ainsi un premier BacMid (BacMid0, non déficient pour la réplication virale en cellules d'insecte).

### 1. Délétion du cène essentiel 1629

Une cassette d'expression bactérienne conférant la résistance à l'ampicilline (Amp^{R}) et présentant en 5' et 3' le site de restriction MauBI - site absent du génome de baculovirus *Ac*MNPV - a été intégré en aval de la cassette d'expression bactérienne Kan^{R} par recombinaison homologue dans la bactérie *E coliEL350.* Au cours de cette recombinaison, un fragment d'ADN codant les 27 acides aminés C-terminaux de la protéine 1629 a été délété, rendant la protéine 1629 non-fonctionnelle (BacMid0/amp^{R}). Le gène de résistance à l'ampicilline a ensuite été éliminé après digestion par MauBI puis religation, générant ainsi le BacMid1. Le génome du baculovirus (i.e. le BacMid1) est alors déficient pour la réplication en cellules d'insectes, car un gène essentiel à la réplication virale (i.e. le gène codant la protéine 1629) est non-fonctionnel. Les bactéries contenant le BacMid1 sont appelées ci-après « bactéries *E. coli* EL350/BacMid1 ».

La **Figure 1** illustre les étapes de préparation du BacMid1.

### Exemple 2 : Construction d'un génome de baculovirus déficient pour la réplication dans lequel 2 gènes essentiels à la réplication virale sont non-fonctionnels (BacMid2)

Le BacMid2 présente la délétion de 2 gènes essentiels à la réplication virale, le gène 1629 et le gène codant l'ADN polymérase virale (DNAPol). A partir du BacMid1, la délétion du gène DNAPol a été réalisée dans les bactéries *E coli* EL350/BacMid1 après électroporation d'un fragment de recombinaison de 4222 bp dans lequel une partie des gènes codant la gp37 (252 acides aminés) et la DNAPol (466 acides aminés C-terminaux) a été délétée et remplacée par une cassette d'expression bactérienne permettant la production d'hygromycine B phosphotransférase (Hygro^{R}) conférant ainsi la résistance à l'hygromycine (Hygro^{R}). Le gène Hygro^{R} a été placé sous le contrôle du promoteur bactérien EM7 (issu du vecteur commercial pSelect-Hygro-mcs, Invitrogen), le terminateur glms a été introduit en aval du gène Hygro^{R} *(*Gay N.J. et al. Biochem J., 1986, 234, 111-117). Les bactéries contenant le BacMid2 *(E. coli* EL350/BacMid2) ont été sélectionnées pour leur résistance à l'hygromycine. Le génome de baculovirus (i.e. le BacMid2) est déficient pour la réplication en cellules d'insectes, car deux gènes essentiels à la réplication virale (i.e. le gène codant la protéine 1629 et le gène codant la DNAPol) sont non-fonctionnels.

La **Figure 2** est un schéma qui illustre l'étape de délétion d'une partie du gène DNAPol pour la préparation du BacMid2.

**Note :** On peut utiliser le BacMid2 pour produire une seule protéine (voir Exemple 4). Il suffit d'avoir deux vecteurs de transfert, l'un apportant le transgène et tout ou partie du gène essentiel délété 1 et l'autre apportant le gène sauvage correspondant au gène essentiel délété 2. Les deux gènes délétés sont réparés lors de la recombinaison homologue.

### Exemple 3 : Construction d'un génome de baculovirus déficient pour la réplication dans lequel 3 gènes essentiels à la réplication virale sont non-fonctionnels (BacMid3)

Le BacMid3 présente la délétion de 3 gènes essentiels, 1629, DNAPol et gp64.

A partir du BacMid2, la délétion du gène gp64 a été effectuée dans les bactéries *E coli* EL350/BacMid2 après électroporation d'un fragment de recombinaison de 3260pb dans lequel la totalité du gène de la cathepsine plus 779 bp de la séquence codant 259 acides aminés de la chitinase et une partie du gène de la gp64, délétion de 566 bp codant 188 acides aminés, a été remplacée par une cassette d'expression bactérienne conférant une résistance à la zéocine (Zeo^{R})(Drocourt et al., Nucleic Acids Research, vol.18 n°13, 1990*).* Le gène Zeo^{R} issu du plasmide commercial pCR^{®}-Blunt (Invitrogen) a été placé sous contrôle du promoteur bactérien T5N25, issu du phage T5 *(*Gentz and Bujard, J. Bacteriology, vol.164 n°1, 1985*)* et suivi du terminateur de transcription rrnBT1 (*E.coli* ribosomal RNA opéron T1 terminator) *(*Kwon et al., J Biol. Chem., vol 274 n°41, 1999*).* Les bactéries contenant le BacMid3 *(E. coli* EL350/BacMid3) ont été sélectionnées pour leur résistance à la zéocine. Le génome de baculovirus (i.e. le BacMid3) est déficient pour la réplication en cellules d'insectes, car trois gènes essentiels à la réplication virale (i.e. le gène codant la protéine 1629 et le gène codant la DNAPol et le gène codant la gp64) sont non-fonctionnels.

La **Figure 3** est un schéma qui illustre l'étape de délétion de gp64 pour la préparation du BacMid3.

### Exemple 4 : Utilisation du BacMid2

Un vecteur de transfert pVT/gp37 a été construit pour pouvoir générer des baculovirus recombinants exprimant 2 transgènes. Pour cela, le fragment EcoRI F du génome de baculovirus AcMNPV contenant le gène gp37 et le gène DNAPol a été cloné dans un plasmide bactérien pUC, générant ainsi le pUC/gp37.

Ce plasmide a ensuite été modifié de la façon suivante : une grande partie du gène codant la gp37 a été délété (724pb), l'ATG initiateur a été muté et remplacé par deux sites de restriction uniques XbaI et AvrII permettant l'intégration d'un transgène sous contrôle du promoteur naturel de la gp37. Ces modifications ont ainsi conduit à l'obtention du vecteur de transfert pVT/gp37.

Les cellules Sf9 ont été transfectées par lipofection avec les vecteurs de transfert pVT/PH et pVT/gp37 chargés avec les transgènes et l'ADN du BacMid2. Les virus générés après la recombinaison homologue ont été clonés par la méthode des plages de lyse. La production de la protéine recombinante a été vérifiée par une méthode adaptée (e.g. par exemple ELISA, Western blot, dosage enzymatique). Le génome des virus recombinants a été vérifié par Southern blot et la séquence du transgène intégré dans le génome viral a été vérifiée par séquençage après amplification PCR.

La **Figure 4** est un schéma qui illustre le vecteur de transfert pVT/gp37 pour l'expression d'un gène X (où X est un gène différent du gène codant la chaine lourde d'un anticorps).

Les génomes de baculovirus recombinants générés après recombinaison homologue entre le BacMid2 et les vecteurs de transfert n'expriment plus la gp37 (protéine non-essentielle à la réplication virale).

Pour que l'ADN viral soit réparé dans les 2 locus du BacMid2, une seconde recombinaison doit avoir lieu avec un vecteur de transfert PH chargé ou non avec un transgène. Dans tous les cas, l'ADN du génome de baculovirus sera réparé et donc infectieux.

Il sera possible également d'utiliser le pVT/PH contenant une séquence sauvage, c'est-à-dire contenant la cassette d'expression sauvage (non modifiée) conduisant à la production de polyédrine. Le pVT/PH pourra également être « vide » c'est-à-dire ne pas contenir de transgène ni le gène polyédrine.

De la même manière il sera possible d'intégrer le transgène dans le locus PH. Dans ce cas, un pVT/gp37 non délété (gène non essentiel fonctionnel) ou délété totalement ou partiellement tel que décrit dans la **Figure 4** sera utilisé pour réparer le locus gp37/DNApol. Il est à noter que la séquence du gène gp37 qui est présent dans le pVT/gp37 décrit dans la **Figure 4****,** a été modifiée, l'ATG initiateur (ATGi) a été muté et le gène gp37 a été délété de 240 acides aminés, comme expliqué ci-après :

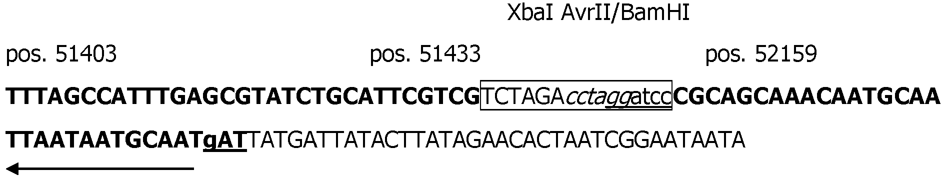
XbaI AvrII/BamHI
pos. 51403 pos. 51433 pos. 52159
TTTAGCCATTTGAGCGTATCTGCATTCGTCGTCTAGAcctaggatccCGCAGCAAACAATGCAA
TTAATAATGCAATgATTATGATTATACTTATAGAACACTAATCGGAATAATA

### Sens de transcription du gène

### Légende :

Séquence du gène en gras
ATG initiateur souligné
Polylinker XbaI/AvrII/BamHI en encadré
La séquence nucléique présentée ci-dessus est la séquence SEQ ID NO : 16

La **Figure 5** est un schéma qui illustre la construction et l'utilisation du vecteur de transfert PH pVT/PH pour l'expression d'un transgène X (où X est un transgène différent du gène codant une chaine légère d'un anticorps).

### Expression de la chaine lourde d'un anticorps.

### Construction d'un pVT/gp37 spécifique, le pVT/gp37-Cγ1

Ce vecteur de transfert contient la cassette d'expression suivante :
- Promoteur viral P10 sauvage (SEQ ID NO :1)
- Séquence d'ADN codant une séquence signal d'une immunoglobuline humaine (séquence de sécrétion)
- 2 sites de restriction uniques pour le clonage en phase de la région variable (VH) de l'anticorps (région qui donne la spécificité de l'anticorps)
- Séquence d'ADN qui code une région constante d'IgG (γ1-4) epsilon, mu, ou alpha humaine.

La **Figure 6** est un schéma qui illustre la construction et l'utilisation du vecteur de transfert pVT/gp37Cγ1 pour l'expression de la chaine lourde d'un anticorps.

### Expression de la chaine légère d'un anticorps.

### Construction d'un pVT/PH spécifique, le pVT/PH-CL.

Ce vecteur de transfert contient la cassette d'expression suivante :
- Promoteur viral P10 P10S1B (SEQ ID NO : 3)
- Séquence d'ADN codant une séquence signal d'une immunoglobuline humaine (séquence de sécrétion)
- 2 sites de restriction uniques pour le clonage en phase de la région variable (VL) de l'anticorps (région qui donne la spécificité de l'anticorps)
- Séquence d'ADN qui code une région constante de chaine légère (CL) kappa (κ) ou lambda (λ) d'IgG humaine.

La **Figure 7** est un schéma qui illustre la construction et l'utilisation du vecteur de transfert pVT/PHC· pour l'expression de la chaine légère d'un anticorps.

### Exemple 5 : Utilisation du BacMid3.

Un vecteur de transfert, pVT/Chit-Cath a été construit pour pouvoir générer des génomes de baculovirus recombinants exprimant 3 transgènes.

Le fragment BstXI-XbaI issu des régions EcoRI E et H du baculovirus AcMNPV a été cloné dans un plasmide pUC. Une délétion EcoNI-EcoRI de 1175 pb permet d'inactiver les gènes codant la chitinase, non essentielle et la cathepsine non essentielle également. L'addition d'un site XbaI entre les sites EcoNI et EcoRI permet d'intégrer un transgène. Ces modifications ont ainsi conduit à la l'obtention du vecteur de transfert pVT/Chit-Cath.

Les cellules Sf9 sont transfectées par lipofection avec les vecteurs de transfert pVT/PH, pVT/gp37 et pVT/chitCath chargés avec les transgènes et l'ADN du BacMid3. Les virus générés au cours de la recombinaison homologue ont été clonés par la méthode des plages de lyse. La production de la protéine recombinante a été contrôlée par une méthode adaptée, ELISA, Western blot, dosage enzymatique ... le génome des virus recombinants a été contrôlé par Southern blot et la séquence du transgène a été contrôlée après amplification PCR.

La **Figure 8** est un schéma qui illustre la construction du vecteur pVT/Chit-Cath et sa recombinaison homologue avec le BacMid3.

### Exemple 6 : Production d'un anticorps monoclonal anti-CD4 (13BBII) en utilisant le BacMid2.

Les ADNc codant les régions VH et VL de l'anticorps ont été intégrées respectivement dans les vecteurs de transfert pVT/PH-C· et pVT/gp37-Cγ1. Des baculovirus recombinants ont été générés après recombinaison homologue entre les 2 pVT et l'ADN du BacMid2 de l'Exemple 4 :
L'ADNc codant la région VL de l'anticorps a été introduit dans le pVTPH/Ck qui recombine avec la région PH/1629 du BacMid2,
L'ADNc codant la région VH de l'anticorps a été cloné dans le pVT/gp37-Cγ1 qui recombine avec la région gp37 du BacMid2.

Les cellules Sf9 ont été transfectées par lipofection avec le BacMid2 et les 2 vecteurs de transfert obtenus dans l'Exemple 4 puis incubées pendant 4 jours à 28°C. Les surnageants de culture ont été prélevés et les baculovirus recombinants générés, sécrétés dans le milieu de culture ont été clonés par la technique des plages de lyse.

L'organisation du génome des baculovirus recombinants a été contrôlée par Southern blot (voir **Figure 9A****)** et les transgènes intégrés (i.e. VL et VH) ont été vérifiés après amplification PCR, clonage puis séquençage. Les anticorps recombinants sécrétés dans le milieu de culture ont été purifiés sur colonne de Protéine A Sepharose (GE Healthcare) puis analysés après migration en gel de polyacrylamide et coloration à l'argent **(****Figure 9B****).**

### Exemple 7 : Utilisation du BacMid3 pour la production de VLP (Virus-Like-Particle).

### Production de VLP de grippe

Pour produire ces VLPs, les 3 gènes du virus de la grippe, M, HA et NA ont été co-exprimés. Ces 3 gènes ont été intégrés dans les trois vecteurs de transfert nécessaires pour recombiner avec le BacMid3 de l'Exemple 5 :
Le gène M a été introduit dans le vecteur de transfert pVT/PH comme décrit dans la **Figure 5****.**
Le gène HA a été introduit dans le vecteur de transfert pVT/gp37 comme décrit dans la **Figure 4****.**
Le gène NA a été introduit dans le vecteur de transfert pVT/Chit/Cath comme décrit dans la **Figure 8****.**

Les cellules Sf9 ont été transfectées par lipofection avec le BacMid3 et les 3 vecteurs de transfert obtenus ci-dessus, puis incubées pendant 4 jours à 28°C. Les baculovirus recombinants générés puis sécrétés dans le surnageant de culture ont été clonés par la méthode des plages de lyse.

L'organisation des génomes de baculovirus recombinants a été contrôlée par Southern blot (voir **Figure 10****)** et les gènes intégrés ont été vérifiés après amplification PCR, clonage puis séquençage. Le Southern blot a été réalisé sur l'ADN génomique du virus recombinant exprimant les 3 protéines HA, NA et M du virus de la grippe. Cette expérience réalisée avec des sondes spécifiques de ces 3 gènes, a permis de détecter la présence des ADNc codant les 3 protéines dans le génome de baculovirus recombinant.

### Exemple 8 : Utilisation du BacMid3 pour la production d'anticorps bispécifiques.

L'anticorps bispécifique construit selon la demande internationale WO 2013/005194 est constitué d'une chaine lourde composée des domaines VH+CH1+CH2+CH3 d'un anticorps 1, fusionnée en N-terminal aux domaines VH+CH1 d'un anticorps 2. Des mutations introduites à l'interface des régions CL et CH1 de l'anticorps 1 favorisent les appariements corrects entre les domaines VL1 et VL2 des chaines légères L1 et L2 qui sont produites séparément et les domaines VH1 et VH2 correspondants. La production de cet anticorps nécessite la production simultanée et en quantité égale de 3 chaines, la chaine lourde fusionnée, la chaine légère L1 et la chaine légère L2.
L'ADNc codant la chaine légère L1 a été introduit dans le vecteur de transfert pVT/PH comme décrit dans la **Figure 5****.**
L'ADNc codant la chaîne légère L2 a été introduit dans le vecteur de transfert pVT/gp37 comme décrit dans la **Figure 4****.**
L'ADNc codant la chaine lourde fusionnée a été introduit dans le vecteur de transfert pVT/Chit-Cath comme décrit dans la **Figure 8****.**

**La** **Figure 11** est un schéma qui illustre en A la structure de l'anticorps bispécifique en B l'analyse par électrophorèse en gel de polyacrylamide de l'anticorps bispécifique purifié sur colonne de Protéine A Sepharose (GE Healthcare).

### Exemple 9 : Construction du BacMid2-Fur permettant la génération de baculovirus recombinants exprimant des protéines mannosylées correctement maturées.

Le Principe général qui a été utilisé pour introduire dans les bacmids les gènes permettant d'optimiser les modifications post traductionnelles des protéines (BacMid2/MPT (MPT : Modification Post-Traductionnelle) est décrit dans la Figure 14 et détaillée dans l'Exemple 10. Lorsque plusieurs gènes sont nécessaires, ils sont intégrés de manière itérative dans des régions ou gènes non essentiels à la réplication du virus. Le Tableau 2 ci-dessous décrit les sites d'intégration et la nature des gènes intégrés dans les différents bacmids qui ont été construits.

**Tableau 2.**

| Site d'intégration dans le génome viral | Promoteur utilisé pour contrôler l'expression | | Nom du gène impliqué dans la modification post traductionnelle | | |
|---|---|---|---|---|---|
| | *Origine du promoteur* | *ARN polymerase utilisée* | *Gene* | *Origine du gène* | *Référence* |
| *Intergénique v-ubi (orf35)* /*39k (orf36)* pos. 29226 | P9 promoter of *Jc*NDV | Cellulaire | GNT-II | Humaine | Tan et al. 1995 |
| *egt (orf15)* pos. 12786 Integration dans le gène *egt* | Promoteur du gène *gp64* de *Op*MNPV | Cellulaire et virale | β1,4GalTI | Bovine | d'Agostaro et al. 1989 |
| *iap2 (orf71)* pos. 61222 Intégration dans le gène *iap2 e*t délétion de 335nt (112 aa) de *iap2* | Promoteur du gène *ie1* de *CfMNPV* | Cellulaire | *CMP NeuAc synthase* | Humaine | Munster et al.1998 |
| | Promoteur du gène *ie1* de *LdMNPV* | Cellulaire | NeuAc synthase | Humaine | Lawrence et al. 2000 |
| *Intergénique orf51*/*orf52* pos. 44298 | Promoteur du gène *ie1* du WSSV | Cellulaire | ST3GalIV | Humaine | Kitagawa and Paulson, 1994 |
| | Promoteur du gène *ie1* de WSSV | Cellulaire | ST6GalI | Humaine | Grundmann et al. 1990 |
| | Promoteur du gène *actine 3* de *B. mori* et promoteur du gène *ie1* de WSSV | Cellulaire | ST3GalIV + ST6GalI | Humaine | |
| *pif1 (Orf119)* pos. 100697 Intégration à la place du gène *pif1* qui est entièrement délété | Promoteur du gène *ie1* du WSSV | Cellulaire | ST6GalI | Humaine | |
| *chit*/*cath (orf126*/*orf127)* pos. 106160 Délétion de 787nt de *chit*(263 aa N-terminal) et 342nt de *cath* (114 aa N-terminal) | Promoteur synthétique P10S1 | Virale | *Sf9-fur* | Lépidoptères Cellule Sf9 | Cieplik et al. 1998 |

| | | | | | |
|---|---|---|---|---|---|
| Numérotation des bases conforme à la séquence du virus AcMNPV déposée dans GenBank sous la référence « NC_001623, Autographa californica nucleopolyhedrosis genome, complete sequence» ***Légende* du tableau 2 :** Les gènes impliqués dans l'élaboration des modifications post-traductionnelles (exemple : glycosylation, clivage endoprotéolytique) ont été insérés dans des gènes/régions non essentiels des bacmids. Excepté dans le cas de la surexpression de la furine cellulaire qui est produite sous le contrôle d'un promoteur tardif fort P10S1, les promoteurs utilisés pour contrôler l'expression de ces gènes sont précoces de manière à produire ces enzymes avant la biosynthèse des protéines d'intérêt qui seront exprimées sous le contrôle de promoteurs tardifs. | | | | | |

Le BacMid2-Fur a été construit à partir du BacMid2 obtenu à l'Exemple 2. Le gène codant la furine de la cellule de lépidoptère Sf9 *(fur)* a été cloné en aval d'un promoteur tardif synthétique P10S1 de séquence :

5’-ATAAGTATTTTAATCTTTTCGTTTGTATATTAATTAAAATACTATACTGTATAAAAAAACCTATAAA

TATCCCGGATTATTCATACCGTCCCACCATCGGGCGTACGCCACC-3’ (SEQ ID NO : 17).

Le gène *fura* été intégré dans le locus chitinase-cathepsine. Le vecteur de transfert, pVT/Chit-Cath dont la construction est décrite dans l'Exemple 5 a été utilisé. La cassette d'expression comprenant le gène fursous contrôle du promoteur synthétique P10S1 a été introduite au site unique XbaI du pVT/Chit-Cath (position 106160 dans le génome du baculovirus), pour donner le plasmide pVT/Chit-Cath-Fur. Le gène *fur* a été cloné dans le même sens que le gène cathepsine inactivé.

Une cassette d'expression bactérienne «zéocine resistance (*Zeo^{R}*)» composée comme suit : [*Promoteur bactérien T5N25-Zeo^{R}-terminateur rrnBT1*] contenant un site Bsu36I de part et d'autre a été cloné au site EcoRI du pVT/Chit-Cath-Fur, pour donner le plasmide pVT/Chit-Cath-Fur-*Zeo^{R}*. Cette seconde cassette permet l'expression du gène Zeo^{R} et confère ainsi à la bactérie porteuse de ce plasmide la résistance à la zéocine.

Le fragment de recombinaison de 5927 pb a été préparé après digestion du plasmide pVT/Chit-Cath-Fur-Zéo^{R} par BglII générant ainsi des régions flanquantes pour la recombinaison homologue de 652 pb et 704 pb de part et d'autre du fragment. Après électroporation dans la bactérie EL350/BacMid2, les bactéries ont été sélectionnées sur zéocine. Comme décrit dans la **Figure 14****,** la cassette d'expression bactérienne Zéo^{R} a été éliminée par digestion Bsu36I, réparation puis ligation.

On a ainsi obtenu le BacMid2/Fur. Les génomes de ces nouveaux BacMids ont été contrôlés par Southern (Figure 15) puis par séquençage du gène *fur* intégré.

### Exemple 10: Construction du BacMid2-Gal permettant la génération de baculovirus recombinants exprimant des protéines galactosylées.

Le BacMid2-Gal a été construit à partir du BacMid2 obtenu à l'Exemple 2. Les ADNc codant 2 glycosyltransférases manquantes dans les cellules de lépidoptères et nécessaires à la biosynthèse des glycannes galactosylés, la *N*-acétylglucosaminyltransférase II humaine (GNT-II) (EC 2.4.1.143, Accession n° NM_002408.3) et la β 1,4 galactosyltransférase (β1,4GalT) bovine (EC 2.4.1.38, Accession n° NM_177512.2) ont été introduits dans des gènes ou régions non essentiels du BacMid2 par recombinaison homologue. De manière à ce que les activités enzymatiques de β1,4GalT et de GNT-II soient exprimées avant la synthèse du ou des transgène(s) d'intérêt codant un polypeptide d'intérêt, les transgènes codant GNT-II et β1,4GalT ont été clonés en aval de promoteurs précoces viraux tel que décrit dans le **tableau 2.**

**La** **Figure 14** illustre de manière générale les différentes étapes nécessaires à la construction d'un BacMid comprenant un ou plusieurs transgène(s) codant chacun une enzyme de maturation des protéines BacMid2/MPT. En particulier dans la **Figure 14****,** le transgène codant l'enzyme de maturation des protéines est le gène codant la GNTII (gène GNTII).

L'addition des transgènes codant respectivement la GNT-II et la β1,4GalT a été effectuée de manière itérative dans le BacMid2.

### - Insertion du transgène GNT-II

Ce transgène a été introduit en position 29226 du génome viral par recombinaison homologue entre les orf35 (v-ubi) et orf36 (39k) désignée région intergénique IG35/36. Pour pouvoir insérer la cassette d'expression dans le génome du BacMid, les sites uniques de clonage XbaI (italique) et Bsu36I (souligné) ont été intégrés par PCR dans la région IG35/36 avec les amorces suivantes :
anti-Sens ig35/36
   5'-CCTGGTAATTTTTGACCACGG-3' (position 28806 dans le génome viral) (SEQ ID NO : 7)
   et
Sens mut ig35/36
   5'-GCCTTAGG*TCTAGA*GTATATTTAATGGTTTTTATTATTGTTATTATTAATACCCTCC-3' (SEQ ID NO : 6)
   puis
Anti-sens mut ig35/36
   5'-C *TCTAGA*CCTAAGGCATAAAAGTTTTTTATTTAATCTGACATATTTGTATCTTGTGTATTATCGC-3' (SEQ ID NO : 5)
   et
Sens ig35/36
   5'-CGCAGCAATTCCAGCGAGC-3' (position 29657 dans le génome viral) (SEQ ID NO : 4)

Le fragment PCR obtenu de 861 bp a été cloné dans un plasmide pGEM^{®}Teasy et contrôlé par séquençage, pour donner le plasmide pGEM-IG35/36.

Deux cassettes d'expression ont été introduites dans le plasmide pGEM-IG35/36 ci-dessus.

Une cassette d'expression virale, composée comme suit [*Promoteur P9 du densovirus -JcNDV - transgène codant GNTII - stop TkpA*] a été insérée au niveau du site XbaI, pour donner le plasmide pGEM-IG35/36-GNTII. Le Promoteur P9 du densovirus JcNDV est décrit dans Shirk PD, Bossin H, Furlong RB, Gillett JL. Regulation of Junonia coenia densovirus P9 promoter expression. Insect Mol Biol. 2007 Oct;16(5):623-33. Epub 2007 Aug 22.

La cassette d'expression bactérienne « zeocine resistance » (Zeo^{R}) (obtenue à partir du plasmide commercial pCR^{®}Blunt, InVitrogen) composée comme suit : [*Promoteur bactérien T5N25 - Zeo^{R}- terminateur rrnBT1]* a été cloné au niveau du site Bsu36I. Cette seconde cassette permet l'expression du gène Zeo^{R} et conférera ainsi à la bactérie porteuse la résistance à la zéocine, pour donner le plasmide pGEM-IG35/36-GNTII-Zeo^{R}. Le promoteur bactérien T5N25 est décrit dans Gentz R, Bujard H. Promoters recognized by Escherichia coli RNA polymerase selected by function: highly efficient promoters from bacteriophage T5. J Bacteriol. 1985 Oct;164(1):70-7. Le terminateur de transcription rrnBT1 est décrit dans Kwon YS, Kang C. Bipartite modular structure of intrinsic, RNA hairpin-independent termination signal for phage RNA polymerases. J Biol Chem. 1999 Oct 8;274(41):29149-55.

Le fragment de recombinaison de 3493 bp <IG35/36-*GNTII*-*Zeo^{R}*> obtenu après digestion par EcoRI du plasmide généré ci-dessus pGEM-IG35/36-GNTII-*Zeo^{R}* et présentant des régions flanquantes pour la recombinaison homologue de 420 bp et 428 pb de part et d'autre du fragment de recombinaison, a été électroporé dans les bactéries EL350/BacMid2. Les bactéries contenant le BacMid2/GNTII-Zeo^{R} (*E coli* EL350/BacMid2/GNTII-Zeo^{®}) ont été sélectionnées pour leur résistance à la kanamycine, l'hygromycine et la zéocine. L'ADN de 3 clones de Bacmid2/GNTII-Zeo^{®} sélectionnés a été extrait puis les gènes GNT-II et Zeo^{R} insérés dans la région IG35/36 ont été contrôlés par PCR puis séquençage.

La cassette d'expression bactérienne flanquée de part et d'autre d'un site Bsu36I a ensuite été éliminée par simple digestion par Bsu36I, réparation des extrémités de l'ADN avec l'ADN polymérase de Klenow puis ligation du plasmide sur lui-même. Il convient de noter que la séquence Bsu36I « réparée » [5' CCTNATNAGG 3'] a été conservée dans le Bacmid2/GNTII ainsi généré après ligation du plasmide. Cette séquence est donc présente dans le baculovirus recombinant et elle peut constituer une signature spécifique.

Le transgène codant la GNTII a été cloné dans le même sens que le gène 39K.

Le BacMid2/GNTII a ainsi été obtenu puis contrôlé comme décrit plus haut avant d'être utilisé pour l'insertion du gène codant la β1,4GalT.

### - Insertion du gène β1,4GalT

Le transgène codant la β1,4GalT a été intégré dans le locus du gène non essentiel *egt* (Ecdysteroïd glycosyltransferase, ORF15, position dans le génome position 11426 - 12946 du génome viral AcMNPV) du BacMid2/GNTII selon le principe général décrit ci-dessus. Le fragment PstI-BamHI de 5110 bp (position 9999 à 15110 dans le génome viral d'AcMNPV) contenant le gène *egt,* a été préalablement cloné dans un plasmide pUC pour donner le plasmide pUC-EGT. Puis la cassette d'expression virale comprenant l'ADNc codant la β1,4GalT bovine sous contrôle du promoteur gp67 d'OpMNPV a été introduite dans le gène *egt* par insertion (inactivation du gène par insertion) au site unique XbaI (position 12782 dans le génome du baculovirus) présent dans la séquence codante du gène *egt,* pour donner le plasmide pUC-EGT-GalT. Le transgène codant la β1,4GT a été cloné dans le même sens que le gène egt.

Un adaptateur NsiI-Bsu36I-NsiI a ensuite été inséré dans le site NsiI situé en aval du gène β1,4GalT, ce qui a permis l'introduction de la cassette d'expression bactérienne Zéo^{R} en Bsu36I générant le plasmide pUC-EGT-GalT-Zéo^{R}.

Le fragment de recombinaison de 3128 bp a été préparé après digestion du plasmide ci-dessus pUC-EGT-GalT-Zéo^{R} par SnaBI-NruI générant ainsi des régions flanquantes pour la recombinaison homologue de 474 bp et 866 pb de part et d'autre du fragment. Après électroporation dans la bactérie EL350/BacMid2-GNTII, les bactéries ont été sélectionnées sur zéocine. Comme précédemment, La cassette d'expression bactérienne Zéo^{R} a été éliminée par digestion Bsu36I, réparation puis ligation.

On a ainsi obtenu le BacMid2/GNTII/β1,4GalT (aussi appelé BacMid2-Gal ou BacGal). Le génome de BacMid2-Gal a été contrôlé par Southern puis séquençage de tous les gènes intégrés.

### Exemple 11 : Construction du BacMid2Gal-Fur permettant la génération de baculovirus recombinants exprimant des protéines galactosylées correctement maturées.

Le BacMid2-Gal-Fur a été construit comme décrit pour le BacMid2-Fur (Exemple 9).

Les bactéries EL350/BacMid2-Gal ont été électroporées avec le fragment de recombinaison de 5927 pb décrit dans l'Exemple 9, puis sélectionnées sur zéocine. Comme précédemment, la cassette d'expression bactérienne Zéo^{R} a été éliminée par digestion Bsu36I, réparation puis ligation. On a ainsi obtenu le BacMid2Gal-Fur. Le génome du bacmid a été contrôlé par Southern (Figure 15) puis séquençage du nouveau gène intégré.

### Exemples 12 : Construction du BacMid-Sia3 (ou BacSia3).

Les transgènes codant la CMPNeuAc synthase humaine (CMPNeuAc synthase ou CMPNeuAcS) (EC 2.7.7.43, accession n° NM_018686.5), la NeuAc synthase humaine (NeuAc Synthase ou NeuAcS) (EC 2.5.1.56, accession n° AF257466) et l'α2,3 sialyltransférase humaine (ST3), ST3GalIV (EC 2.4.99.4, accession n° X74570) ont été insérés dans le BacMid2/GNTII-β1,4GT de manière itérative selon le principe général décrit dans la **Figure 14****.**

### • Clonage des deux transgènes codant respectivement la NeuAc synthase et la CMP NeuAc Synthase dans le locus iap2 du BacMid2-Gal.

La Demanderesse a choisi de cloner ces deux enzymes en tête-bêche sous le contrôle de promoteurs très précoces, le promoteur IE1 (immediate-early 1) du baculovirus de *Choristoreura fumiferana* pour le contrôle l'expression du gène CMP NeuAc synthase et celui du baculovirus de *Lymantria dispar* pour le contrôle de l'expression du gène NeuAc synthase (voir **Tableau 2**)

La région comprenant le gène iap2 (ORF71) du baculovirus AcMNPV (Position dans le génome 61016-61765) a été préalablement amplifiée par double PCR avec les amorces suivantes :
Sens iap2 5'- GATATTGTGTGCTCAATGTC-3' (Position 60736 dans le génome viral) (SEQ ID NO : 8)
Anti-sens BstBI 5'- CCTAAGGTCTAGATTCGAATACGTGTGTCG -3' (SEQ ID NO : 9) puis
Sens BstBI 5'- CGAATCTAGACCTTAGGCCGCGGCTAAGCGTTAAACC -3' (SEQ ID NO : 10)
Anti-sens iap2 5'- CGATCACCGTCGCTGTCGTCTTC -3' (Position 61951 dans le génome viral) (SEQ ID NO : 11)

Ces PCR successives ont également permis (i) d'intégrer les sites uniques Bsu36I (en souligné ci-dessus) et XbaI (en double souligné ci-dessus) et (ii) de déléter une grande partie de la séquence codant iap2, délétion de 335 bp/112 acides aminés. Le fragment amplifié de 896 bp a été cloné dans un plasmide pGEM^{®}Teasy, pour donner le plasmide pGEM-IAP2.

Une cassette d'expression virale, composée comme suit [*Stop SV40-CMPNeuAc Synthase-Promoteur IE1Cf-Promoteur IE1Ld-NeuAc Synthase]* a été insérée au site XbaI du pGEM-IAP2, pour donner le plasmide pGEM-iap2-CMPNeuAcS-NeuAcS.

La cassette d'expression bactérienne «zeocine resistance » (*Zeo^{R}*) composée comme suit : *[Promoteur bactérien T5N25-Zed^{R}-terminateur rrnBT1*] a été cloné au site Bsu36I du pGEM-iap2-CMPNeuAcS-NeuAcS, pour donner le plasmide pGEM-iap2-CMPNeuAcS-NeuAcS-*Zeo^{R}*. Cette seconde cassette permet l'expression du gène Zeo^{R} et confère ainsi à la bactérie porteuse de ce plasmide la résistance à la zéocine.

Le fragment de recombinaison [*CMPNeuAc-NeuAcS-Zeo^{R}*] de 4548 pb a été préparé après digestion du plasmide pGEM-iap2-CMPNeuAcS-NeuAcS-*Zeo^{R}* par l'endonucléase de restriction NotI qui génère des régions flanquantes pour la recombinaison homologue de 486 bp et 396pb de part et d'autre du fragment. Les bactéries EL350/BacMid2-GNTII-β1,4GT ont été électroporées avec le fragment de recombinaison générant ainsi le BacMid2-GNTII-β1,4GT-CMPNeuAcS-NeuAcS-Zéo^{R}. Comme précédemment, la cassette de résistance à la zéocine a été éliminée après digestion par Bsu36I, réparation puis religation. On a ainsi obtenu le BacMid2-GNTII-β1,4GT-CMPNeuAcS-NeuAcS.

### • Clonage du transgène a2,3-sialyltransférase IV (ST3GalIV) dans la région intergénique comprise entre les orf51 et orf52 (IG51/52) du BacMid2-GNTII-β1,4GT-CMPNeuAcS-NeuAcS.

Cette région localisée dans le fragment EcoRI N du baculovirus *Ac*MNPV a été isolée après amplification par double PCR avec les amorces suivantes :
Sens IG51/52 5'- GGAAAACTCTTTCCGAAGACGAAC (position 43814 dans le génome viral) (SEQ ID NO : 12) et
Anti-sens Xba/IG51/52 5'-CCTAAGGTCTAGAGTGCCTTTTGTTTGCTATTTTGCGCCG-3' (SEQ ID NO : 13)
   puis
Sens Xba/IG51/52 5'-CTCTAGACCTTAGGTCCGCGCTCTCCCACGC-3' (SEQ ID NO : 14) et
Anti-sens IG51/52 5'- GGTGCAGAACATAATGACGTGGCCTTAC (position 44723 dans le génome viral) (SEQ ID NO : 15)

Au cours de ces PCR successives il y a addition de 2 sites uniques Bsu36I (en souligné ci-dessus) et XbaI (en double souligné ci-dessus) dans la région intergénique ORF51/ORF52 ce qui permettra une intégration de la cassette d'expression ST3 au site XbaI en position 44298 dans le génome viral. Le fragment obtenu de 922 bp a été cloné dans un pGEM^{®}T easy (Promega), pour donner le plasmide pGEM-IG51/52.

Comme pour les autres enzymes, la ST3GalIV doit être présente dans les cellules avant que les glycoprotéines d'intérêt soient exprimées. Nous avons choisi le promoteur IE1 du virus de crevette WSSV (White Spot Syndrome Virus) identifié comme étant un promoteur fonctionnel en cellule *Sf*9 de type « immediate-early » (Voir **Tableau 2**)(Liu and al., Virology, 2005 ; Liu and al. J of virology, 2007 ; Gao and al., J. Biotechnology, 2007).

Une cassette d'expression virale, composée comme suit *[promoteur WSSV-ST3*] a été insérée au site XbaI du pGEM-IG51/52, pour donner le plasmide pGEM-IG51/52-ST3. La ST3GalIV a été clonée dans le sens inverse de l'orf51.

La cassette d'expression bactérienne «zéocine resistance (*Zeo^{R}*)» composée comme suit : *[Promoteur bactérien T5N25-Zec-terminateur rrnBT1]* a été cloné au site Bsu36I du pGEM-ORF51-ST3, pour donner le plasmide pGEM-IG51/52-ST3-*Zeo^{R}*. Cette seconde cassette permet l'expression du gène Zeo^{R} et confère ainsi à la bactérie porteuse de ce plasmide la résistance à la zéocine.

Un fragment de recombinaison [*ST3GalIV-Zeo^{R}*] de 2589 pb a été généré à partir du pGEM-IG51/52-ST3-*Zeo^{R}* par digestion par l'endonucléase de restriction NotI. Cette digestion a généré des régions flanquantes pour la recombinaison homologue de 498 bp et 411pb de part et d'autre de la cassette d'expression. La recombinaison homologue a été réalisée dans la bactérie EL350/BacMid2-GNTII-B1,4GT-CMPNeuAcS-NeuAcS, générant le BacMid2-GNTII-β1,4GT-CMPNeuAcS-NeuAcS-ST3 (ou BacSia3). Le génome du BacSia3 a été contrôlé par Southern puis séquençage de tous les gènes intégrés.

### Exemple 13 : Construction du BacMid-Sia6 (ou BacSia6).

### • Clonage du transgène a2,6-sialyltransferase I (ST6GalI) dans la région intergénique orf51/orf52 (IG51/52) du BacMid2-GNTII-ß1,4GT-CMPNeuAcS-NeuAcS.

La méthode de clonage du gène codant l'a2,6 sialyltransférase humaine, ST6GalI (EC 2.4.99.1, accession n° X17247) est similaire à celle décrite à l'Exemple 12 pour le transgène codant la ST3GalIV, résumée comme suit :
- Une cassette d'expression virale, composée comme suit [*promoteur WSSV*-*ST6*] a été insérée au site XbaI du pGEM-IG51/52, pour donner le plasmide pGEM-IG51/52-ST6. La ST6GalI est clonée dans le sens inverse de l'orf51
- La cassette d'expression bactérienne «zeocine resistance » (*Zeo^{R}*) composée comme suit : *[Promoteur bactérien T5N25-Zeo^{R}*-*terminateur rrnBT1*] a été cloné au site Bsu36I du pGEM-ORF51-ST6 générant le plasmide pGEM-IG51/52-ST6-*Zeo^{R}*. Cette seconde cassette permet l'expression du gène Zeo^{R} et confère ainsi à la bactérie porteuse de ce plasmide la résistance à la zéocine.
- Un fragment de recombinaison [*ST6GalI-Ze^{R}*] de 2825 pb a été généré à partir du pGEM-IG51/52-ST6-*Zeo^{R}* par digestion par l'endonucléase de restriction NotI. Cette digestion a généré des régions flanquantes pour la recombinaison homologue de 498 bp et 411pb de part et d'autre de la cassette d'expression. La recombinaison homologue a été réalisée dans la bactérie EL350/BacMid2-GNTII-B1,4GT-CMPNeuAcS-NeuAcS, générant le BacMid2-GNTII-β1,4GT-CMPNeuAcS-NeuAcS-ST6 (ou **BacSia6).** Le génome de **BacSia6** a été contrôlé par Southern puis séquençage de tous les gènes intégrés.

### Exemple 14 : Construction du BacMid-Sia6II (ou BacSia6-II).

### a. Clonage du transgène a2,6-sialyltransferase I (ST6GalI) dans l'ORF119 (PIF1) du BacMid2-GNTII- β1,4GT-CMPNeuAcS-NeuAcS.

L'ORF119, (position dans le génome 100699 - 102291), codant PIF1, protéine non essentielle à la réplication virale, se situe dans le fragment EcoRI E du baculovirus AcMNPV. Un fragment de part et d'autre du gène a été obtenu après amplification par double PCR avec les amorces suivantes :
Senspif1 5'-GAATACAACGCCACATCTATTCCTAGTACAAC-3' (position 100247 dans le génome viral) (SEQ ID NO : 18) et
pif1bac5' 5' -CTAGAGGCGTTAACCTAAGGTACTTATTGGAGAATGTCCGAGT ATTTTTG- 3' (SEQ ID NO : 19)
   puis
pif1for3' 5'- CCTTAGGTTAACGCCTCTAGAACATGAGCATTTTAAAAGTTGTAGAAGCG-3' (SEQ ID NO : 20) et
RevPif1 5'- CATTAACAATTACTACGGCGCATTTTGACCATC-3' (position 102825 dans le génome viral) (SEQ ID NO : 21)

Ces PCR successives ont permis, d'éliminer la totalité de l'ORF119, d'intégrer les sites uniques Bsu36I (en souligné ci-dessus) et XbaI (en double souligné ci-dessus). Le site XbaI permettra l'intégration de la cassette d'expression ST6 en position 100697 dans le génome virale. Le fragment obtenu de 998 bp a été cloné dans un pGEM^{®}T easy (Promega), pour donner le plasmide pGEM-PIF1.

La cassette d'expression virale, décrite dans l'exemple 12 *[promoteur WSSV-ST6*] a été insérée au site XbaI du pGEM-PIF1, pour donner le plasmide pGEM-PIF1-ST6. La ST6GalI a été clonée dans le sens de pif1.

La cassette d'expression bactérienne «zéocine resistance (*Zeo^{R}*)» composée comme suit : *[Promoteur bactérien T5N25-Zec-terminateur rrnBT1]* a été cloné au site Bsu36I du pGEM-PIF1-ST6, pour donner le plasmide pGEM-PIF1-ST6-*Zeo^{R}*. Cette seconde cassette permet l'expression du gène Zeo^{R} et confère ainsi à la bactérie porteuse de ce plasmide la résistance à la zéocine.

Un fragment de recombinaison [*ST6GalI*-*Zeo^{R}*] de 2903 pb a été généré à partir du pGEM-PIF1-ST6-*Zeo^{R}* par digestion par l'endonucléase de restriction NotI. Cette digestion a généré des régions flanquantes pour la recombinaison homologue de 498 bp et 411pb de part et d'autre de la cassette d'expression. La recombinaison homologue a été réalisée dans la bactérie EL350/BacMid2-GNTII-B1,4GT-CMPNeuAcS-NeuAcS, générant le BacMid2-GNTII-β1,4GT-CMPNeuAcS-NeuAcS-ST6-II (ou BacSia6-II). Le génome du BacSia6-II a été contrôlé par Southern (Figure 19B) puis séquençage de tous les gènes intégrés.

### Exemple 15 : Construction du BacMid-Sia3/6 ou BacSia3/6.

### • Clonage des transgènes ST6GalI et ST3GalIV en tête bèche dans la région intergénique orf51/orf52 (IG51/52) dans le BacMid2-GNTII-β1,4GT-CMPNeuAcS-NeuAcS.

La méthode de clonage est similaire à celle décrite aux Exemples 12 et 13 pour le transgène codant la ST3GalIV et le transgène codant la ST6GalI, résumée comme suit :
- Une cassette d'expression virale comprenant un transgène codant ST6GalI et un transgène codant ST3GalIV, composée comme suit *[ST6-promoteur WSSV⁻promoteur Actine 3 B. mori-ST3-Stop actine 3*] a été insérée au site XbaI du pGEM-IG51/52, pour donner le plasmide pGEM-IG51/52-ST3/ST6.
- La cassette d'expression bactérienne «zéocine résistance » (*Zeo^{R}*) composée comme suit : *[Promoteur bactérien T5N25-Zeo^{R}-terminateur rrnBT1]* a été cloné au site Bsu36I du pGEM-IG51/52-ST3/ST6, pour donner le plasmide pGEM-IG51/52-ST3/ST6-*Zeo^{R}*. Cette seconde cassette permet l'expression du gène Zeo^{R} et confère ainsi à la bactérie porteuse de ce plasmide la résistance à la zéocine.

Un fragment de recombinaison [ST3/*ST*6*-Zeo^{R}*] de 5008 pb a été généré à partir du pGEM-IG51/52-ST3/ST6-*Zeo^{R}* par digestion par l'endonucléase de restriction NotI. Cette digestion a généré des régions flanquantes pour la recombinaison homologue de 490 bp et 426pb de part et d'autre de la cassette d'expression. La recombinaison homologue a été réalisée dans la bactérie EL350/BacMid2-GNTII-B1,4GT-CMPNeuAcS-NeuAcS, pour donner le BacMid2-GNTII-β1,4GT-CMPNeuAcS-NeuAcS-ST3/ST6 (ou BacSia3/6). Le génome de BacSia3/6 été contrôlé par Southern puis séquençage de tous les gènes intégrés.

### Exemple 16 : Utilisation du BacMid2-Fur pour la production de la glycoprotéine gp160 du VIH1 mature.

La gp160 du VIH1 doit subir une étape de maturation pour que le virus soit infectieux et que les glycoprotéines de surface du virus s'organisent en trimères. Ces structures sont considérées aujourd'hui comme essentielles pour la formation d'épitopes d'intérêt nécessaires à l'élaboration d'un vaccin contre le VIH-1. Cette maturation est effectuée par la furine cellulaire qui va cliver la gp160 en gp120 + gp41. La production de la gp160 sous une forme recombinante conduit en général à une forme partiellement maturée et cela quel que soit le système d'expression.

De manière à obtenir une gp160 complètement maturée nous avons intégré le gène codant furine de la cellule Sf9 dans le génome viral sous le contrôle d'un promoteur très actif, un promoteur P10-like appelé P10S1 et construit le BacMid2-Fur (Exemple 9).

A partir de ce bacmid, nous avons construit un virus double-recombinant exprimant 2 protéines du VIH1, la polyprotéine Pr55Gag et la gp160. La production de ces 2 protéines conduit à la sécrétion dans le milieu de culture de virus-like-particles (VLP). Dans cette expérience nous avons concentré (noté C et NC pour non concentré) les VLP sécrétées avec une solution de « Retro Concentin^{™} Virus precipitation » (SBI, référence RV100A-1). Les différents échantillons obtenus après infection avec un virus sauvage BACWT ou des virus multirecombinants exprimant la gp160 et la Pr55Gag (BAC/gp160/Gag), la gp160 et la Pr55Gag et la furine (BAC/gp160/Gag ) ou encore des virus mono-recombinants comme le virus BAC gp120 qui exprime uniquement la gp120 soluble et le virus BACGag qui exprime uniquement la polyprotéine Pr55Gag (BACGag) ont été analysés en western blot avec un anticorps anti-gp120, Panel A (anticorps polyclonal de chèvre dirigé contre la gp120 du VIH1, référence Ab21179, Abcam) ou un anticorps anti-Gag, Panel B (Anti-p55 + p24 + p17, Référence Ab63917, Abcam).

Comme le montre la Figure 15C, l'activité de la furine cellulaire n'est pas suffisante pour maturer la totalité de la gp160 qui est produite (*Puits BACgp160*/*Gag*). Par contre, lorsque qu'il y a surexpression de cette enzyme, il y a une maturation complète de la gp160 en gp120 (*Puits* BACgp160/Gag/fur).

### Exemple 17: Utilisation du BacMid2-Gal pour la production d'anticorps galactosylés.

Une *N*-galactosylation terminale étant une caractéristique de la glycosylation de l'Asn297 située dans le domaine constant des IgG **(****Figures 12** **et** **13****),** les exemples décrits ci-dessous concernent la production d'anticorps recombinants.

Le BacMid2-Gal a été utilisé comme le BacMid2 décrit précédemment (Exemples 4 et 6) pour la génération en une seule étape d'un baculovirus double recombinant **(****Figures 16** **et** **17****).** Comme pour le BacMid2, la co-transfection avec le BacMid2-Gal a conduit à un taux très élevé, voisin de 100%, de virus recombinants **(****Figures 9A****).**

### Insertion de transgènes codant les chaines lourde et légère d'un anticorps.

### 1. Principe de la génération des baculovirus recombinants.

Les ADNc codant les régions variables VH et VL de l'anticorps d'intérêt ont été insérés dans les vecteurs de transfert (pVT) baculovirus spécifiques des chaînes lourde et légère des anticorps, pVT/gp37-H (pour le clonage de la région variable de la chaîne lourde) **(****Figure 6****)** et pVT/PH-L (pour le clonage de la région variable de la chaîne légère) **(****Figure 7****).** Ces vecteurs ont été décrits dans l'article (*Juliant et al., 2013).* Les cellules Sf9 ont ensuite été transfectées avec les 2 pVT chargés et de l'ADN BacMid2-Gal. Des virus double-recombinants co-exprimant les chaînes lourde et légère de l'anticorps ont alors été produits en une seule étape **(****Figure 17****).**

### 2. Construction d'un virus recombinant exprimant un anticorps galactosylé.

Des virus recombinants exprimant les mêmes anticorps ont été produits à partir du BacMid2 et du BacMid2-Gal. Les anticorps produits après infection des cellules Sf9 avec le virus recombinant issu du BacMid2 servent de contrôle puisqu'ils présentent une glycosylation « insecte », c'est-à-dire des motifs glycanniques de type de paucimannosidique et dans une moindre proportion de type oligomannosidique **(****Figure 12****).**

### ∘ Clonage dans les vecteurs de transfert.

Les fragments d'ADNc codant les régions variables des chaînes lourde et légère de l'anticorps d'intérêt ont été clonés dans les vecteurs de transfert respectifs (pVT/gp37-H et pVT/PH-L). Au cours de ce clonage, les gènes complets codant les 2 chaînes d'anticorps sont reconstitués **(****Figures 6** **et** **7****).**

### ∘ Génération et clonage des baculo virus recombinants.

Les cellules Sf9 ont été transfectées par lipofection avec les pVT/gp37-H et pVT/PH-L chargés et de l'ADN du Bacmid2-Gal ou du BacMid2 **(****Figure 17****).** Après 7 jours d'infection à 28°C, les baculovirus sécrétés dans le surnageant de culture ont été clonés par la technique des plages de lyse.

### ∘ Contrôle de l'organisation génomique des virus recombinants.

Plusieurs clones baculoviraux ont été sélectionnés, amplifiés et leur génome extrait pour être analysé par Southern blot. Les gènes codant les chaînes lourde et légère insérés dans le génome baculoviral ont également été amplifiés par PCR puis séquencés.

### ∘ Production et purification des anticorps recombinants.

Des cellules Sf9 adaptées à la croissance en milieu sans sérum ont été infectées à une moi de 3 PFU/cellule. Après 3 jours d'infection, le surnageant de culture a été récolté et déposé sur une colonne de Protéine A Sepharose (GE-Healthcare). La qualité des anticorps a été vérifiée après migration en gel de polyacrylamide et coloration argentique.

### ∘ Analyse de la glycosylation par lectine blot.

. Principe du lectine blot : Les lectines sont des molécules qui se fixent spécifiquement sur des motifs glycanniques. Il est ainsi très simple de mettre en évidence la présence d'un glycanne particulier lié à une protéine après électrophorèse en gel de polyacrylamide, transfert sur une membrane de nitrocellulose et incubation de la membrane avec une lectine conjuguée à la biotine (exemple la lectine RCA₁₂₀ biotinylée, référence B1085, Vector Laboratories) ou à la digoxigénine (exemples les lectines du kit « DIG Glycan Differenciation Kit », référence 11210238001, Roche). La présence des lectines est alors détectée indirectement grâce à un anticorps dirigé contre la biotine ou la digoxigénine lui-même conjugué à la peroxydase ou à phosphatase alcaline. La présence de ces enzymes est alors détectée grâce à leur activité enzymatique qui génèrera soit un précipité brun rouge pour la peroxydase ou une coloration bleue pour la phosphatase alcaline.

### ∘ Expérimentation

La production des anticorps a été contrôlée par Western blot. Les protéines ont été séparées par électrophorèse sur un gel de polyacrylamide à 10% en présence de SDS et de 2-mercaptoéthanol puis transférées sur une membrane de nitrocellulose (Protran^{™} 0,45 µm NC, GE Healthcare). Le transfert des protéines a été vérifié après coloration par le rouge ponceau. La membrane a été incubée avec (Figure 18A) un anticorps polyclonal de mouton dirigé contre les IgG humaines et conjugué à la peroxydase (Référence NA933V, GE Healthcare) ou (Figure 18B) avec un anticorps polyclonal de mouton dirigé contre les IgG de souris et conjugué à la peroxydase (Référence NA931V, GE Healthcare). La peroxydase a été révélée par chimioluminescence avec le système SuperSignal^{®} West Pico chemiluminescent Substrate (référence : 34077, Thermo scientific).

Analyse en lectine blot (Figure 18C). Dans cet exemple, nous avons utilisé la lectine RCA₁₂₀ biotinylée (*Ricinus communis* agglutinin) qui se fixe spécifiquement sur les résidus betagalactosyl. Les protéines ont été séparées par électrophorèse sur un gel de polyacrylamide comme décrit plus haut pour le western blot, transférées sur membrane Protran^{™} 0,45 µm NC (GE Healthcare) puis incubée en présence de RCA₁₂₀ biotinylée. La lectine a été révélée indirectement après incubation de la membrane avec un anticorps anti-biotine conjugué à la peroxydase (Anticorps de chèvre, référence A4541, Sigma). La révélation a été effectuée en présence d'un substrat chimioluminescent (SuperSignal^{®} West Pico chemiluminescent Substrate, Thermo scientific)

### 3. Résultats

Des anticorps recombinants humains (Figure 18A) et murins (Figure 18B) ont été produits, purifiés sur Protéine A Sepharose (GE Healthcare) puis analysés en Western blot (Figure 18A et B) et en lectine blot (Figure 18C). Les Figures 18A et B confirment bien la présence des anticorps recombinants humains Puits 2 et 3 et murins : *Puits* 4 et 5. La Figure 18C, montre que seuls les anticorps - humains ou murins - produits par les cellules infectées avec les baculovirus recombinants générés à partir du BacMid2-Gal sont reconnus par le RCA₁₂₀ (*Puits* 3 et 5). Les anticorps produits au cours de l'infection par les virus recombinants issus du BacMid2 ne sont pas reconnus par la lectine, *Puits* 2 et 4.

Ces expériences démontrent bien que le virus BacGal est capable de complémenter la cellules Sf9 pour pouvoir produire des glycoprotéines galactosylées.

### Exemple 18 : Utilisation du BacMid-Sia3

### 1. Construction d'un baculovirus recombinant exprimant sa glycoprotéine d'enveloppe gp64 sialylée en alpha 2,3.

L'activité du BacSia3 a été contrôlée en utilisant comme protéine modèle la glycoprotéine de surface du virus, la gp64. La glycoprotéine gp64 est la glycoprotéine majeure du baculovirus, elle est impliquée dans les toutes premières étapes de l'infection. Elle est localisée à la surface du virus. Il a été montré que cette glycoprotéine était susceptible d'être galactosylée et sialylée (Jarvis et al. 1995). Pour cela, un virus recombinant a été obtenu par recombinaison homologue entre le BacMidSia3 et les vecteurs de transfert pVTPH et pVT/gp37 vides. La présence de motifs α2,3 sialyl- a été démontrée grâce à un lectine blot réalisé avec la lectine di-CBM40 décrit dans l'article (Ribeiro et al., 2016).

### • Génération et clonage des baculo virus recombinants.

Les cellules Sf9 ont été transfectées par lipofection avec les pVTPH et pVT/gp37 vides et de l'ADN du Bacmid2 (contrôle) ou le BacMid-Sia3 obtenu dans l'Exemple 12 et selon le principe de la Figure 19A. Après 7 jours d'infection à 28°C, les virus sécrétés dans le surnageant de culture ont été clonés par la technique des plages de lyse. Quatre clones viraux ont été sélectionnés, amplifiés et leur génome extrait pour être analysé par Southern blot. Le gène inséré dans le génome viral a été amplifié par PCR puis séquencé.

### • Analyse de la glycosylation par lectine blot.

La lectine utilisée dans cet exemple est la di-CBM40 biotinylée. Le protocole qui a été utilisé est semblable à celui qui est décrit dans l'Exemple 17. Après saturation, la membrane a été incubée avec la lectine diCBM40-Biotinylée diluée 1/200 (5,7 µg/ml) en TBS-T ou avec la lectine SNA-Dig (Roche, Kit DIG Glycan differentiation Kit) dilué 1/1000 en TBS-T. La révélation des membranes a été réalisée comme décrit dans l'exemple 17. La présence de la gp64 a été contrôlée par western blot en présence d'un anticorps anti-gp64 (Anticorps monoclonal de souris AcV5 référence SC65499, Santa Cruz Biotechnology).

### 2. Résultats

Comme le montre la figure 20A, la gp64 est détectée dans tous les échantillons étudiés. Dans le panel B, la lectine SNA qui reconnaît très spécifiquement les acides sialiques liés en α2,6 se lie à la gp64 uniquement lorsqu'elle est produite avec un virus qui co-exprime la ST6 (voir Exemple 19) mais ne se lie pas à la gp64 qui est produite au cours de l'infection avec un virus qui co-exprime la ST3.

La Figure 20C, montre par contre que la lectine di-CBM40 reconnaît bien la gp64 qui a été produite au cours de l'infection avec le virus produisant la ST3. Il n'y a pas non plus de marquage de la gp64 mannosylée qui est produite lorsque les cellules sont infectées avec un virus sauvage. On remarquera cependant un marquage plus faible mais net de la gp64 présentant des acides sialiques liés en α2,6.

Ces expériences démontrent bien que le virus BACSia3 est capable de complémenter la cellules Sf9 pour pouvoir produire des glycoprotéines sialylées en α2,3.

### Exemple 19 : Utilisation du BacMid-Sia6

### 1. Construction d'un baculovirus recombinant exprimant une protéine recombinante sialylée en alpha 2,6.

L'activité du BacSia6 a été contrôlée en utilisant comme protéine modèle la Glycoprotéine G du virus de la Stomatite Vésiculaire, VSVg, la protéine X et la glycoprotéine gp64 du baculovirus.

### • Génération et clonage des baculo virus recombinants.

Le fragment d'ADNc codant la protéine d'intérêt a été cloné dans le pVTPH selon le principe général décrit dans la **Figure 19A****.** Les cellules *Sf*9 ont été transfectées par lipofection avec les pVTPH chargés, le pVT/gp37 modifié et de l'ADN du Bacmid2 (contrôle) ou du BacMid-Sia6 obtenu dans l'Exemple 13. Après 7 jours d'infection à 28°C, les virus sécrétés dans le surnageant de culture ont été clonés par la technique des plages de lyse. Quatre clones viraux ont été sélectionnés, amplifiés et leur génome extrait pour être analysé par Southern blot. Les gènes insérés dans le génome viral ont été amplifiés par PCR puis séquencés.

### • Production des protéines recombinantes

Les protéines ont été produites comme décrit dans l'Exemple 18.

### • Analyse de la glycosylation par lectine blot.

La présence des protéines recombinantes a été contrôlée par Western lot. Après transfert des protéines, les membranes de nitrocellulose ont été incubées en présence des différents anticorps spécifiques, anti-VSVg (anticorps monoclonal de souris conjugué à la peroxydase, référence A5977, Sigma), anti-gp64 (anticorps monoclonal de souris AcV5 référence SC65499, Santa Cruz Biotechnology). La révélation a été effectuée soit directement comme décrit dans l'Exemple 16 lorsque l'anticorps est directement conjugué à la peroxydase soit après incubation avec un anticorps secondaire conjugué à la peroxydase (sérum de lapin anti-IgG de souris conjugué à la peroxydase, référence A9044). La peroxydase a été révélée par chimioluminescence avec le système ECL SuperSignal^{®} West Pico chemiluminescent Substrate (référence 34077, Thermo scientific).

La lectine qui a été utilisée dans cet exemple est la SNA (*Sambuscus nigra* agglutinin) qui reconnaît les acides sialiques liés en α2,6. La SNA a été révélée comme décrit dans l'Exemple 17.

### 2. Résultats

### a. Glycosylation de la protéine X exprimée par le baculovirus recombinant généré à partir du BacSia6

Comme le montre le Western lot, **Figure 21A****,** lorsque la protéine X a été produite avec un virus recombinant issu du BacMid2, sa taille est nettement inférieure à celle de la protéine du commerce qui a été produite par des cellules de mammifères, ici la cellule CHO (comparer les Puits 1 et 3). Par contre, lorsqu'elle a été produite avec un baculovirus recombinant issu du BacSia6, la protéine X a présenté une taille comparable à celle qui a été produite en cellules CHO (comparer Puits 2 et Puits 3).

L'analyse par lectine Blot, **Figure 21B****,** a confirmé la présence d'acides sialiques liés en α2,6 sur cette protéine lorsqu'elle a été produite avec un baculovirus recombinant issu du BacSia6 (Puits 2). La protéine X a en effet été reconnue par la SNA (protocole de révélation décrit dans l'Exemple 17), expliquant ainsi l'augmentation de la masse molaire de la protéine X. Cette expérience a également montré l'absence d'un tel motif sur la protéine commerciale qui a été produite en cellules CHO (*Puits* 3), en effet la lignée CHO exprime uniquement une α2,3 sialyltransférase. Ainsi comme le montre la **Figure 21C****,** la protéine X exprimée en CHO (Puits 3) a été reconnue par la MAA (protocole de révélation décrit dans l'Exemple 17) qui est spécifique des acides sialiques liés en α2,3, ce qui n'est pas le cas de la protéine qui a été exprimée avec le baculovirus recombinant issu du BacSia6 (Puits 2).

### b. Glycosylation de la VSVg exprimée par le baculovirus recombinant généré à partir du BacSia6.

La VSVg étant une protéine membranaire nous avons analysé les culots des cellules infectées. Comme le montre la **Figure 22A** (Puits 2 et 3), la protéine VSVg a été produite après infection des cellules Sf9 avec les 2 virus recombinants qu'ils soient issus du BacMid2 ou du BacSia6.

Par contre, l'analyse par lectine blot avec la SNA (protocole de révélation décrit dans l'Exemple 17) a montré un marquage intense de la protéine VSVg uniquement lorsqu'elle est exprimée à partir du baculovirus issu du BacSia6 **(Figure 22B,** Puits 2). Avec la protéine VSVg produite après infection avec le baculovirus issu du BacMid2 on a uniquement noté un marquage non spécifique **(Figure 22B,** Puits 3). La protéine « contrôle positif » qui est fourni avec le Kit de différenciation des glycannes (Dig Glycan Differentiation Kit), la fétuine, a très bien été marquée par la SNA **(Figure 22B,** Puits 1).

### c. Glycosylation de la gp64 du baculovirus recombinant généré à partir du BacSia6.

Nous avons également vérifié que le virus recombinant exprimant la VSVg sialylée (voir ci-dessus) portait également une gp64 sialylée. Pour cela, les baculovirus sécrétés dans le surnageant de culture ont été sédimentés (35 000 rpm pendant 60 minutes, centrifugeuse Beckman Optima LE-80K, rotor TI-70-1) puis repris par un tampon de lyse pour être analysés en Western blot puis lectine blot. Comme précédemment la fétuine a été utilisée comme marqueur positif à la SNA **(Figure 22C** et **Figure 22D,** Puits 1).

La **(Figure 22C** montre bien la présence de la gp64 dans les Puits 2 et 3. Comme ce que nous observons avec la VSVg, la gp64 qui a été produite après infection avec le virus issu du BacSia6 (Puits 2) est la seule qui est reconnue par la SNA (protocole de révélation décrit dans l'Exemple 17) **Figure 22D,** confirmant ainsi que la gp64 est sialylée en α2,6. On notera également la présence de marquages non spécifiques dans les *Puits* 2 et 3 (marquages identiques dans les 2 Puits).

Ces expériences démontrent bien que le virus BacSia6 est capable de complémenter la cellules Sf9 pour pouvoir produire des glycoprotéines sialylées en α2,6.

### Exemple 20 : le virus BacMid Sia6-II

Nous avons analysé le génome du BacMidSia6II en Southern blot pour contrôler son organisation génomique.

Comme le montre la **Figure 19B****,** le profil de restriction et le patron d'hybridation est conforme au résultat attendu. Le gène de la ST6 est donc bien intégré dans le locus pif1.

### Exemple 21 : Utilisation du BacMid Sia3/6

### 1. Construction d'un baculovirus recombinant exprimant une protéine recombinante sialylée en alpha 2,3 et en alpha 2,6.

L'activité du BacSia3-6 a été contrôlée en utilisant comme protéine modèle la glycoprotéine de surface du virus, gp64. Pour cela, un virus recombinant a été obtenu par recombinaison homologue entre le BacMidSia3/6 et les vecteurs de transfert pVTPH et pVT/gp37 vides. La présence de motifs α2,3 sialyl- et α2,6 sialyl- a été analysée par lectine blot réalisé en présence de la lectine di-CBM40 qui reconnaît les acides sialiques en α2,3 et dans une moindre mesure les acides sialiques liés en α2,6 et la SNA qui ne reconnaît que les acides sialiques liées en α2,6 et pas ceux qui sont liés en α2,3.

### • Génération et clonage des baculo virus recombinants.

Les cellules Sf9 ont été transfectées par lipofection avec les pVTPH et pVT/gp37 vides et de l'ADN du Bacmid2 (contrôle) ou le BacMid-Sia3/6 obtenu dans l'Exemple 15. Après 7 jours d'infection à 28°C, les virus sécrétés dans le surnageant de culture ont été clonés par la technique des plages de lyse. Quatre clones viraux ont été sélectionnés, amplifiés et leur génome extrait pour être analysé par Southern blot. Le gène inséré dans le génome viral a été amplifié par PCR puis séquencé.

### • Production des protéines recombinantes

Les protéines ont été produites comme décrit dans l'Exemple 17.

### • Analyse de la glycosylation par lectine blot

Les protocoles d'analyse ont été identiques à ceux décrits des Exemples 20, 21 et 22.

### 2. Résultats

La gp64 produite par le virus GalSia3-6 est la seule qui est reconnue à la fois par la SNA (Figure 20B) et par la di-CBM40 (Figure 20C), ce qui démontre bien que les 2 types d'acides sialiques sont liés à la gp64.

Ces expériences démontrent bien que le virus BacSia3-6 est capable de complémenter les cellules Sf9 pour pouvoir produire des glycoprotéines sialylées en α2,3 et en α2,6.

### REFERENCES

### BREVETS

WO 01/12829
WO 2013/005194

### REFERENCES BIBLIOGRAPHIQUES

Palmberger D, Wilson IB, Berger I, Grabherr R, Rendic D. SweetBac: a new approach for the production of mammalianised glycoproteins in insect cells. PLoS One. 2012;7(4):e34226.
Chang GD, Chen CJ, Lin CY, Chen HC, Chen H. Improvement of glycosylation in insect cells with mammalian glycosyltransferases. J Biotechnol. 2003 Apr 10;102(1):61-71.
Possee RD, Hitchman RB, Richards KS, Mann SG, Siaterli E, Nixon CP, Irving H, Assenberg R, Alderton D, Owens RJ, King LA. Generation of baculovirus vectors for the high-throughput production of proteins in insect cells. Biotechnol Bioeng. 2008 Dec 15;101(6):1115-22.
Tan J, D'Agostaro AF, Bendiak B, Reck F, Sarkar M, Squire JA, Leong P, Schachter H. The human UDP-N-acetylglucosamine: alpha-6-D-mannoside-beta-1,2-N-acetylglucosaminyltransferase II gene (MGAT2). Cloning of genomic DNA, localization to chromosome 14q21, expression in insect cells and purification of the recombinant protein. Eur J Biochem. 1995 Jul 15;231(2):317-28.
D'Agostaro G, Bendiak B, Tropak M. Cloning of cDNA encoding the membrane-bound form of bovine beta 1,4-galactosyltransferase. Eur J Biochem. 1989 Jul 15;183(1):211-7.
Münster AK, Eckhardt M, Potvin B, Mühlenhoff M, Stanley P, Gerardy-Schahn R. Mammalian cytidine 5'-monophosphate N-acetylneuraminic acid synthetase: a nuclear protein with evolutionarily conserved structural motifs. Proc Natl Acad Sci U S A. 1998 Aug 4;95(16):9140-5.
Lawrence SM, Huddleston KA, Pitts LR, Nguyen N, Lee YC, Vann WF, Coleman TA, Betenbaugh MJ. Cloning and expression of the human N-acetylneuraminic acid phosphate synthase gene with 2-keto-3-deoxy-D-glycero- D-galacto-nononic acid biosynthetic ability. J Biol Chem. 2000 Jun 9;275(23):17869-77.
Kitagawa H, Paulson JC. Cloning of a novel alpha 2,3-sialyltransferase that sialylates glycoprotein and glycolipid carbohydrate groups. J Biol Chem. 1994 Jan 14;269(2):1394-401. Grundmann U, Nerlich C, Rein T, Zettlmeissl G. Complete cDNA sequence encoding human beta-galactoside alpha-2,6-sialyltransferase. Nucleic Acids Res. 1990 Feb 11;18(3):667.
Cieplik M, Klenk HD, Garten W. Identification and characterization of spodoptera frugiperda furin: a thermostable subtilisin-like endopeptidase. Biol Chem. 1998 Dec;379(12):1433-40.
Juliant S, Lévêque M, Cérutti P, Ozil A, Choblet S, Violet ML, Slomianny MC, Harduin-Lepers A, Cérutti M. Engineering the baculovirus genome to produce galactosylated antibodies in lepidopteran cells. Methods Mol Biol. 2013;988:59-77.
Jarvis DL, Finn EE. Biochemical analysis of the N-glycosylation pathway in baculovirus-infected lepidopteran insect cells. Virology. 1995 Oct 1;212(2):500-11.
Ribeiro JP, Pau W, Pifferi C, Renaudet O, Varrot A, Mahal LK, Imberty A. Characterization of a high-affinity sialic acid-specific CBM40 from Clostridium perfringens and engineering of a divalent form. Biochem J. 2016 Jul 15;473(14):2109-18
Pijlman, Gorben P., Jessica E. van Schijndel, and Just M. Vlak. "Spontaneous excision of BAC vector sequences from bacmid-derived baculovirus expression vectors upon passage in insect cells." Journal of General Virology 84.10 (2003): 2669-2678.

## Revendications

1. Procédé pour produire un baculovirus recombinant dont le génome comprend un ou plusieurs transgène(s) codant chacun une enzyme de maturation des protéines et n transgènes codant chacun un polypeptide d'intérêt, ledit procédé comprend les étapes de :
a) Préparer, dans une cellule d'insecte, un génome de baculovirus recombinant capable de se répliquer qui comprend un ou plusieurs transgène(s) codant chacun une enzyme de maturation des protéines et n transgènes codant chacun un polypeptide d'intérêt, par recombinaison homologue entre :
a1) un génome de baculovirus déficient pour la réplication dans lequel n gènes essentiels à la réplication virale sont non-fonctionnels et qui comprend un ou plusieurs transgène(s) codant chacun une enzyme de maturation des protéines, et
a2) n vecteurs de transfert comprenant chacun:
i) une séquence nucléotidique permettant de restaurer la fonction d'un des n gènes essentiels à la réplication virale non-fonctionnel,
ii) un des n transgènes codant un polypeptide d'intérêt,
l'ensemble des séquences nucléotidiques i) des n vecteurs de transfert étant capables de restaurer la réplication du génome de baculovirus déficient pour la réplication,
n étant un nombre entier au moins égal à 2 ; et
b) Générer un baculovirus recombinant dans une cellule d'insecte qui comprend le génome de baculovirus recombinant obtenu à l'étape a),
ledit procédé étant **caractérisé en ce que** la recombinaison se fait en une seule étape dans la cellule d'insecte.

2. Procédé selon la revendication 1, dans lequel génome de baculovirus déficient pour la réplication de l'étape a1) est préparé, dans une cellule bactérienne, par recombinaison homologue entre :
- un génome de baculovirus déficient pour la réplication dans lequel n gènes essentiels à la réplication virale sont non-fonctionnels, et
- une ou plusieurs séquence(s) nucléotidique(s) comprenant chacune un ou plusieurs transgène(s) codant chacun une enzyme de maturation des protéines.

3. Procédé selon l'une quelconque des revendications 1 ou 2, dans lequel les gènes essentiels à la réplication virale sont sélectionnés parmi 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), helicase (ORF95), vp80 (ORF104), ORF106-107, odv-ec43 (ORF109), gp64/67 (ORF128), ORF132, ORF133, odv-ec27 (ORF144), ORF146, ie1 (ORF147), lef-2 (ORF6).

4. Procédé selon l'une quelconque des revendications 1 à 3, dans lequel les n gènes essentiels à la réplication virale non-fonctionnels sont chacun adjacents à un gène non-essentiel à la réplication virale.

5. Procédé selon la revendication 4, dans lequel le gène non-essentiel à la réplication virale est sélectionné parmi Ph (ORF 8), ORF11, ORF13, egt (ORF15), v-ubiquitin (ORF35), 39K (ORF36), ORF38, p43 (ORF39), lef-12 (ORF41), pcna (ORF49), ORF52, ORF55, Fp (ORF61), ORF63, gp37 (ORF64), ORF68, ORF72, ORF74, ORF82, cg30 (ORF88), ORF91, pif-4 (ORF96), he65 (ORF105), ORF108, ORF110, cathepsine (ORF127), p24 (ORF129), pp34 (ORF131), ORF134, ORF145, odv-e56 (ORF148), ORF5.

6. Procédé selon l'une quelconque des revendications 1 à 5, dans lequel les n transgènes codant un polypeptide recombinent chacun au niveau d'un gène non-essentiel à la réplication virale adjacent à un gène essentiel à la réplication virale non fonctionnel.

7. Procédé selon l'une quelconque des revendications 1 à 6, dans lequel l'enzyme de maturation des protéines est choisie parmi une peptidase signal, une furine, une proprotéine convertase, une glycosyltransférase, une glycosidase, une protéine chaperone, une disulfide isomérase, une acyltransférase, une méthyltransférase, une hydroxylase, une transglutaminase, une farnésyltransférase, une géranylgéranyl-transférase, une N-myristoyltransférase, une palmityltransférase, une protéine kinase, une phosphatase, une transpeptidase, une carboxylase, une ubiquitine ligase.

8. Procédé selon l'une quelconque des revendications 2 à 7, dans lequel le ou les transgène(s) codant une enzyme de maturation des protéines recombinent chacun au niveau d'un gène non-essentiel à la réplication virale, de préférence au niveau d'un gène non-essentiel à la réplication virale non-adjacent à un gène essentiel à la réplication virale non fonctionnel

9. Procédé selon la revendication 8, dans lequel le gène non-essentiel à la réplication virale est sélectionné parmi ptp (ORF1), ctx (ORF3), ORF4, ORF7, odv-e26 (ORF16), ORF17, ORF18, ORF19, ARIF-1 ORF20-21, pif2 (ORF22), protéine F (ORF23), iap1 (ORF27), lef6 (ORF28), ORF29, ORF30, sod (ORF31), fgf (ORF32), gta (ORF42), ORF43, ORF44, ORF45, odv-e66 (ORF46), ORF47, ORF56, ORF57, chaB-like (ORF58/59), chaB-like (ORF60), mtase (ORF69), hcf-1 (ORF70), iap2 (ORF71), ORF86, ORF87, ORF111, ORF114, pif3 (ORF115), ORF116, ORF117, pif1 (ORF119), ORF120, ORF121, ORF122, pk2 (ORF123), ORF124, lef7 (ORF125), chitinase (ORF126), gp16 (ORF130), p35 (ORF135), p26 (ORF136), p10 (ORF137), p74 (ORF138), ORF149, ORF150, ie2 (ORF151), pe38 (ORF153) et ORF154.

10. Procédé selon l'une quelconque des revendications 1 à 9, dans lequel n est un nombre entier allant de 2 à 31, par exemple allant de 2 à 10, et de préférence n est égal ou supérieur à 3.

## Patentansprüche

1. Verfahren zur Herstellung eines rekombinanten Baculovirus, dessen Genom ein oder mehrere Transgene umfasst, die jeweils für ein Proteinreifungsenzym kodieren, sowie n Transgene, die jeweils für ein gewünschtes Polypeptid kodieren, wobei das Verfahren folgende Schritte umfasst:
a) Herstellung eines replikationsfähigen rekombinanten Baculovirus-Genoms in einer Insektenzelle, das ein oder mehrere Transgene umfasst, die jeweils für ein Proteinreifungsenzym kodieren, sowie n Transgene, die jeweils für ein gewünschtes Polypeptid kodieren, durch homologe Rekombination zwischen:
a1) einem replikationsdefizienten Baculovirus-Genom, in dem n Gene, die für die virale Replikation essentiell sind, funktionsunfähig sind und das ein oder mehrere Transgene umfasst, die jeweils für ein Proteinreifungsenzym kodieren, und
a2) n Transfervektoren, die jeweils enthalten:
i) eine Nukleotidsequenz, die die Funktion eines der n funktionsunfähigen, für die virale Replikation essentiellen Gene wiederherstellen kann,
ii) eines der n Transgene, das für ein gewünschtes Polypeptid kodiert, wobei die Gesamtheit der Nukleotidsequenzen gemäß i) der n Transfervektoren in der Lage ist, die Replikation des replikationsdefizienten Baculovirus-Genoms wiederherzustellen,
n ist eine ganze Zahl, die mindestens 2 beträgt; und
b) Erzeugung eines rekombinanten Baculovirus in einer Insektenzelle, das das in Schritt a) erhaltene rekombinante Baculovirus-Genom umfasst,
wobei sich das Verfahren dadurch auszeichnet, dass die Rekombination in einem einzigen Schritt in der Insektenzelle erfolgt.

2. Verfahren nach Anspruch 1, wobei ein replikationsdefizientes Baculovirus-Genom gemäß Schritt a1) in einer Bakterienzelle durch homologe Rekombination zwischen:
- einem replikationsdefizienten Baculovirus-Genom, in dem n für die virale Replikation essentielle Gene funktionsunfähig sind, und
- einer oder mehreren Nukleotidsequenz(en), die jeweils ein oder mehrere Transgene umfassen, die jeweils für ein Proteinreifungsenzym kodieren,
hergestellt wird.

3. Verfahren nach einem der Ansprüche 1 oder 2, wobei die für die virale Replikation essentiellen Gene ausgewählt sind aus: 1629 (ORF9), Pk1 (ORF10), lef-1 (ORF14), ORF34, lef-11 (ORF37), p47 (ORF40), lef8 (ORF50), DNAJ-Domäne (ORF51), ORF53, vp1054 (ORF54), lef-9 (ORF62), DNA-Pol (ORF65), lef-3 (ORF67), ORF73, ORF75, ORF81, p95 (ORF83), vp39 (ORF89), lef-4 (ORF90), p33 (ORF92), Helikase (ORF95), vp80 (ORF104), ORF106-107, odv-ec43 (ORF109), gp64/67 (ORF128), ORF132, ORF133, odv-ec27 (ORF144), ORF146, ie1 (ORF147) und lef-2 (ORF6).

4. Verfahren nach einem der Ansprüche 1 bis 3, wobei die n funktionsunfähigen, für die virale Replikation essentiellen Gene jeweils an ein nicht essentielles Gen für die virale Replikation angrenzen.

5. Verfahren nach Anspruch 4, wobei das nicht essentielle Gen für die virale Replikation ausgewählt ist aus: Ph (ORF8), ORF11, ORF13, egt (ORF15), v-ubiquitin (ORF35), 39K (ORF36), ORF38, p43 (ORF39), lef-12 (ORF41), pcna (ORF49), ORF52, ORF55, Fp (ORF61), ORF63, gp37 (ORF64), ORF68, ORF72, ORF74, ORF82, cg30 (ORF88), ORF91, pif-4 (ORF96), he65 (ORF105), ORF108, ORF110, cathepsin (ORF127), p24 (ORF129), pp34 (ORF131), ORF134, ORF145, odv-e56 (ORF148) und ORF5.

6. Verfahren nach einem der Ansprüche 1 bis 5, wobei die n Transgene, die jeweils für ein Polypeptid kodieren, an dem Lokus eines Gens rekombinieren, das nicht für die virale Replikation essentiell ist und das einem funktionsunfähigen, für die virale Replikation essentiellen Gen benachbart ist.

7. Verfahren nach einem der Ansprüche 1 bis 6, wobei das Proteinreifungsenzym ausgewählt ist aus: einer Signalpeptidase, einer Furin, einer Proproteinkonvertase, einer Glycosyltransferase, einer Glycosidase, einem Chaperon-Protein, einer Disulfidisomerase, einer Acyltransferase, einer Methyltransferase, einer Hydroxylase, einer Transglutaminase, einer Farnesyltransferase, einer Geranylgeranyltransferase, einer N-Myristoyltransferase, einer Palmityltransferase, einer Phosphatase, einer Transpeptidase, einer Carboxylase und einer Ubiquitinligase.

8. Verfahren nach einem der Ansprüche 2 bis 7, wobei das bzw. die Transgen(e), das/die jeweils für ein Proteinreifungsenzym kodieren, an dem Lokus eines Gens rekombinieren, das nicht für die virale Replikation essentiell ist, vorzugsweise an dem Lokus eines Gens, das nicht für die virale Replikation essentiell und nicht benachbart zu einem funktionsunfähigen, für die virale Replikation essentiellen Gen ist.

9. Verfahren nach Anspruch 8, wobei das nicht essentielle Gen für die virale Replikation ausgewählt ist aus: ptp (ORF1), ctx (ORF3), ORF4, ORF7, odv-e26 (ORF16), ORF17, ORF18, ORF19, ARIF-1 ORF20-21, pif2 (ORF22), Protein F (ORF23), iap1 (ORF27), lef6 (ORF28), ORF29, ORF30, sod (ORF31), fgf (ORF32), gta (ORF42), ORF43, ORF44, ORF45, odv-e66 (ORF46), ORF47, ORF56, ORF57, chaB-like (ORF58/59), chaB-like (ORF60), mtase (ORF69), hcf-1 (ORF70), iap2 (ORF71), ORF86, ORF87, ORF111, ORF114, pif3 (ORF115), ORF116, ORF117, pif1 (ORF119), ORF120, ORF121, ORF122, pk2 (ORF123), ORF124, lef7 (ORF125), Chitinase (ORF126), gp16 (ORF130), p35 (ORF135), p26 (ORF136), p10 (ORF137), p74 (ORF138), ORF149, ORF150, ie2 (ORF151), pe38 (ORF153) und ORF154.

10. Verfahren nach einem der Ansprüche 1 bis 9, wobei n eine ganze Zahl im Bereich von 2 bis 31 ist, beispielsweise im Bereich von 2 bis 10, und vorzugsweise n gleich oder größer als 3 ist.

## Claims

1. Method for producing a recombinant baculovirus of which the genome comprises one or more transgene(s) each encoding a protein maturation enzyme and n transgenes each encoding a polypeptide of interest, said method comprising the steps of:
a) Preparing, in an insect cell, a recombinant baculovirus genome capable of replicating which comprises one or more transgene(s), each encoding a protein maturation enzyme and n transgenes each encoding a polypeptide of interest, by homologous recombination between:
a1) a replication deficient baculovirus genome in which n genes essential for viral replication are non-functional and which comprises one or more transgene(s), each encoding a protein maturation enzyme, and
a2) n transfer vectors each comprising:
i) a nucleotide sequence enabling to restore the function of one of the n non-functional genes essential for viral replication,
ii) one of the n transgenes encoding a polypeptide of interest,
the set of nucleotide sequences i) of the n transfer vectors being capable of restoring the replication of the replication deficient baculovirus genome,
n being an integer at least equal to 2; and
b) Generating a recombinant baculovirus in an insect cell which comprises the recombinant baculovirus genome obtained at step a),
said method being **characterized in that** the recombination takes place in a single step in the insect cell.

2. Method according to claim 1, wherein a replication deficient baculovirus genome of step a1) is prepared, in a bacterial cell, by homologous recombination between:
- a replication deficient baculovirus genome in which n genes essential for viral replication are non-functional, and
- one or more nucleotide sequence(s) each comprising one or more transgene(s) each encoding a protein maturation enzyme.

3. Method according to either one of claim 1 or 2, wherein the genes essential for viral replication are selected from: 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), helicase (ORF95), vp80 (ORF104), ORF106-107, odv-ec43 (ORF109), gp64/67 (ORF128), ORF132, ORF133, odv-ec27 (ORF144), ORF146, ie1 (ORF147), and lef-2 (ORF6).

4. Method according to any one of claims 1 to 3, wherein the n non-functional genes essential for viral replication are each adjacent to a gene not essential for viral replication.

5. Method according to claim 4, wherein the gene not essential for viral replication is selected from: Ph (ORF 8), ORF11, ORF13, egt (ORF15), v-ubiquitin (ORF35), 39K (ORF36), ORF38, p43 (ORF39), lef-12 (ORF41), pcna (ORF49), ORF52, ORF55, Fp (ORF61), ORF63, gp37 (ORF64), ORF68, ORF72, ORF74, ORF82, cg30 (ORF88), ORF91, pif-4 (ORF96), he65 (ORF105), ORF108, ORF110, cathepsin (ORF127), p24 (ORF129), pp34 (ORF131), ORF134, ORF145, odv-e56 (ORF148), and ORF5.

6. Method according to any one of claims 1 to 5, wherein the n transgenes encoding a polypeptide each recombine at the locus of a gene not essential for viral replication adjacent to a non-functional gene essential for viral replication.

7. Method according to any one of claims 1 to 6, wherein the protein maturation enzyme is selected from: a signal peptidase, a furin, a proprotein convertase, a glycosyltransferase, a glycosidase, a chaperone protein, an isomerase disulphide, an acyltransferase, a methyltransferase, a hydroxylase, a transglutaminase, a farnesyltransferase, a geranylgeranyl-transferase, a N-myristoyltransferase, a palmityltransferase, a phosphatase, a transpeptidase, a carboxylase, and a ubiquitin ligase.

8. Method according to any one of claims 2 to 7, wherein the transgene(s) encoding a protein maturation enzyme each recombine at the locus of a gene not essential for viral replication, preferably at the locus of a gene not essential for viral replication non-adjacent to a non-functional gene essential for viral replication.

9. Method according to claim 8, wherein the gene not essential for viral replication is selected from: ptp (ORF1), ctx (ORF3), ORF4, ORF7, odv-e26 (ORF16), ORF17, ORF18, ORF19, ARIF-1 ORF20-21, pif2 (ORF22), protein F (ORF23), iap1 (ORF27), lef6 (ORF28), ORF29, ORF30, sod (ORF31), fgf (ORF32), gta (ORF42), ORF43, ORF44, ORF45, odv-e66 (ORF46), ORF47, ORF56, ORF57, chaB-like (ORF58/59), chaB-like (ORF60), mtase (ORF69), hcf-1 (ORF70), iap2 (ORF71), ORF86, ORF87, ORF111, ORF114, pif3 (ORF115), ORF116, ORF117, pif1 (ORF119), ORF120, ORF121, ORF122, pk2 (ORF123), ORF124, lef7 (ORF125), chitinase (ORF126), gp16 (ORF130), p35 (ORF135), p26 (ORF136), p10 (ORF137), p74 (ORF138), ORF149, ORF150, ie2 (ORF151), pe38 (ORF153) and ORF154.

10. Method according to any one of claims 1 to 9, wherein n is an integer ranging from 2 to 31, for example ranging from 2 to 10, and preferably n is equal to or greater than 3.
